(19) Europäisches Patentamt
European Patent Office
Office européen des brevets

(11) EP 3 597 643 A1

(12) **EUROPEAN PATENT APPLICATION**
published in accordance with Art. 153(4) EPC

(43) Date of publication:
**22.01.2020 Bulletin 2020/04**

(21) Application number: **18767516.0**

(22) Date of filing: **12.03.2018**

(51) Int Cl.:
***C07D 277/82*** *(2006.01)* ***C07C 69/78*** *(2006.01)*
***C08F 20/38*** *(2006.01)* ***C09K 19/38*** *(2006.01)*
***G02B 1/111*** *(2015.01)* ***G02B 5/30*** *(2006.01)*

(86) International application number:
**PCT/JP2018/009528**

(87) International publication number:
**WO 2018/168778 (20.09.2018 Gazette 2018/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.03.2017 JP 2017053686**

(71) Applicant: **Zeon Corporation**
**Tokyo 100-8246 (JP)**

(72) Inventors:
- **SAKAMOTO Kei**
  **Tokyo 100-8246 (JP)**
- **OKUYAMA Kumi**
  **Tokyo 100-8246 (JP)**
- **MIMA Takanori**
  **Tokyo 100-8246 (JP)**

(74) Representative: **Beckmann, Claus**
**Kraus & Weisert**
**Patentanwälte PartGmbB**
**Thomas-Wimmer-Ring 15**
**80539 München (DE)**

(54) **POLYMERIZABLE COMPOUND, POLYMERIZABLE LIQUID CRYSTAL MIXTURE, POLYMER, OPTICAL FILM, OPTICALLY ANISOTROPIC BODY, POLARIZING PLATE, DISPLAY DEVICE, ANTIREFLECTION FILM, AND COMPOUND**

(57) Disclosed is a polymerizable compound useful for preparing a polymer that enables the production of a film, such as an optical film, which has a good balance of lightness and saturation and which can improve reverse wavelength dispersion on the short wavelength side while achieving reverse wavelength dispersion on the longer wavelength side. The polymerizable compound of the present disclosure is represented by:

$$P^1-G^1-Y^3\left[B^1-Y^1\right]_p A^1-Z^1-Ar^0-Z^2-Xa-Z^3-Ar^1-Z^4-A^2\left[Y^2-B^2\right]_q Y^4-G^2-P^2$$

**(I-1)**

where in the formula (I-1), $A^1$ and $A^2$ each independently represent an aromatic group which may have a substituent, $B^1$ and $B^2$ each independently represent a cyclic aliphatic group which may have a substituent, or, an aromatic group which may have a substituent, and p and q each independently represent an integer from 0 to 2.

EP 3 597 643 A1

## Description

TECHNICAL FIELD

The present disclosure relates to an optical film and an optically anisotropic body which have a good balance of lightness and saturation and which can improve reverse wavelength dispersion on the short wavelength side while achieving reverse wavelength dispersion on the longer wavelength side, and, a polarizing plate, a display device, and an antireflection film using the optically anisotropic body.

Further, the present disclosure also relates to a polymer which can be used in the preparation of the aforementioned optical film and the optically anisotropic body, a polymerizable liquid crystal mixture and a polymerizable compound which can be used in the preparation of the polymer, and, a compound which can be used in the preparation of the polymerizable compound.

BACKGROUND

Examples of retardation plates used in various devices such as flat panel displays include quarter-wave plates that convert linearly polarized light to circularly polarized light and half-wave plates that perform 90° conversion of the plane of vibration of polarization of linearly polarized light. These retardation plates can accurately impart a retardation of 1/4λ or 1/2λ of the wavelength of light with respect to specific monochromatic light.

However, conventional retardation plates have a problem that polarized light that passes therethrough and is output therefrom is converted to colored polarized light. The reason is that since a constituent material of the retardation plate has a property of wavelength dispersion with respect to retardation, and a distribution arises in the polarization state of each wavelength for white light, which is a composite wave in which light in the visible region is mixed, it is impossible to achieve accurate adjustment to polarized light with a retardation of 1/4λ or 1/2λ over the entire wavelength region of input light.

In order to solve this problem, various retardation plates which are wideband retardation plates that can achieve uniform retardation with respect to light over a wide wavelength region having so-called reverse wavelength dispersion have been considered.

On the one hand, it has been desired to reduce the thickness of the flat panel display device as much as possible along with an improvement in functionality and widespread use of information terminals such as mobile personal computers and mobile phones. Therefore, a reduction in the thickness of the retardation plates which are components has also been desired.

In terms of methods of achieving thickness-reduction, a method in which a retardation plate is produced by applying a polymerizable composition comprising a low-molecular weight polymerizable compound onto a film substrate to form an optical film has been regarded as the most effective method in recent years. For this reason, there has been much development of polymerizable compounds that are capable of forming optical films having excellent reverse wavelength dispersion, and also polymerizable compositions in which these compounds are used.

Specifically, a polymerizable compound has been provide for use in the production of an optical film such as a polarizing plate or a retardation plate capable of uniform conversion of polarized light over a wide wavelength band (for example, refer to WO 2014/010325 (PTL 1)).

CITATION LIST

Patent Literature

PTL 1: WO 2014/010325

SUMMARY

(Technical Problem)

Here, in order to exert an excellent reverse wavelength dispersion over a wide wavelength band, optical films and the like are required to exhibit ideal retardation characteristics such that the retardation value increases in proportion to the wavelength on both the longer wavelength side and the short wavelength side. However, as described in PTL 1, in a conventional polymerizable compound, the obtainable optical film and the like had room for improvement in terms of securing the reverse wavelength dispersion on the longer wavelength side while bringing the retardation properties on the short wavelength side closer to the ideal retardation properties to improve the reverse wavelength dispersion on the short wavelength side.

Further, in recent years, from the viewpoint of improving the display quality of display devices which use optical film and the like, it is also required in optical film and the like, to reduce the difference between the retardation in which the lightness is the lowest and the retardation in which the retardation and the saturation are the lowest.

The present disclosure was conceived in view of the above-described circumstances, and it could be helpful to provide a polymer capable of forming an optical film and an optically anisotropic body which has a good balance of lightness and saturation and which can improve reverse wavelength dispersion on the short wavelength side while achieving reverse wavelength dispersion on the longer wavelength side.

Further, it could be helpful to provide a polymerizable liquid crystal mixture and a polymerizable compound which can be used in the preparation of the aforementioned polymer, and, a compound which can be used in the preparation of the polymerizable compound.

Furthermore, it could be helpful to provide an optical film and an optically anisotropic body which has a good balance of lightness and saturation and which can improve reverse wavelength dispersion on the short wavelength side while achieving reverse wavelength dispersion on the longer wavelength side, and, a polarizing plate, a display device, and an antireflection film using the optically anisotropic body, .

As used herein, the phrase "having a good balance of lightness and saturation" means that the "the difference between the retardation with the lowest lightness and the retardation with the lowest saturation is small".

(Solution to Problem)

The present inventors performed keen research for solving the aforementioned problems, and discovered that the use of a predetermined polymerizable compound represented by the following formula (1-1) makes it possible to obtain a polymer capable of forming an optical film and and an optically anisotropic body which has a good balance of lightness and saturation and which can improve reverse wavelength dispersion on the short wavelength side while achieving reverse wavelength dispersion on the longer wavelength side, and that the use of the polymer enables preparation of an optical film and the like which has a good balance of lightness and saturation and which can improve reverse wavelength dispersion on the short wavelength side while achieving reverse wavelength dispersion on the longer wavelength side. As a result, the present disclosure was completed.

Accordingly, the present disclosure provides a polymerizable compound, a polymerizable liquid crystal mixture, a polymer, an optical film, an optically anisotropic body, a polarizing plate, a display device, an antireflection film, and a compound as described below.

[1] A polymerizable compound represented by formula (I-1):

$$P^1-G^1-Y^3\left[B^1-Y^1\right]_pA^1-Z^1-Ar^0-Z^2-Xa-Z^3-Ar^1-Z^4-A^2\left[Y^2-B^2\right]_qY^4-G^2-P^2$$

**(I-1)**

where in the formula (1-1),

$Ar^0$ represents an aromatic hydrocarbon ring group having at least $D^0$ as a substituent, or, an aromatic heterocyclic ring group having at least $D^0$ as a substituent,

$Ar^1$ represents an aromatic hydrocarbon ring group having at least $D^1$ as a substituent, or, an aromatic heterocyclic ring group having at least $D^1$ as a substituent,

$D^0$ and $D^1$ each independently represent an organic group having 1 to 67 carbon atoms and having at least one aromatic ring selected from the group consisting of an aromatic hydrocarbon ring and an aromatic heterocyclic ring,

Xa represents an organic group having 1 to 20 carbon atoms which may have a substituent,

$Z^1$ to $Z^4$ each independently represent a single bond, -O-, $-O-CH_2-$, $-CH_2-O-$, $-O-CH_2-CH_2$, $-CH_2-CH_2-O-$, -C(=O)-O-, -O-C(=O)-, -C(=O)-S-, -S-C(=O)-, $-NR^{20}-C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-C(=O)-O-$, $-O-C(=O)-CH_2-$, $-CH_2-O-C(=O)-$, $-C(=O)-O-CH_2-$, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-, and $R^{20}$ represents a hydrogen atom or an alkyl group having 1 to 6 carbon atoms,

$A^1$ and $A^2$ each independently represent an aromatic group which may have a substituent,

$B^1$ and $B^2$ each independently represent a cyclic aliphatic group which may have a substituent, or, an aromatic group which may have a substituent,

$Y^1$ to $Y^4$ each independently represent a single bond, -O-, -C(=O)-, -C(=O)-O-, -O-C(=O)-, $-NR^{21}-C(=O)-$,

$-C(=O)-NR^{21}-$, $-O-C(=O)-O-$, $-NR^{21}-C(=O)-O-$, $-O-C(=O)-NR^{21}-$, or, $-NR^{21}-C(=O)-NR^{22}-$, and $R^{21}$ and $R^{22}$ each independently represent a hydrogen atom or an alkyl group having 1 to 6 carbon atoms,

$G^1$ and $G^2$ each independently represent an organic group of either an alkylene group having 1 to 20 carbon atoms, or, an alkylene group having 3 to 20 carbon atoms in which at least one methylene group ($-CH_2-$) is substituted with -O- or -C(=O)-, and the hydrogen atom included in the organic group of $G^1$ and $G^2$ may be substituted with an alkyl group having 1 to 5 carbon atoms, an alkoxy group having 1 to 5 carbon atoms, or, a halogen atom, where the methylene group ($-CH_2-$) at each terminal of $G^1$ and $G^2$ is not substituted with -O- or -C(=O)-,

one of $P^1$ and $P^2$ represents a hydrogen atom or a polymerizable group, and the other of $P^1$ and $P^2$ represents a polymerizable group, and p and q each independently represent an integer from 0 to 2,

where when a plurality of $B^1$, $B^2$, $Y^1$, and $Y^2$ are present, these may be the same or different.

[2] The polymerizable compound according to [1], wherein each of $Ar^0$ and $Ar^1$ is independently represented by any of the following formulas (II-1) to (II-7):

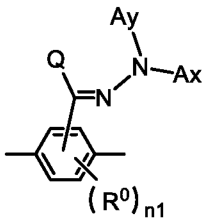
(II-1)

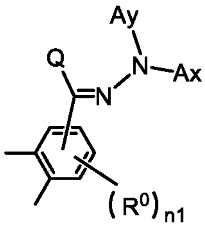
(II-2)

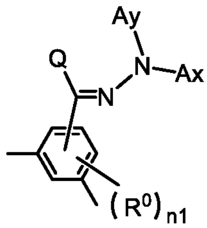
(II-3)

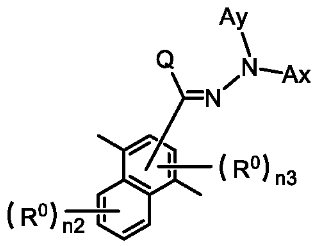
(II-4)

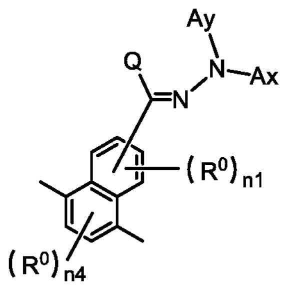
(II-5)

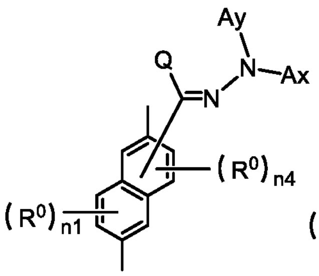
(II-6)

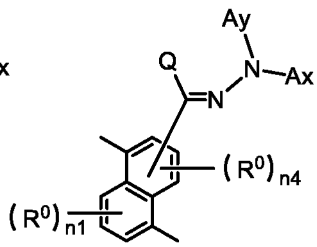
(II-7)

where in the formulas (II-1) to (II-7),

Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring of Ax may have a substituent,

Ay represents a hydrogen atom or an organic group having 1 to 30 carbon atoms which may have a substituent,

Q represents a hydrogen atom or an alkyl group having 1 to 6 carbon atoms, and

$R^0$ represents a halogen atom, a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N-N-dialkylamino group having 2 to 12 carbon atoms, an alkoxy group having 1 to 6 carbon atoms, a nitro group, $-C(=O)-R^a$, $-O-C(=O)-R^a$, $-C(=O)-O-R^a$, or $-SO_2R^a$, $R^a$ represents an alkyl group having 1 to 6 carbon atoms, or, an aromatic hydrocarbon ring group having 6 to 20 carbon atoms which may have an alkyl group having 1 to 6 carbon atoms or an alkoxy group having 1 to 6 carbon atoms as a substituent, and n1 represents an integer from 0 to 3, n2 represents an integer from 0 to 4, n3 is 0 or 1, and n4 represents an integer from 0 to 2,

where when a plurality of $R^0$ are present, these may be the same or different.

[3] The polymerizable compound according to [2], wherein the polymerizable compound is represented by any of the following formulas (III-1) to (III-6):

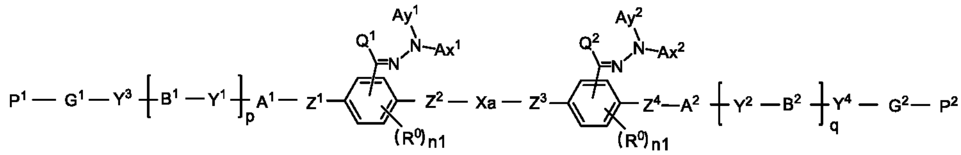

(III-1)

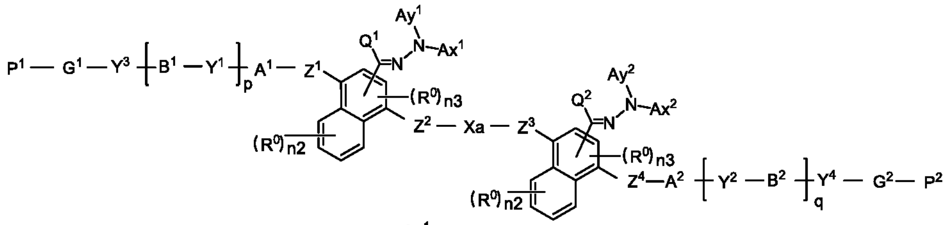

(III-2)

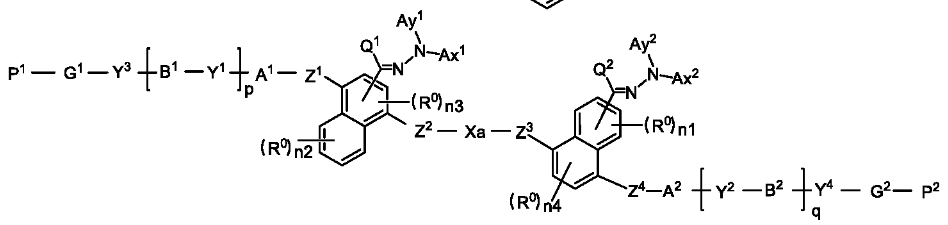

(III-3)

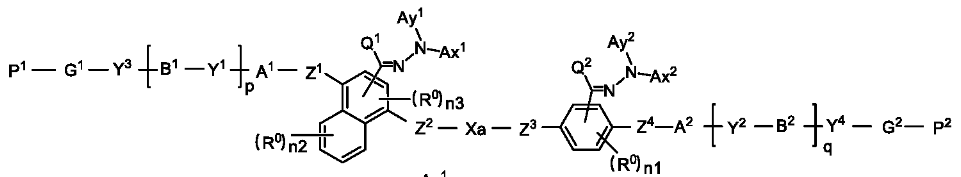

(III-4)

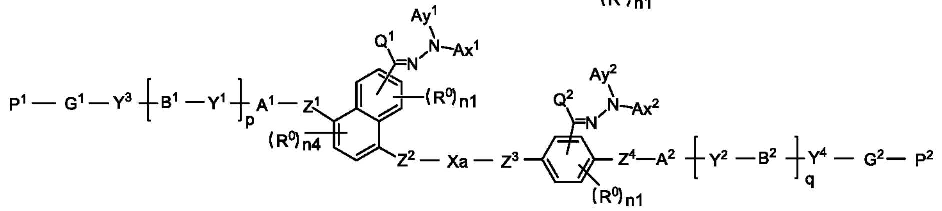

(III-5)

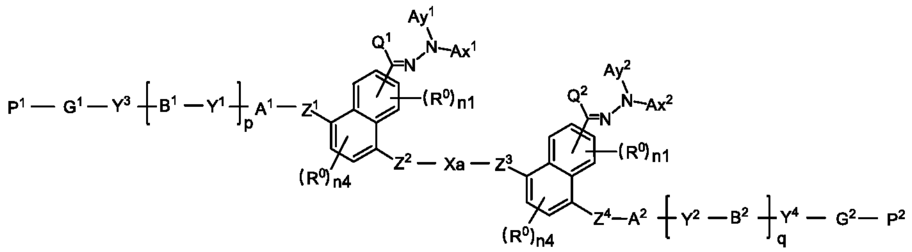

(III-6)

where in the formulas (III-1) to (III-6),

$Z^1$ to $Z^4$, $A^1$, $A^2$, $B^1$, $B^2$, $Y^1$ to $Y^4$, $G^1$, $G^2$, $P^1$, $P^2$, Xa, $R^0$, n1, n2, n3, n4, p, and q are the same as defined above. $Ax^1$ and $Ax^2$ each independently represent an organic group having at least one aromatic ring selected from the group consisting of an aromatic hydrocarbon ring having 6 to 30 carbon atoms and an aromatic heterocyclic ring having 2 to 30 carbon atoms, and the aromatic rings of $Ax^1$ and $Ax^2$ may have a substituent,
$Ay^1$ and $Ay^2$ each independently represent a hydrogen atom or an organic group having 1 to 30 carbon atoms which may have a substituent, and
$Q^1$ and $Q^2$ each independently represent a hydrogen atom or an alkyl group having 1 to 6 carbon atoms, where when a plurality of $B^1$, $B^2$, $Y^1$, $Y^2$, and $R^0$ are present, these may be the same or different.

[4] The polymerizable compound according to [3], wherein $Ay^1$ and $Ay^2$ each independently represent a hydrogen atom, an alkyl group having 1 to 20 carbon atoms which may have a substituent, an alkenyl group having 2 to 20 carbon atoms which may have a substituent, an alkynyl group having 2 to 20 carbon atoms which may have a substituent, a cycloalkyl group having 3 to 12 carbon atoms which may have a substituent, an aromatic hydrocarbon

ring group having 6 to 30 carbon atoms which may have a substituent, or, an aromatic heterocyclic ring group having 2 to 30 carbon atoms which may have a substituent.

[5] The polymerizable compound according to [3] or [4], wherein $Ax^1$ and $Ax^2$ are each independently represented by the following formula (V):

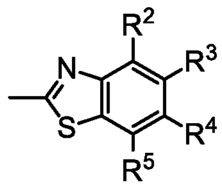

(V)

where in the formula (V), $R^2$ to $R^5$ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, $-OCF_3$, O-C(=O)$R^b$, or -C(=O)-O-$R^b$,

$R^b$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or, an aromatic hydrocarbon ring group having 5 to 18 carbon atoms which may have a substituent, and

each of $R^2$ to $R^5$ may be the same or different, and one or more ring constituents C-$R^2$ to C-$R^5$ may be replaced by a nitrogen atom.

[6] The polymerizable compound according to any one of [1] to [5], wherein $P^1$ and $P^2$ are each independently represented by the following formula (IV):

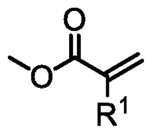

(IV)

where in the formula (IV), $R^1$ represents a hydrogen atom, a methyl group, or a chlorine atom.

[7] The polymerizable compound according to any one of [1] to [6], wherein p and q are both 0.

[8] The polymerizable compound according to any one of [1] to [6], wherein p and q are both 1, and, $B^1$ and $B^2$ each independently represent a cyclic aliphatic group which may have a substituent.

[9] The polymerizable compound according to any one of [1] to [7], wherein the polymerizable compound represented by the formula (1-1) is represented by any of the following formulas (VI-1) to (VI-3):

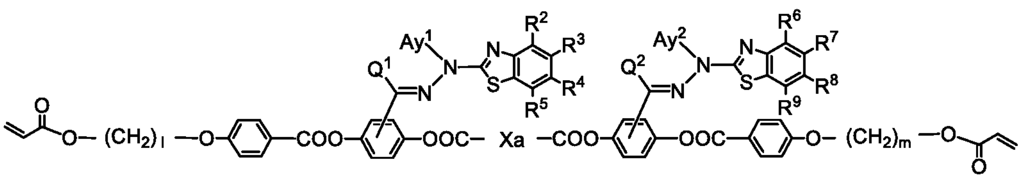

(VI-1)

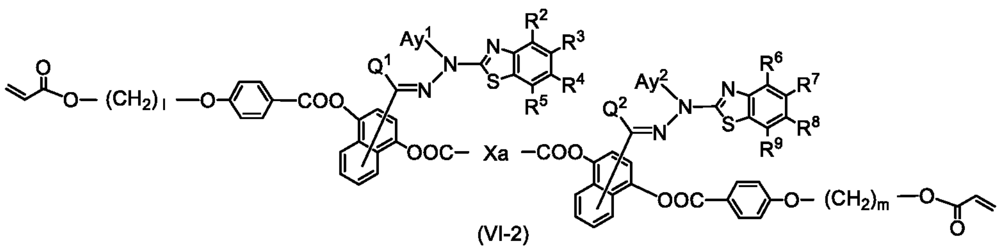

(VI-2)

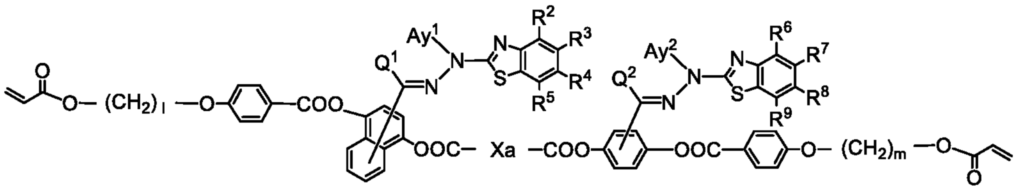

(VI-3)

where in the formulas (VI-1) to (VI-3),

Xa is the same as defined above,
$R^2$ to $R^9$ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, $-OCF_3$, O-C(=O)-$R^b$, or -C(=O)-O-$R^b$,
$R^b$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or, an aromatic hydrocarbon ring group having 5 to 18 carbon atoms which may have a substituent,
one or more ring constituents C-$R^2$ to C-$R^9$ may be replaced by a nitrogen atom.
$Ay^1$ and $Ay^2$ each independently represent a hydrogen atom or an organic group having 1 to 30 carbon atoms which may have a substituent,
$Q^1$ and $Q^2$ each independently represent a hydrogen atom or an alkyl group having 1 to 6 carbon atoms, and
1 and m each independently represent an integer from 1 to 18.

[10] The polymerizable compound according to any one of [1] to [9], wherein Xa is represented by any of the following formulas (VII-1) to (VII-29) :

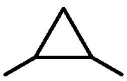
(VII-1)

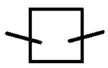
(VII-2)

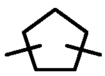
(VII-3)

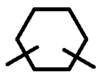
(VII-4)

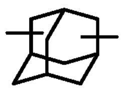
(VII-5)

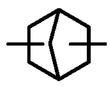
(VII-6)

$-CH_2-$ (VII-7)

$-(CH_2)_2-$ (VII-8)

$-(CH_2)_3-$ (VII-9)

$-(CH_2)_4-$ (VII-10)

$-(CH_2)_5-$ (VII-11)

$-(CH_2)_6-$ (VII-12)

$-(CH_2)_7-$ (VII-13)

$-(CH_2)_8-$ (VII-14)

$-(CH_2)_9-$ (VII-15)

$-(CH_2)_{10}-$ (VII-16)

$-(CH_2)_{11}-$ (VII-17)

$-(CH_2)_{12}-$ (VII-18)

$-(CH_2)_{13}-$ (VII-19)

$-(CH_2)_{14}-$ (VII-20)

$-(CH_2)_{15}-$ (VII-21)

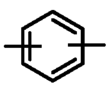
(VII-22)

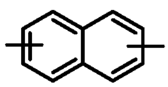
(VII-23)

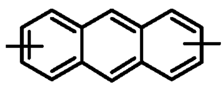
(VII-24)

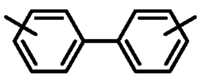
(VII-25)

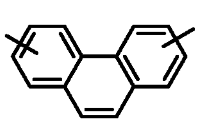
(VII-26)

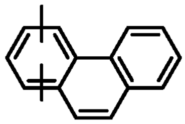
(VII-27)

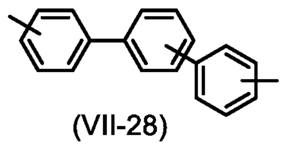
(VII-28)

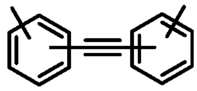
(VII-29)

[11] A polymerizable liquid crystal mixture comprising the polymerizable compound according to any one of [1] to [10] as a main component.

[12] The polymerizable liquid crystal mixture according to [11] comprising the polymerizable compound according to any one of [1] to [10] and a polymerizable compound having a different chemical structure than the following formula (I-1), wherein

an area value of the polymerizable compound according to any one of [1] to [10] measured by high-performance liquid chromatography (HPLC) is a value greater than 50% of a sum of area values of the polymerizable compound according to any one of [1] to [10] and the polymerizable compound having a different chemical structure than the formula (I-1).

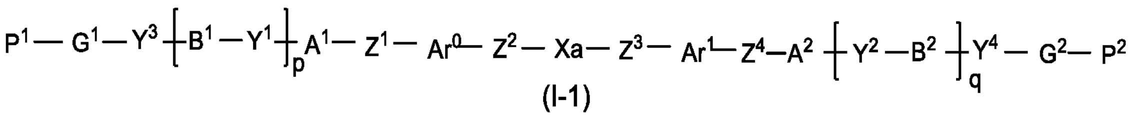
(I-1)

where in the formula (I-1),

$Ar^0$ represents an aromatic hydrocarbon ring group having at least $D^0$ as a substituent, or, an aromatic heterocyclic ring group having at least $D^0$ as a substituent,
$Ar^1$ represents an aromatic hydrocarbon ring group having at least $D^1$ as a substituent, or, an aromatic heterocyclic ring group having at least $D^1$ as a substituent,
$D^0$ and $D^1$ each independently represent an organic group having 1 to 67 carbon atoms and having at least one aromatic ring selected from the group consisting of an aromatic hydrocarbon ring and an aromatic heterocyclic ring,
Xa represents an organic group having 1 to 20 carbon atoms which may have a substituent,
$Z^1$ to $Z^4$ each independently represent a single bond, -O-, $-O-CH_2-$, $-CH_2-O-$, $-O-CH_2-CH_2-CH_2-CH_2-O-$, -C(=O)-O-, -O-C(=O)-, -C(=O)-S-, -S-C(=O)-, $-NR^{20}-C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-C(=O)-O-$, $-O-C(=O)-CH_2-$, $-CH_2-O-C(=O)-$, $-C(=O)-O-CH_2-$, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-, and $R^{20}$ represents a hydrogen atom or an alkyl group having 1 to 6 carbon atoms,
$A^1$ and $A^2$ each independently represent an aromatic group which may have a substituent,
$B^1$ and $B^2$ each independently represent a cyclic aliphatic group which may have a substituent, or, an aromatic group which may have a substituent,
$Y^1$ to $Y^4$ each independently represent a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or, $-NR^{21}-C(=O)-NR^{22}-$ , and $R^{21}$ and $R^{22}$ each independently represent a hydrogen atom or an alkyl group having 1 to 6 carbon atoms,
$G^1$ and $G^2$ each independently represent an organic group of either an alkylene group having 1 to 20 carbon atoms, or, an alkylene group having 3 to 20 carbon atoms in which at least one methylene group ($-CH_2-$) is substituted with -O- or -C(=O)-, and the hydrogen atom included in the organic group of $G^1$ and $G^2$ may be substituted with an alkyl group having 1 to 5 carbon atoms, an alkoxy group having 1 to 5 carbon atoms, or, a halogen atom, where the methylene group ($-CH_2-$) at each terminal of $G^1$ and $G^2$ is not substituted with -O- or -C(=O)-,
one of $P^1$ and $P^2$ represents a hydrogen atom or a polymerizable group, and the other of $P^1$ and $P^2$ represents a polymerizable group, and p and q each independently represent an integer from 0 to 2,

where when a plurality of $B^1$, $B^2$, $Y^1$, and $Y^2$ are present, these may be the same or different.

[13] The polymerizable liquid crystal mixture according to [11] or [12], comprising the polymerizable compound according to any one of [1] to [10], and a polymerizable compound represented by the following formula (1-2), wherein an area value of the polymerizable compound according to any one of [1] to [10] measured by high-performance liquid chromatography (HPLC) is a value greater than 50% of a sum of area values of the polymerizable compound according to any one of [1] to [10] and the polymerizable compound represented by the formula (1-2):

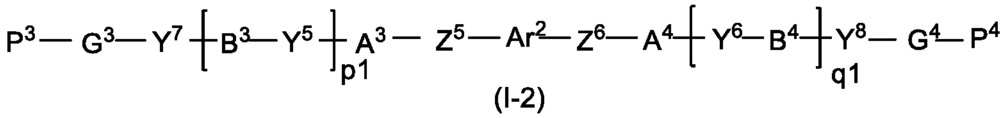

(I-2)

where in the formula (1-2),

$Ar^2$ represents an aromatic hydrocarbon ring group having at least $D^2$ as a substituent, or, an aromatic heterocyclic ring group having at least $D^2$ as a substituent,
$D^2$ represents an organic group having 1 to 67 carbon atoms and having at least one aromatic ring selected from the group consisting of an aromatic hydrocarbon ring and an aromatic heterocyclic ring,
$Z^5$ and $Z^6$ each independently represent a single bond, -O-, -O-$CH_2$-, -$CH_2$-O-, -O-$CH_2$-$CH_2$-$CH_2$-$CH_2$-O-, -C(=O)-O-, -O-C(=O)-, -C(=O)-S-, -S-C(=O)-, -$NR^{20}$-C(=O)-, -C(=O)-$NR^{20}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-C(=O)-O-, -O-C(=O)-$CH_2$-, -$CH_2$-O-C(=O)-, -C(=O)-O-$CH_2$-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C≡C-, and $R^{20}$ represents a hydrogen atom or an alkyl group having 1 to 6 carbon atoms,
$A^3$, $A^4$, $B^3$ and $B^4$ each independently represent a cyclic aliphatic group which may have a substituent, or, an aromatic group which may have a substituent,
$Y^5$ to $Y^8$ each independently represent a single bond, -O-, -C(=O)-, -C(=O)-O-, -O-C(=O)-, -$NR^{21}$-C(=O)-, -C(=O)-$NR^{21}$-, -O-C(=O)-O-, -$NR^{21}$-C(=O)-O-, -O-C(=O)-$NR^{21}$-, or, -$NR^{21}$-C(=O)-$NR^{22}$-, and $R^{21}$ and $R^{22}$ each independently represent a hydrogen atom or an alkyl group having 1 to 6 carbon atoms,
$G^3$ and $G^4$ each independently represent an organic group of either an alkylene group having 1 to 20 carbon atoms, or, an alkylene group having 3 to 20 carbon atoms in which at least one methylene group (-$CH_2$-) is substituted with -O- or -C(=O)-, and the hydrogen atom included in the organic group of $G^3$ and $G^4$ may be substituted with an alkyl group having 1 to 5 carbon atoms, an alkoxy group having 1 to 5 carbon atoms, or, a halogen atom,
one of $P^3$ and $P^4$ represents a hydrogen atom or a polymerizable group, and the other of $P^3$ and $P^4$ represents a polymerizable group, and p1 and q1 each independently represent an integer from 0 to 2.
where when a plurality of $B^3$, $B^4$, $Y^5$, and $Y^6$ are present, these may be the same or different.

[14] The polymerizable liquid crystal mixture according to [13], wherein $Ar^2$ is represented by any of the following formulas (II-1) to (II-7):

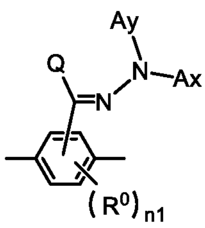

(II-1)

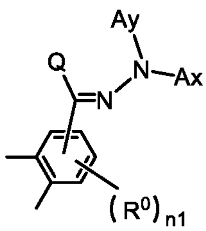

(II-2)

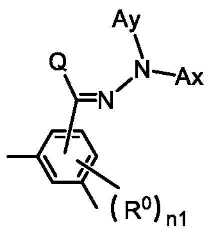

(II-3)

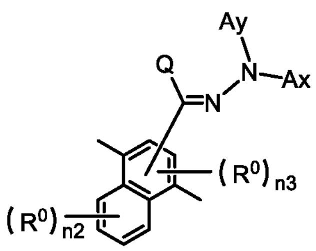

(II-4)

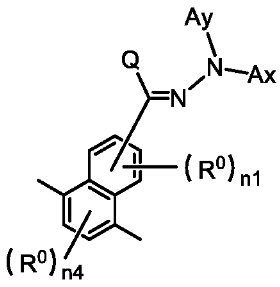

(II-5)

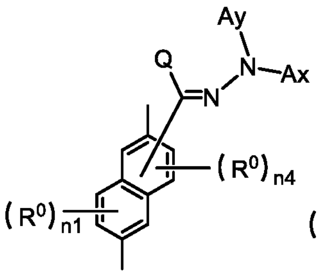

(II-6)

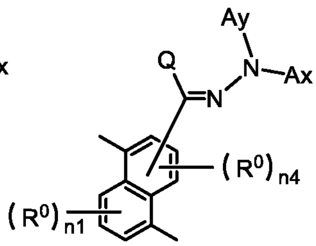

(II-7)

where in the formulas (II-1) to (II-7),

Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring of Ax may have a substituent,
Ay represents a hydrogen atom or an organic group having 1 to 30 carbon atoms which may have a substituent,
Q represents a hydrogen atom or an alkyl group having 1 to 6 carbon atoms, and
$R^0$ represents a halogen atom, a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N-N-dialkylamino group having 2 to 12 carbon atoms, an alkoxy group having 1 to 6 carbon atoms, a nitro group, $-C(=O)-R^a$, $-O-C(=O)-R^a$, $-C(=O)-O-R^a$, or $-SO_2R^a$, $R^a$ represents an alkyl group having 1 to 6 carbon atoms, or, an aromatic hydrocarbon ring group having 6 to 20 carbon atoms which may have an alkyl group having 1 to 6 carbon atoms or an alkoxy group having 1 to 6 carbon atoms as a substituent, and n1 represents an integer from 0 to 3, n2 represents an integer from 0 to 4, n3 is 0 or 1, and n4 represents an integer from 0 to 2.
where when a plurality of $R^0$ are present, these may be the same or different.

[15] The polymerizable liquid crystal mixture according to [13] or [14], wherein $P^3$ and $P^4$ are each independently represented by the following formula (IV):

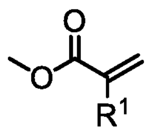

(IV)

where in the formula (IV), $R^1$ represents a hydrogen atom, a methyl group, or a chlorine atom.
[16] A polymer obtainable by polymerizing the polymerizable liquid crystal mixture according to any one of [11] to [15].
[17] An optical film composed of the polymer according to [16] as a constituent material.
[18] An optically anisotropic body comprising a layer composed of the polymer according to [16] as a constituent material.
[19] A polarizing plate comprising the optically anisotropic body according to [18] and a polarizing film.
[20] A display device comprising the polarizing plate according to [19].
[21] An antireflection film comprising the polarizing plate according to [19].
[22] A compound represented by any of the following formulas (XI-1) to (XI-6):

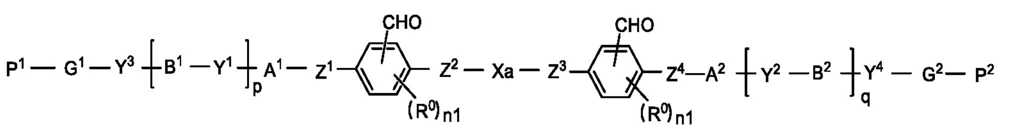

(XI-1)

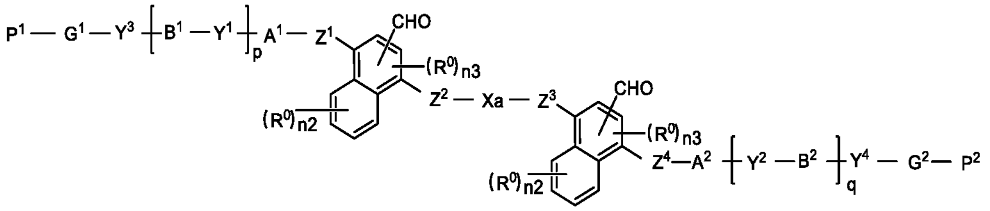

(XI-2)

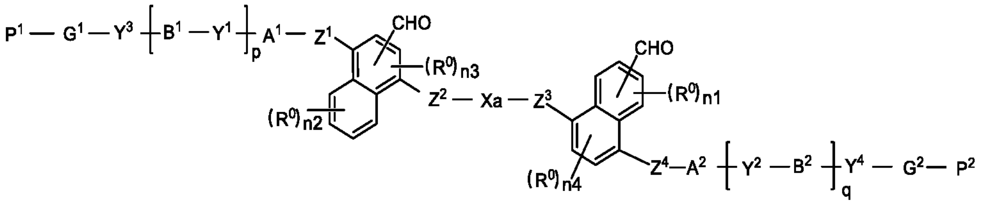

(XI-3)

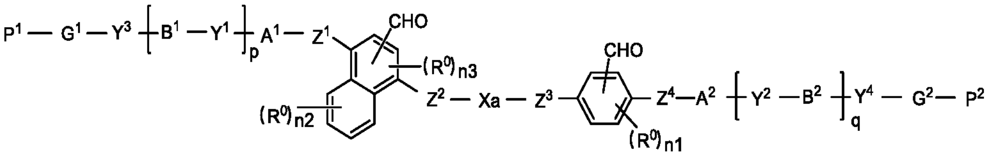

(XI-4)

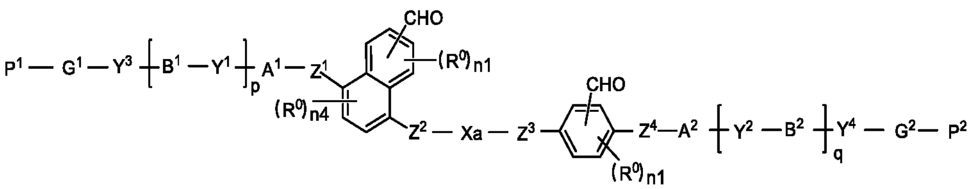

(XI-5)

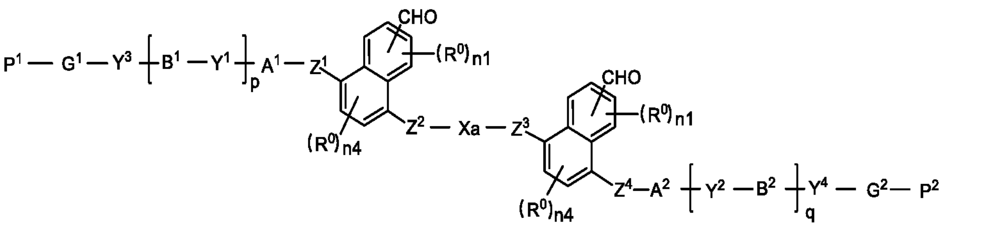

(XI-6)

where in the formulas (XI-1) to (XI-6),

Xa represents an organic group having 1 to 20 carbon atoms which may have a substituent,
$Z^1$ to $Z^4$ each independently represent a single bond, -O-, -O-$CH_2$-, -$CH_2$-O-, -O-$CH_2$-$CH_2$, -$CH_2$-$CH_2$-O-, -C(=O)-O-, -O-C(=O)-, -C(=O)-S-, -S-C(=O)-, -$NR^{20}$-C(=O)-, -C(=O)-$NR^{21}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-C(=O)-O-, -O-C(=O)-$CH_2$-, -$CH_2$-O-C(=O)-, -C(=O)-O-$CH_2$-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C≡C-, and $R^{20}$ represents a hydrogen atom or an alkyl group having 1 to 6 carbon atoms,
$A^1$ and $A^2$ each independently represent an aromatic group which may have a substituent,
$B^1$ and $B^2$ each independently represent a cyclic aliphatic group which may have a substituent, or, an aromatic group which may have a substituent,
$Y^1$ to $Y^4$ each independently represent a single bond, -O-, -C(=O)-, -C(=O)-O-, -O-C(=O)-, -$NR^{21}$-C(=O)-, -C(=O)-$NR^{21}$-, -O-C(=O)-O-, -$NR^{21}$-C(=O)-O-, -O-C(=O)-$NR^{21}$-, or, -$NR^{21}$-C(=O)-$NR^{22}$-, and $R^{21}$ and $R^{22}$ each independently represent a hydrogen atom or an alkyl group having 1 to 6 carbon atoms,
$G^1$ and $G^2$ each independently represent an organic group of either an alkylene group having 1 to 20 carbon atoms, or, an alkylene group having 3 to 20 carbon atoms in which at least one methylene group (-$CH_2$-) is substituted with -O- or -C(=O)-, and the hydrogen atom included in the organic group of $G^1$ and $G^2$ may be substituted with an alkyl group having 1 to 5 carbon atoms, an alkoxy group having 1 to 5 carbon atoms, or, a halogen atom, where the methylene group (-$CH_2$-) at each terminal of $G^1$ and $G^2$ is not substituted with -O- or

-C(=O)-,
one of $P^1$ and $P^2$ represents a hydrogen atom or a polymerizable group, and the other of $P^1$ and $P^2$ represents a polymerizable group,
p and q each independently represent an integer from 0 to 2, and
$R^0$ represents a halogen atom, a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N-N-dialkylamino group having 2 to 12 carbon atoms, an alkoxy group having 1 to 6 carbon atoms, a nitro group, -C(=O)-$R^a$, -O-C(=O)-$R^a$, -C(=O)-O-$R^a$, or -$SO_2R^a$, $R^a$ represents an alkyl group having 1 to 6 carbon atoms, or, an aromatic hydrocarbon ring group having 6 to 20 carbon atoms which may have an alkyl group having 1 to 6 carbon atoms or an alkoxy group having 1 to 6 carbon atoms as a substituent, and n1 represents an integer from 0 to 3, n2 represents an integer from 0 to 4, n3 is 0 or 1, and n4 represents an integer from 0 to 2,
where when a plurality of $R^0$, $B^1$, $B^2$, $Y^1$, and $Y^2$ are present, these may be the same or different.

[23] The compound according to [22] represented by any of the following formulas (XII-1) to (XII-3):

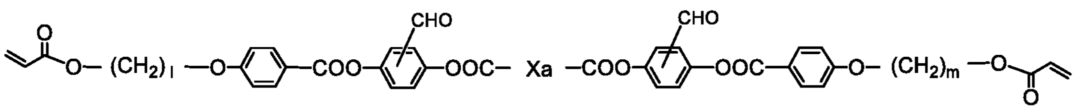

(XII-1)

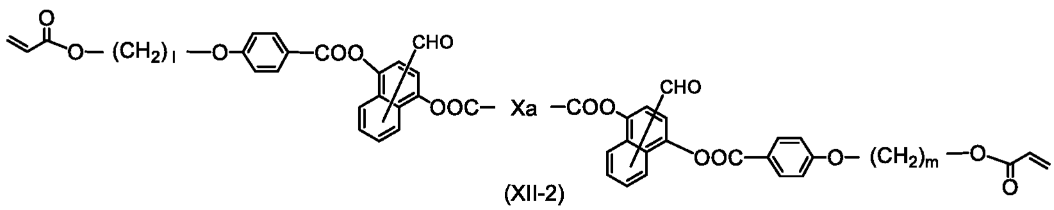

(XII-2)

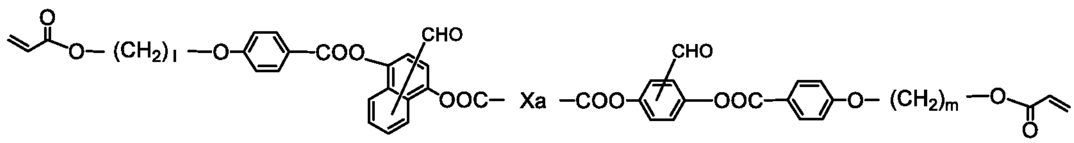

(XII-3)

where in the formulas (XII-1) to (XII-3),

Xa is the same as defined above, and
1 and m each independently represent an integer from 1 to 18.

(Advantageous Effect)

According to the present disclosure, a polymer capable of forming an optical film and an optically anisotropic body which has a good balance of lightness and saturation and which can improve reverse wavelength dispersion on the short wavelength side while achieving reverse wavelength dispersion on the longer wavelength side, and, a polymerizable compound and a polymerizable liquid crystal mixture useful in the preparation of the polymer are provided.
Further, according to the present disclosure, a compound useful in the preparation of the aforementioned polymerizable compound is provided.
Moreover, according to the present disclosure, an optical film and an optically anisotropic body which has a good balance of lightness and saturation and which can improve reverse wavelength dispersion on the short wavelength side while achieving reverse wavelength dispersion on the longer wavelength side, and, a polarizing plate, a display device, and an antireflection film using the optically anisotropic body are provided.

BRIEF DESCRIPTION OF THE DRAWING

In the accompanying drawing:

FIG. 1 is a diagram for explaining an assumed optical system used when calculating the difference between the retardation when the lightness of the optically anisotropic body of the present disclosure is the lowest and the retardation when the saturation is the lowest.

DETAILED DESCRIPTION

The present disclosure is described in detail as follows. As used herein, the phrase "may have a substituent" means "unsubstituted, or, having a substituent". Further, when an organic group such as an alkyl group or an aromatic hydrocarbon ring group contained in the general formula has a substituent, the number of carbon atoms of the organic group having the substituent does not include the number of carbon atoms of the substituent. For example, when the aromatic hydrocarbon ring group having 6 to 20 carbon atoms has a substituent, the number of carbon atoms of the aromatic hydrocarbon ring group having 6 to 20 does not include the number of carbon atoms of such a substituent. Furthermore, in the present disclosure, the phrase "alkyl group" means a chain (linear or branched) saturated hydrocarbon group, and the "alkyl group" does not include a "cycloalkyl group" which is a cyclic saturated hydrocarbon group.

Here, the polymerizable compound and the polymerizable liquid crystal mixture of the present disclosure are not specifically limited, and can be used, for example, when preparing the polymer of the present disclosure.

Furthermore, the polymer of the present disclosure is not specifically limited, and can be used, for example, as a constituent material of the optical film of the present disclosure and as a constituent material of a layer of the optically anisotropic body of the present disclosure. Further, the optically anisotropic body of the present disclosure is not specifically limited, and can be used in, for example, the production of a polarizing plate of the present disclosure. Furthermore, the polarizing plate of the present disclosure is not specifically limited, and can be used in, for example, the production of a display device and an antireflection film of the present disclosure.

Further, the compound (intermediate) of the present disclosure is not specifically limited, and can be used, for example, when preparing the polymerizable compound of the present disclosure.

Polymerizable compound

The polymerizable compound of the present disclosure is a compound represented by the following formula (A), preferably a compound represented by the following formula (I-1) (which may be hereinafter referred to as the "polymerizable compound (1-1)"), and can be advantageously used in the preparation of the polymer, the optical film and the optically anisotropic body which are described later. Note that $Z^1$ to $Z^4$, $A^1$, $A^2$, $Ar^0$, $Ar^1$ and Xa of formula (A) are respectively the same as defined above for $Z^1$ to $Z^4$, $A^1$, $A^2$, $Ar^0$, $Ar^1$ and Xa of the formula (I-1), and at least one of the organic groups in the formula (A), and preferably both organic groups have a polymerizable group at the terminal.

$$\text{organic group} -A^1-Z^1-Ar^0-Z^2-Xa-Z^3-Ar^1-Z^4-A^2- \text{organic group} \qquad \text{(A)}$$

$$P^1-G^1-Y^3\left[B^1-Y^1\right]_p A^1-Z^1-Ar^0-Z^2-Xa-Z^3-Ar^1-Z^4-A^2\left[Y^2-B^2\right]_q Y^4-G^2-P^2$$

**(I-1)**

Note that as described later an optical film which has a good balance of lightness and saturation and which can improve reverse wavelength dispersion on the short wavelength side while achieving reverse wavelength dispersion on the longer wavelength side can be advantageously produced using the compound represented by the formula (A), specifically, the polymerizable compound (I-1).

Here, in the formula (I-1), $Ar^0$ represents an aromatic hydrocarbon ring group having at least $D^0$ as a substituent, or, an aromatic heterocyclic ring group having at least $D^0$ as a substituent.

Further, $Ar^1$ represents an aromatic hydrocarbon ring group having at least $D^1$ as a substituent, or, an aromatic heterocyclic ring group having at least $D^1$ as a substituent.

Here, $D^0$ and $D^1$ each independently represent an organic group having 1 to 67 carbon atoms, preferably having 2 to 67 carbon atoms, and having at least one aromatic ring selected from the group consisting of an aromatic hydrocarbon ring and an aromatic heterocyclic ring. Namely, $D^0$ and $D^1$ may respectively consist of only an aromatic ring, or may consist of an organic group having an aromatic ring.

Moreover, examples of the aromatic hydrocarbon ring group of $Ar^0$ and $Ar^1$ include a 1,4-phenylene group, a 1,3-phenylene group, a 1,4-naphthalene group, a 2,6-naphthalene group, a 1,5-naphthalene group, an anthracenyl-9,10-diyl group, an anthracenyl-1,4-diyl group, and an anthracenyl-2,6-diyl group.

Thereamong, as the aromatic hydrocarbon ring group, a 1,4-phenylene group, 1,4-naphthalene group, or a 2,6-naphthalene group is preferable, and a 1,4-phenylene group is particularly preferable.

Further, examples of the aromatic heterocyclic ring group of $Ar^0$ and $Ar^1$ include a benzothiazole-4,7-diyl group, a 1,2-benzoisothiazole-4,7-diyl group, a benzoxazole-4,7-diyl group, indole-4,7-diyl group, a benzimidazole-4,7-diyl group, a benzopyrazole-4,7-diyl group, a 1-benzofuran-4,7-diyl group, a 2-benzofuran-4,7-diyl group, a benzo[1,2-d:4,5-d']dithiazolyl-4,8-diyl group, a benzo[1,2-d:5,4-d']dithiazolyl-4,8-diyl group, a benzothiophenyl-4,7-diyl group, a 1H-isoindole-1,3 (2H)-dione-4,7-diyl group, a benzo[1,2-b:5,4-b']dithiophenyl-4,8-diyl group, a benzo[1,2-b:4,5-b']dithiophenyl-4,8-diyl group, a benzo[1,2-b:5,4-b']difuranyl-4,8-diyl group, a benzo[1,2-b:4,5-b']difuranyl-4,8-diyl group, a benzo[2,1-b:4,5-b']dipyrrole-4,8-diyl group, a benzo[1,2-b:5,4-b']dipyrrole-4,8-diyl group, and a benzo[1,2-d:4,5-d']diimidazole-4,8-diyl group.

Thereamong, preferred as the aromatic heterocyclic ring group are: a benzothiazole-4,7-diyl group, a benzoxazole-4,7-diyl group, a 1-benzofuran-4,7-diyl group, a 2-benzofuran-4,7-diyl group, a benzo[1,2-d:4,5-d']dithiazolyl-4,8-diyl group, a benzo[1,2-d:5,4-d']dithiazolyl-4,8-diyl group, a benzothiophenyl-4,7-diyl group, a 1H-isoindole-1,3 (2H)-dione-4,7-diyl group, a benzo[1,2-b:5,4-b']dithiophenyl-4,8-diyl group, a benzo[1,2-b:4,5-b']dithiophenyl-4,8-diyl group, a benzo[1,2-b:5,4-b']difuranyl-4,8-diyl group, and a benzo[1,2-b:4,5-b']difuranyl-4,8-diyl group.

The aromatic hydrocarbon ring group and the aromatic heterocyclic ring group of $Ar^0$ and $Ar^1$ may have a substituent $R^0$ which is described later in addition to $D^0$ and $D^1$.

Further, in the present disclosure, the term "aromatic ring" refers to a cyclic structure that exhibits aromaticity in a broad sense according to Huckel's rule, i.e., a cyclic conjugated structure that includes (4n+2) $\pi$ electrons, and a cyclic structure that exhibits aromaticity in which lone pairs of hetero atoms such as sulfur, oxygen, nitrogen are involved in the $\pi$ electron system, such as thiophene, furan, and benzothiazole.

Further, the number of $\pi$ electrons contained in $Ar^0$ and $Ar^1$ is normally 12 or more, preferably 12 to 36, and more preferably 12 to 30.

Note that examples of the aromatic hydrocarbon ring of $D^0$ and $D^1$ include a benzene ring, a naphthalene ring, an anthracene ring, a phenanthrene ring, a pyrene ring, and a fluorene ring.

Thereamong, an aromatic hydrocarbon ring is preferably a benzene ring, a naphthalene ring, or an anthracene ring.

Further, examples of the aromatic heterocyclic ring of $D^0$ and $D^1$ include a 1H-isoindole-1,3 (2H)-dione ring, a 1-benzofuran ring, a 2-benzofuran ring, an acridine ring, an isoquinoline ring, an imidazole ring, an indole ring, an oxadiazole ring, an oxazole ring, an oxazolopyrazine ring, an oxazolopyridine ring, an oxazolopyridazine ring, an oxazolopyrimidine ring, a quinazoline ring, a quinoxaline ring, a quinoline ring, a cinnoline ring, a thiadiazole ring, a thiazole ring, a thiazolopyrazine ring, a thiazolopyridine ring, a thiazolopyridazine ring, a thiazolopyrimidine ring, a thiophene ring, a triazine ring, a triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, and a benzopyranone ring.

Thereamong, preferred as the aromatic heterocyclic ring are a monocyclic aromatic heterocyclic ring such as a furan ring, a pyran ring, a thiophene ring, an oxazole ring, an oxadiazole ring, a thiazole ring, and a thiadiazole ring; a fused aromatic heterocyclic ring such as a benzothiazole ring, a benzoxazole ring, a quinoline ring, a 1-benzofuran ring, a 2-benzofuran ring, a benzothiophene ring, a 1H-isoindole-1,3 (2H)-dione ring, a benzo[c]thiophene ring, a thiazolopyridine ring, a thiazolopyrazine ring, a benzoisoxazole ring, a benzoxadiazole ring, and a benzothiadiazole ring.

Moreover, examples of the organic group having 1 to 67 carbon atoms having at least one aromatic ring selected from the group consisting of an aromatic hydrocarbon ring and an aromatic heterocyclic ring serving as $D^0$ or $D^1$ include, but is not specifically limited to, an aromatic hydrocarbon ring group which may have a substituent, an aromatic heterocyclic ring group which may have a substituent, and a group represented by formula: $-C(R^f)=N-N(R^g)R^h$, or, formula:

$$-C(R^f)=N-N=C(R^{g1})R^h.$$

Note that in the aforementioned formulas, $R^f$ represents a hydrogen atom or an alkyl group having 1 to 6 carbon atoms such as a methyl group, an ethyl group, a propyl group, or an isopropyl group.

Further, in the aforementioned formulas, $R^g$ and $R^{g1}$ each independently represent a hydrogen atom, or an organic group having 1 to 30 carbon atoms which may have a substituent. Here, examples of the organic group of 1 to 30 carbon atoms and the substituent thereof include the same as those listed as the specific examples of the organic group having 1 to 30 carbon atoms and the substituent thereof of Ay which is described later.

Furthermore, in the aforementioned formulas, $R^h$ represents an organic group having at least one aromatic ring selected from the group consisting of an aromatic hydrocarbon ring having 6 to 30 carbon atoms and an aromatic heterocyclic ring having 2 to 30 carbon atoms. Here, specific examples of the organic group having at least one aromatic ring selected from the group consisting of an aromatic hydrocarbon ring having 6 to 30 carbon atoms and an aromatic heterocyclic ring having 2 to 30 carbon atoms include the same as those listed as the specific examples of the organic group having at least one aromatic ring selected from the group consisting of an aromatic hydrocarbon ring having 6 to 30 carbon atoms and an aromatic heterocyclic ring having 2 to 30 carbon atoms of Ax which is described later.

Specifically, examples of the aromatic hydrocarbon ring group serving as $D^0$ or $D^1$ include a phenyl group, a naphthyl group, an anthracenyl group, a phenanthrenyl group, a pyrenyl group, and a fluorenyl group.

Thereamong, a phenyl group and an anthracenyl group are preferable as the aromatic hydrocarbon ring group.

Further, examples of the aromatic heterocyclic ring group serving as $D^0$ or $D^1$ include, a phthalimide group, a 1-benzofuranyl group, a 2-benzofuranyl group, an acridinyl group, an isoquinolinyl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxadiazolyl group, a benzoxazolyl group, a benzothiadiazolyl group, a benzothiazolyl group, a benzothienyl group, a benzotriazinyl group, a benzotriazolyl group, a benzopyrazolyl group, and a benzopyranonyl group.

Thereamong, preferred as the aromatic heterocyclic ring group are: a monocyclic aromatic heterocyclic ring group such as a furanyl group, a pyranyl group, a thienyl group, an oxazolyl group, a furazanyl group, a thiazolyl group, and a thiadiazolyl group; and a fused aromatic heterocyclic ring group such as a benzothiazolyl group, a benzoxazolyl group, a quinolyl group, a 1-benzofuranyl group, a 2-benzofuranyl group, a benzothienyl group, a phthalimide group, a benzo[c]thienyl group, a thiazolopyridyl group, a thiazolopyrazinyl group, a benzoisoxazolyl group, a benzoxadiazolyl group, and a benzothiadiazolyl group.

The aromatic hydrocarbon ring and an aromatic heterocyclic ring of $D^0$ and $D^1$, and, the aromatic hydrocarbon ring group and the aromatic heterocyclic ring group serving as $D^0$ or $D^1$ may have a substituent.

Examples of the substituent include: a halogen atom such as a fluorine atom and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group; a N-N-dialkylamino group having 1 to 12 carbon atoms such as a dimethylamino group; an alkoxy group having 1 to 6 carbon atoms such as a methoxy group, an ethoxy group and an isopropoxy group; a nitro group; an aromatic hydrocarbon ring group having 6 to 20 carbon atoms such as a phenyl group and a naphthyl group; $-OCF_3$; $-C(=O)-R^{b1}$; $-O-C(=O)-R^{b1}$; $-C(=O)-O-R^{b1}$; and $-SO_2R^a$.

Here, $R^{b1}$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or, an aromatic hydrocarbon ring group having 5 to 18 carbon atoms which may have a substituent.

Further, $R^a$ represents: an alkyl group having 1 to 6 carbon atoms such as a methyl group and an ethyl group; or an aromatic hydrocarbon ring group having 6 to 20 carbon atoms which may have an alkyl group having 1 to 6 carbon atoms or an alkoxy group having 1 to 6 carbon atoms as a substituent, such as a phenyl group, a 4-methylphenyl group, or a 4-methoxyphenyl group.

Thereamong, examples of the substituents of the aromatic hydrocarbon ring and the aromatic heterocyclic ring of $D^0$ and $D^1$, and, the substituents of the aromatic rings of the aromatic hydrocarbon ring group and the aromatic heterocyclic ring group serving as $D^0$ or $D^1$ are preferably a halogen atom, a cyano group, a nitro group, an alkyl group having 1 to 6 carbon atoms, and, an alkoxy group having 1 to 6 carbon atoms, and an alkyl group having 1 to 6 carbon atoms in which at least one hydrogen atom is substituted with a halogen atom.

Note that the aromatic hydrocarbon ring and the aromatic heterocyclic ring of $D^0$ and $D^1$, and, the aromatic hydrocarbon ring group and the aromatic heterocyclic ring group serving as $D^0$ or $D^1$ may have a plurality of substituents selected from the aforementioned substituents. When the aromatic hydrocarbon ring and the aromatic heterocyclic ring, and the aromatic hydrocarbon ring group and the aromatic hydrocarbon ring group have a plurality of substituents, the substituents may be the same or different.

Specific examples of an alkyl group having 1 to 20 carbon atoms and the substituent thereof in the case when $R^{b1}$ is an alkyl group having 1 to 20 carbon atoms which may have a substituent, an alkenyl group having 2 to 20 carbon atoms and the substituent thereof in the case when $R^{b1}$ is an alkenyl group having 2 to 20 carbon atoms which may have a substituent, a cycloalkyl group having 3 to 12 carbon atoms and the substituent thereof in the case when $R^{b1}$ is

a cycloalkyl group having 3 to 12 carbon atoms which may have a substituent, and an aromatic hydrocarbon ring group having 5 to 18 carbon atoms and the substituent thereof in the case when $R^{b1}$ is an aromatic hydrocarbon ring group having 5 to 18 carbon atoms which may have a substituent are the same as the specific examples of an alkyl group having 1 to 20 carbon atoms and the substituent thereof in the case when $R^b$ is an alkyl group having 1 to 20 carbon atoms which may have a substituent, an alkenyl group having 2 to 20 carbon atoms and the substituent thereof in the case when $R^b$ is an alkenyl group having 2 to 20 carbon atoms which may have a substituent, a cycloalkyl group having 3 to 12 carbon atoms and the substituent thereof in the case when $R^b$ is a cycloalkyl group having 3 to 12 carbon atoms which may have a substituent, and an aromatic hydrocarbon ring group having 5 to 18 carbon atoms and the substituent thereof in the case when $R^b$ is an aromatic hydrocarbon ring group having 5 to 18 carbon atoms which may have a substituent, and are described later.

Moreover, the aforementioned $Ar^0$ and $Ar^1$ each independently represent, for example, a phenylene group substituted with a group represented by formula: $-C(R^f)=N-N(R^g)R^h$ or formula: $-C(R^f)=N-N=C(R^{g1})R^h$, a benzothiazole-4,7-diyl group substituted with a 1-benzofuran-2-yl group, a benzothiazole-4,7-diyl group substituted with a 5-(2-butyl)-1-benzofuran-2-yl group, a benzothiazole-4,7-diyl group substituted with a 4,6-dimethyl-1-benzofuran-2-yl group, a benzothiazole-4,7-diyl group substituted with a 6-methyl-1-benzofuran-2-yl group, a benzothiazole-4,7-diyl group substituted with a 4,6,7-trimethyl-1-benzofuran-2-yl group, a benzothiazole-4,7-diyl group substituted with a 4,5,6-trimethyl-1-benzofuran-2-yl group, a benzothiazole-4,7-diyl group substituted with a 5-methyl-1-benzofuran-2-yl group, a benzothiazole-4,7-diyl group substituted with a 5-propyl-1-benzofuran-2-yl group, a benzothiazole-4,7-diyl group substituted with a 7-propyl-1-benzofuran-2-yl group, a benzothiazole-4,7-diyl group substituted with a 5-fluro-1-benzofuran-2-yl group, a benzothiazole-4,7-diyl group substituted with a phenyl group, a benzothiazole-4,7-diyl group substituted with a 4-fluorophenyl group, a benzothiazole-4,7-diyl group substituted with a 4-nitrophenyl, a benzothiazole-4,7-diyl group substituted with a 4-trifluoromethylphenyl group, a benzothiazole-4,7-diyl group substituted with a 4-cyanophenyl group, a benzothiazole-4,7-diyl group substituted with a 4-methanesulfonylphenyl group, a benzothiazole-4,7-diyl group substituted with a thiophene-2-yl group, a benzothiazole-4,7-diyl group substituted with a thiophene-3-yl group, a benzothiazole-4,7-diyl group substituted with a 5-methylthiophene-2-yl group, a benzothiazole-4,7-diyl group substituted with a 5-chlorothiophene-2-yl group, a benzothiazole-4,7-diyl group substituted with a thieno[3,2-b]thiophene-2-yl group, a benzothiazole-4,7-diyl group substituted with a 2-benzothiazolyl group, a benzothiazole-4,7-diyl group substituted with a 4-biphenyl group, a benzothiazole-4,7-diyl group substituted with a 4-propylbiphenyl group, a benzothiazole-4,7-diyl group substituted with a 4-thiazolyl group, a benzothiazole-4,7-diyl group substituted with a 1-phenylethylene-2-yl group, a benzothiazole-4,7-diyl group substituted with a 4-pyridyl group, a benzothiazole-4,7-diyl group substituted with a 2-furyl group, a benzothiazole-4,7-diyl group substituted with a naphtho[1,2-b]furan-2-yl group, a 1H-isoindole-1,3(2H)-dione-4,7-diyl group substituted with a 5-methoxy-2-benzothiazolyl group, a 1H-isoindole-1,3 (2H)-dione-4,7-diyl group substituted with a phenyl group, a 1H-isoindole-1,3 (2H)-dione-4,7-diyl group substituted with a 4-nitrophenyl, or a 1 H-isoindole-1,3 (2H)-dione-4,7-diyl group substituted with a 2-thiazolyl group. Here, $R^f$, $R^g$, $R^{g1}$ and $R^h$ are the same as defined above.

Here, a group independently represented by any of the following formulas (II-1) to (II-7) is preferable as $Ar^0$ and $Ar^1$.

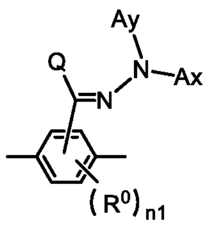

(II-1)

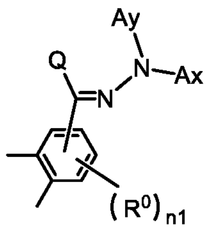

(II-2)

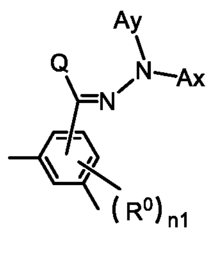

(II-3)

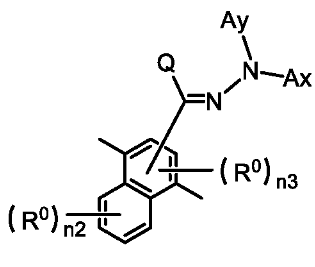

(II-4)

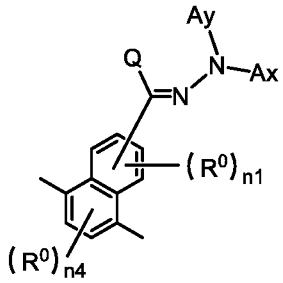
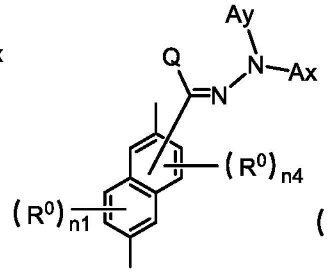
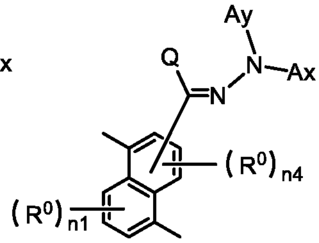

(II-5) (II-6) (II-7)

In the aforementioned formulas (II-1) to (II-7), Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring of Ax may have a substituent, Ay represents a hydrogen atom or an organic group having 1 to 30 carbon atoms which may have a substituent, and Q represents a hydrogen atom or an alkyl group having 1 to 6 carbon atoms. Here, examples of the alkyl group having 1 to 6 carbon atoms of Q include a methyl group, an ethyl group, an n-propyl group, and an isopropyl group.

Further, $R^0$ represents: a halogen atom such as a fluorine atom, a chlorine atom, and a bromine atom; a cyano group; an alkyl group having 1 to 6 carbon atoms such as a methyl group, an ethyl group, a propyl group, an isopropyl group, a butyl group, a sec-butyl group, and a tertiary butyl group; an alkenyl group having 2 to 6 carbon atoms; an alkyl group having 1 to 6 carbon atoms in which at least one hydrogen atom is substituted with a halogen atom, an N-N-dialkylamino group having 2 to 12 carbon atoms; an alkoxy group having 1 to 6 carbon atoms such as a methoxy group and an isopropoxy group; a nitro group; $-C(=O)-R^a$; $-O-C(=O)-R^a$; $-C(=O)-O-R^a$; or $-SO_2R^a$, $R^a$ represents an alkyl group having 1 to 6 carbon atoms such as a methyl group and an ethyl group, or, an aromatic hydrocarbon ring group having 6 to 20 carbon atoms which may have an alkyl group having 1 to 6 carbon atoms or an alkoxy group having 1 to 6 carbon atoms as a substituent, such as a phenyl group, a 4-methylphenyl group, or a 4-methoxyphenyl group. When there is a plurality of substituents, the plurality of substituents may be the same or different from each other. From the viewpoint of solubility improvement, $R^0$ is preferably a halogen atom, a cyano group, an alkyl group having 1 to 6 carbon atoms, an alkyl group having 1 to 6 carbon atoms in which at least one hydrogen atom is substituted with a halogen atom, an alkoxy group having 1 to 6 carbon atoms, or a nitro group.

Furthermore, n1 represents an integer from 0 to 3, n2 represents an integer from 0 to 4, n3 is 0 or 1, and n4 represents an integer from 0 to 2. Moreover, it is preferable that n1 = 0, n2 = 0, n3 = 0 and n4 = 0.

Note that the configurations represented by the following formulas (ii-1) to (ii-21) are furthermore independently preferable as $Ar^0$ and $Ar^1$. Note that the adjacent $Z^n$ (in the case of $Ar^0$, n = 1 or 2, in the case of $Ar^1$, n = 3 or 4, and the following formulas illustrate the cases when n = 1 or 2 as representative examples) are described for the sake of convenience to clarify the bonding state in the following formulas. In the formulas, Ax, Ay, Q, $R^0$, n1, n2, n3 and n4 are defined as stated above, and preferred examples thereof are also the same as stated above. Thereamong, formulas (ii-1), (ii-2) (ii-10) and (ii-12) are particularly preferable.

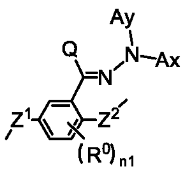
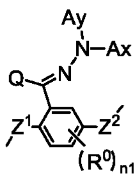
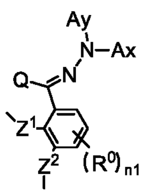
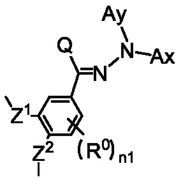
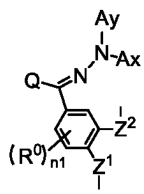
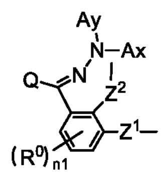
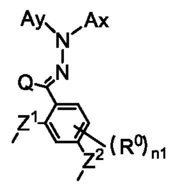

(ii-1) (ii-2) (ii-3) (ii-4) (ii-5) (ii-6) (ii-7)

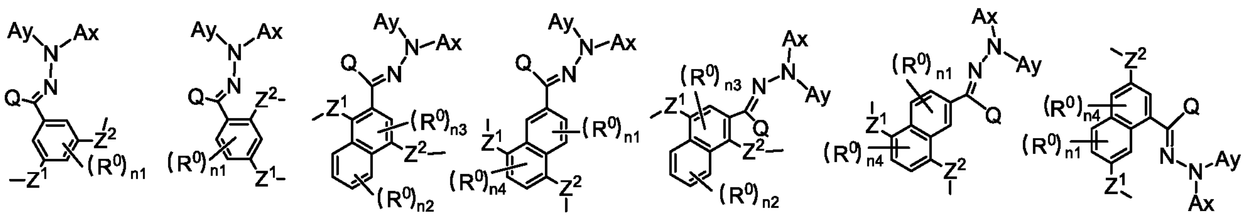

(ii-8) (ii-9) (ii-10) (ii-11) (ii-12) (ii-13) (ii-14)

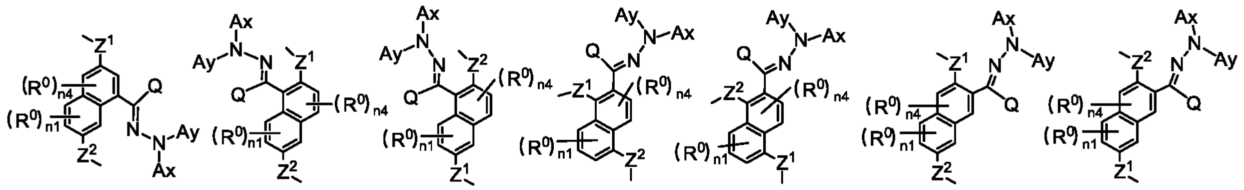

(ii-15) (ii-16) (ii-17) (ii-18) (ii-19) (ii-20) (ii-21)

The organic group having at least one aromatic ring selected from the group consisting of an aromatic hydrocarbon ring of Ax having 6 to 30 carbon atoms and an aromatic heterocyclic ring having 2 to 30 carbon atoms may have a plurality of aromatic rings, or may have an aromatic hydrocarbon ring and an aromatic heterocyclic ring. Further, when there are a plurality of aromatic hydrocarbon rings and a plurality of aromatic heterocyclic rings, each may be the same or different.

Note that examples of the aromatic hydrocarbon ring of Ax include a benzene ring, a naphthalene ring, an anthracene ring, a phenanthrene ring, a pyrene ring, and a fluorene ring.

Thereamong, a benzene ring, a naphthalene ring or an anthracene ring is preferable as the aromatic hydrocarbon ring.

Further, examples of the aromatic heterocyclic ring of Ax include a 1H-isoindole-1,3 (2H)-dione ring, a 1-benzofuran ring, a 2-benzofuran ring, an acridine ring, an isoquinoline ring, an imidazole ring, an indole ring, an oxadiazole ring, an oxazole ring, an oxazolopyrazine ring, an oxazolopyridine ring, an oxazolopyridazine ring, an oxazolopyrimidine ring, a quinazoline ring, a quinoxaline ring, a quinoline ring, a cinnoline ring, a thiadiazole ring, a thiazole ring, a thiazolopyrazine ring, a thiazolopyridine ring, a thiazolopyridazine ring, a thiazolopyrimidine ring, a thiophene ring, a triazine ring, a triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, a benzopyranone ring, a dihydropyran ring, a tetrahydropyran ring, a dihydrofuran ring, and a tetrahydrofuran ring.

Thereamong, preferred as the aromatic heterocyclic ring are: a monocyclic aromatic heterocyclic ring such as a furan ring, a pyran ring, a thiophene ring, an oxadiazole ring, a thiazole ring, and a thiadiazole ring; and a fused aromatic heterocyclic ring such as a benzothiazole ring, a benzoxazole ring, a quinoline ring, a 1-benzofuran ring, a 2-benzofuran ring, a benzothiophene ring, a 1H-isoindole-1,3 (2H)-dione ring, a benzo[c]thiophene ring, a thiazolopyridine ring, a thiazolopyrazine ring, a benzoisoxazole ring, a benzoxadiazole ring, and a benzothiadiazole ring.

The aromatic ring of Ax may have a substituent. Examples of such a substituent include: a halogen atom such as a fluorine atom and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group; an N-N-dialkylamino group having 2 to 12 carbon atoms such as a dimethylamino group; an alkoxy group having 1 to 6 carbon atoms such as a methoxy group, an ethoxy group and an isopropoxy group; a nitro group; an aromatic hydrocarbon ring group having 6 to 20 carbon atoms such as a phenyl group and a naphthyl group; $-OCF_3$; $-C(=O)-R^b$; $-O-C(=O)-R^b$; $-C(=O)-O-R^b$; and $-SO_2R^a$. Here, $R^b$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring group having 5 to 18 carbon atoms and an aromatic heterocyclic ring group having 2 to 18 carbon atoms which may have a substituent. Further, $R^a$ is the same as defined above. Thereamong, preferred as the substituents of the aromatic ring of Ax are a halogen atom, a cyano group, an alkyl group having 1 to 6 carbon atoms, and an alkoxy group having 1 to 6 carbon atoms.

Note that Ax may have a plurality of substituents selected from the aforementioned substituents. When Ax has a plurality of substituents, the substituents may be the same or different.

Examples of the alkyl group having 1 to 20 carbon atoms in the case when $R^b$ is an alkyl group having 1 to 20 carbon atoms which may have a substituent include a methyl group, an ethyl group, an n-propyl group, an isopropyl group, an n-butyl group, an isobutyl group, a 1-methylpentyl group, a 1-ethylpentyl group, a sec-butyl group, a t-butyl group, an n-pentyl group, an isopentyl group, a neopentyl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, and an n-icosyl group. Note that the number of carbon atoms of the alkyl group having 1 to 20 carbon atoms which may have a substituent is preferably 1 to 12, and even more preferably 4 to 10.

Examples of the alkenyl group having 2 to 20 carbon atoms in the case when $R^b$ is an alkenyl group having 2 to 20 carbon atoms which may have a substituent include a vinyl group, a propenyl group, an isopropenyl group, a butenyl group, an isobutenyl group, a pentenyl group, a hexenyl group, a heptenyl group, an octenyl group, a nonenyl group, a decenyl group, an undecenyl group, a dodecenyl group, a tridecenyl group, a tetradecenyl group, a pentadecenyl group, a hexadecenyl group, a heptadecenyl group, an octadecenyl group, a nonadenyl group, and an icosenyl group.

The number of carbons of the alkenyl group having 2 to 20 carbon atoms which may have a substituent is preferably 2 to 12.

Examples of the substituent in the case when $R^b$ is the alkyl group having 1 to 20 carbon atoms or is the alkenyl group having 2 to 20 carbon atoms include: a halogen atom such as a fluorine atom and a chlorine atom; a cyano group; an N-N-dialkylamino group having 2 to 12 carbon atoms such as a dimethylamino group; an alkoxy group having 1 to 20 carbon atoms such as a methoxy group, an ethoxy group, an isopropoxy group, and a butoxy group; an alkoxy group having 1 to 12 carbon atoms substituted with an alkoxy group having 1 to 12 carbon atoms such as a methoxymethoxy group and a methoxyethoxy group; a nitro group; an aromatic hydrocarbon ring group having 6 to 20 carbon atoms such as a phenyl group and a naphthyl group; an aromatic heterocyclic ring group having 2 to 20 carbon atoms such as a triazolyl group, a pyrrolyl group, a furanyl group, a thiophenyl group and a benzothiazole-2-ylthio group; a cycloalkyl group having 3 to 8 carbon atoms such as a cyclopropyl group, a cyclopentyl group and a cyclohexyl group; a cycloalkyloxy group having 3 to 8 carbon atoms such as a cyclopentyloxy group and a cyclohexyloxy group; a cyclic ether group having 2 to 12 carbon atoms such as a tetrahydrofuranyl group, a tetrahydropyranyl group, a dioxolanyl group and a dioxanyl group; an aryloxy group having 6 to 14 carbon atoms such as a phenoxy group and a naphthoxy group; a fluoroalkyl group having 1 to 12 carbon atoms in which at least one hydrogen atom is substituted with a fluorine atom, such as a trifluoromethyl group, a pentafluoroethyl group, and $-CH_2CF_3$; a benzofuryl group; a benzopyranyl group; a benzodioxolyl group; and a benzodioxanyl group. Thereamong, the substituent in the cases when $R^b$ is an alkyl group having 1 to 20 carbon atoms or is an alkenyl group having 2 to 20 carbon atoms is preferably a halogen atom such as a fluorine atom and a chlorine atom; a cyano group; an alkoxy group having 1 to 20 carbon atoms such as a methoxy group, an ethoxy group, an isopropoxy group, and a butoxy group; a nitro group; an aromatic hydrocarbon ring group having 6 to 20 carbon atoms such as a phenyl group and a naphthyl group; an aromatic heterocyclic ring group having 2 to 20 carbon atoms such as a furanyl group and a thiophenyl group; a cycloalkyl group having 3 to 8 carbon atoms such as a cyclopropyl group, a cyclopentyl group and a cyclohexyl group; or a fluoroalkyl group having 1 to 12 carbon atoms in which at least one hydrogen atom is substituted with a fluorine atom such as a trifluoromethyl group, a pentafluoroethyl group, and $-CH_2CF_3$.

Note that in the cases when $R^b$ is the alkyl group having 1 to 20 carbon atoms or the alkenyl group having 2 to 20 carbon atoms, it may have a plurality of substituents selected from the aforementioned substituents. When the alkyl group having 1 to 20 carbon atoms or the alkenyl group having 2 to 20 carbon atoms of $R^b$ has a plurality of substituents, the plurality of substituents may be the same or different.

Examples of the cycloalkyl group having 3 to 12 carbon atoms in the case when $R^b$ is a cycloalkyl group having 3 to 12 carbon atoms which may have a substituent include a cyclopropyl group, a cyclobutyl group, a cyclopentyl group, a cyclohexyl group, and a cyclooctyl group. Thereamong, a cyclopentyl group and a cyclohexyl group are preferable.

Examples of the substituent in the case when $R^b$ is a cycloalkyl group having 3 to 12 carbon atoms include: a halogen atom such as a fluorine atom and a chlorine atom; a cyano group; an N-N-dialkylamino group having 2 to 12 carbon atoms such as a dimethylamino group; an alkyl group having 1 to 6 carbon atoms such as a methyl group, an ethyl group, and a propyl group; an alkoxy group having 1 to 6 carbon atoms such as a methoxy group, an ethoxy group. and an isopropoxy group; a nitro group; and an aromatic hydrocarbon ring group having 6 to 20 carbon atoms such as a phenyl group and a naphthyl group. Thereamong, the substituent in the case when $R^b$ is a cycloalkyl group having 3 to 12 carbon atoms is preferably a halogen atom such as a fluorine atom and a chlorine atom; a cyano group; an alkyl group having 1 to 6 carbon atoms such as a methyl group, an ethyl group, and a propyl group; an alkoxy group having 1 to 6 carbon atoms such as a methoxy group, an ethoxy group and an isopropoxy group; a nitro group; and an aromatic hydrocarbon ring group having 6 to 20 carbon atoms such as a phenyl group and a naphthyl group.

Note that when $R^b$ is a cycloalkyl group having 3 to 12 carbon atoms, there may be a plurality of substituents.

When the cycloalkyl group having 3 to 12 carbon atoms of $R^b$ has a plurality of substituents, the plurality of substituents may be the same or different.

Examples of an aromatic hydrocarbon ring group having 5 to 18 carbon atoms in the case when $R^b$ is an aromatic hydrocarbon ring group having 5 to 18 carbon atoms which may have a substituent include a phenyl group, a 1-naphthyl group, and a 2-naphthyl group. Thereamong, a phenyl group is preferable.

Examples of the substituents of an aromatic hydrocarbon ring group having 5 to 18 carbon atoms which may have a substituent include: a halogen atom such as a fluorine atom and a chlorine atom; a cyano group; an N-N-dialkylamino group having 2 to 12 carbon atoms such as a dimethylamino group; an alkoxy group having 1 to 20 carbon atoms such as a methoxy group, an ethoxy group, an isopropoxy group, and a butoxy group; an alkoxy group having 1 to 12 carbon atoms substituted with an alkoxy group having 1 to 12 carbon atoms such as a methoxymethoxy group and a methoxyethoxy group; a nitro group; an aromatic hydrocarbon ring group having 6 to 20 carbon atoms such as a phenyl group and a naphthyl group; an aromatic heterocyclic ring group having 2 to 20 carbon atoms such as a triazolyl group, a pyrrolyl group, a furanyl group and a thiophenyl group; a cycloalkyl group having 3 to 8 carbon atoms such as a cyclopropyl group, a cyclopentyl group and a cyclohexyl group; a cycloalkyloxy group having 3 to 8 carbon atoms such as a cyclopentyloxy group and a cyclohexyloxy group; a cyclic ether group having 2 to 12 carbon atoms such as a tetrahydrofuranyl group, a tetrahydropyranyl group, a dioxolanyl group, and a dioxanyl group; an aryloxy group having 6 to 14 carbon atoms such as a phenoxy group and a naphthoxy group; a fluoroalkyl group having 1 to 12 carbon atoms in which at least one hydrogen atom is substituted with a fluorine atom such as a trifluoromethyl group, a pentafluoroethyl group, and $-CH_2CF_3$; $-OCF_3$; a benzofuryl group; a benzopyranyl group; a benzodioxolyl group; and a benzodioxanyl group. Thereamong, the substituent of an aromatic hydrocarbon ring group having 5 to 18 carbon atoms is preferably at least one substituent selected from: a halogen atom such as a fluorine atom and a chlorine atom; a cyano group; an alkoxy group having 1 to 20 carbon atoms such as a methoxy group, an ethoxy group, an isopropoxy group, and a butoxy group; a nitro group; an aromatic hydrocarbon ring group having 6 to 20 carbon atoms such as a phenyl group and a naphthyl group; an aromatic heterocyclic ring group having 2 to 20 carbon atoms such as a furanyl group and a thiophenyl group; a cycloalkyl group having 3 to 8 carbon atoms such as a cyclopropyl group, a cyclopentyl group and a cyclohexyl group; a fluoroalkyl group having 1 to 12 carbon atoms in which at least one hydrogen atom is substituted with a fluorine atom such as a trifluoromethyl group, a pentafluoroethyl group, and $-CH_2CF_3$; or $-OCF_3$.

Examples of an aromatic heterocyclic ring group having 2 to 18 carbon atoms in the case when $R^b$ is an aromatic heterocyclic ring group having 2 to 18 carbon atoms which may have a substituent include a thienyl group, a furyl group, a thiazolyl group, a benzothienyl group, a benzofuranyl group, a benzothiazolyl group, and a benzoxazolyl group. Thereamong, a benzothiazolyl group is preferable.

Examples of the substituent of an aromatic heterocyclic ring group having 2 to 18 carbon atoms which may have a substituent include the same substituents as the aromatic hydrocarbon ring group having 5 to 18 carbon atoms which may have a substituent, and preferred examples thereof are also the same as stated above.

Note that an aromatic hydrocarbon ring group having 5 to 18 carbon atoms and an aromatic heterocyclic ring group having 2 to 18 carbon atoms may have a plurality of substituents. When the aromatic hydrocarbon ring group having 5 to 18 carbon atoms and the aromatic heterocyclic ring group having 2 to 18 carbon atoms have a plurality of substituents, the substituent may be the same or different.

Here, the aromatic ring of Ax may have a plurality of substituents which are the same or different, and two adjacent substituents may be bonded to each other to form a ring. The ring formed by two adjacent substituents may be either a monocyclic ring or a fused polycyclic ring, and may be either an unsaturated ring or a saturated ring.

Note that the "number of carbon atoms" of the organic group of Ax having at least one aromatic ring selected from the group consisting of an aromatic hydrocarbon ring having 6 to 30 carbon atoms and an aromatic heterocyclic ring having 2 to 30 carbon atoms means the number of carbon atoms of the aromatic hydrocarbon ring group and the aromatic heterocyclic ring not including the number of carbon atoms of the substituents.

Moreover, examples of the organic group of Ax having at least one aromatic ring selected from the group consisting of an aromatic hydrocarbon ring having 6 to 30 carbon atoms and an aromatic heterocyclic ring having 2 to 30 carbon atoms include the following 1) to 5):

1) a hydrocarbon ring group having 6 to 40 carbon atoms having at least one aromatic hydrocarbon ring having 6 to 30 carbon atoms;
2) a heterocyclic ring group having 2 to 40 carbon atoms having at least one aromatic ring selected from the group consisting of an aromatic hydrocarbon ring having 6 to 30 carbon atoms and an aromatic heterocyclic ring having 2 to 30 carbon atoms;
3) an alkyl group having 1 to 12 carbon atoms substituted with at least one of an aromatic hydrocarbon ring group having 6 to 30 carbon atoms and an aromatic heterocyclic ring group having 2 to 30 carbon atoms;
4) an alkenyl group having 2 to 12 carbon atoms substituted with at least one of an aromatic hydrocarbon ring group having 6 to 30 carbon atoms and an aromatic heterocyclic ring group having 2 to 30 carbon atoms; and

5) an alkynyl group having 2 to 12 carbon atoms substituted with at least one of an aromatic hydrocarbon ring group having 6 to 30 carbon atoms and an aromatic heterocyclic ring group having 2 to 30 carbon atoms.

Examples of the aromatic hydrocarbon ring of "a hydrocarbon ring group having 6 to 40 carbon atoms having at least one aromatic hydrocarbon ring having 6 to 30 carbon atoms" in the aforementioned 1) include the same groups as those listed as specific examples of the aromatic hydrocarbon ring of Ax. Moreover, examples of the hydrocarbon ring group of the aforementioned 1) include an aromatic hydrocarbon ring group having 6 to 30 carbon atoms (a phenyl group, a naphthyl group, an anthracenyl group, a phenanthrenyl group, a pyrenyl group, a fluorenyl group, and the like), an indanyl group, a 1,2,3,4-tetrahydronaphthyl group, and, a 1,4-dihydronaphthyl group.

Specific examples of the aromatic hydrocarbon ring and the aromatic heterocyclic ring of "a heterocyclic ring group having 2 to 40 carbon atoms having at least one aromatic ring selected from the group consisting of an aromatic hydrocarbon ring having 6 to 30 carbon atoms and an aromatic heterocyclic ring having 2 to 30 carbon atoms" in the aforementioned 2) include the same groups as those listed as the specific examples of the aromatic hydrocarbon ring and the aromatic heterocyclic ring of Ax. Moreover, examples of the heterocyclic ring group of the aforementioned 2) include an aromatic heterocyclic ring group having 2 to 30 carbon atoms (a phthalimide group, a 1-benzofuranyl group, a 2-benzofuranyl group, an acridinyl group, an isoquinoryl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridinyl group, a thiazolopyridazinyl group, a thiazolopyrimidinyl group, , a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxazolyl group, a benzothiadiazolyl group, benzothiazolyl group, a benzothiophenyl group, a benzotriazinyl group, a benzotriazolyl group, a benzopyrazolyl group, a benzopyranonyl group, a dihydropyranyl group, a tetrahydropyranyl group, a dihydrofuranyl group, a tetrahydrofuranyl group, and the like), a 2,3-dihydroindolyl group, a 9,10-dihydroacridinyl group, a 1,2,3,4-tetrahydroquinolyl group, a dihydropyranyl group, a tetrahydropyranyl group, a dihydrofuranyl group, and a tetrahydrofuranyl group.

Specific examples of the alkyl group having 1 to 12 carbon atoms of "an alkyl group having 1 to 12 carbon atoms substituted with at least one of an aromatic hydrocarbon ring group having 6 to 30 carbon atoms and an aromatic heterocyclic ring group having 2 to 30 carbon atoms" in the aforementioned 3) include a methyl group, an ethyl group, a propyl group, and an isopropyl group. Moreover, specific examples of the aromatic hydrocarbon ring group having 6 to 30 carbon atoms and the aromatic heterocyclic ring group having 2 to 30 carbon atoms in the aforementioned 3) include the same groups as those listed as the specific examples of the aromatic hydrocarbon ring group having 6 to 30 carbon atoms and the aromatic heterocyclic ring group having 2 to 30 carbon atoms in the aforementioned 1) and 2).

Specific examples of the alkenyl group having 2 to 12 carbon atoms of "an alkenyl group having 2 to 12 carbon atoms substituted with at least one of an aromatic hydrocarbon ring group having 6 to 30 carbon atoms and an aromatic heterocyclic ring group having 2 to 30 carbon atoms" in the aforementioned 4) include a vinyl group and an allyl group. Moreover, specific examples of the aromatic hydrocarbon ring group having 6 to 30 carbon atoms and the aromatic heterocyclic ring group having 2 to 30 carbon atoms in the aforementioned 4) include the same groups as those listed as the specific examples of the aromatic hydrocarbon ring group having 6 to 30 carbon atoms and the aromatic heterocyclic ring group having 2 to 30 carbon atoms in the aforementioned 1) and 2).

Secific examples of the alkynyl group having 2 to 12 carbon atoms of "an alkynyl group having 2 to 12 carbon atoms substituted with at least one of an aromatic hydrocarbon ring group having 6 to 30 carbon atoms and an aromatic heterocyclic ring group having 2 to 30 carbon atoms" in the aforementioned 5) include an ethynyl group and a propynyl group. Moreover, specific examples of the aromatic hydrocarbon ring group having 6 to 30 carbon atoms and the aromatic heterocyclic ring group having 2 to 30 carbon atoms in the aforementioned 5) include the same groups as those listed as the specific examples of the aromatic hydrocarbon ring group having 6 to 30 carbon atoms and the aromatic heterocyclic ring group having 2 to 30 carbon atoms in the aforementioned 1) and 2).

Note that the organic groups listed in the aforementioned 1) to 5) may have one or a plurality of substituents. When there is a plurality of substituents, the plurality of substituents may be the same or different.

Examples of such substituents include: a halogen atom such as a fluorine atom and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group; an N-N-dialkylamino group having 2 to 12 carbon atoms such as a dimethylamino group; an alkoxy group having 1 to 6 carbon atoms such as a methoxy group, an ethoxy group and an isopropoxy group; a nitro group; an aromatic hydrocarbon ring group having 6 to 20 carbon atoms such as a phenyl group and a naphthyl group; $-OCF_3$; $-C(=O)-R^b$; $-O-C(=O)-R^b$; $-C(=O)-O-R^b$; and $-SO_2R^a$. Here, $R^b$ and $R^a$ are the same as defined above, and preferred examples thereof are also

the same as stated above.

Thereamong, each of the substituents having the organic groups listed in the aforementioned 1) to 5) is preferably at least one substituent selected from a halogen atom, a cyano group, an alkyl group having 1 to 6 carbon atoms, and an alkoxy group having 1 to 6 carbon atoms.

Preferred examples of the organic group having at least one aromatic ring selected from the group consisting of an aromatic hydrocarbon ring having 6 to 30 carbon atoms and an aromatic heterocyclic ring having 2 to 30 carbon atoms as Ax are given below. However, the present disclosure is not limited to the following examples. Note that in the following formulas, "-" represents a bond with an N atom extending from any position of the ring (i.e., an N atom bonded to Ax in $Ar^0$ and $Ar^1$ of the formula (I-1)).

1) Specific examples of a hydrocarbon ring group having 6 to 40 carbon atoms having at least one aromatic hydrocarbon ring group having 6 to 30 carbon atoms include the configurations represented by the following formulas (1-1) to (1-21), and the aromatic hydrocarbon ring groups having 6 to 30 carbon atoms represented by formulas (1-9) to (1-21) are preferable.

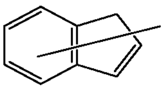
(1-1)
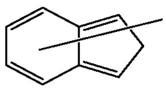
(1-2)
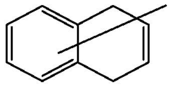
(1-3)
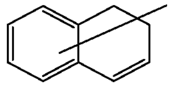
(1-4)
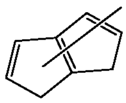
(1-5)
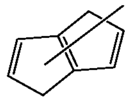
(1-6)

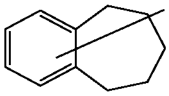
(1-7)
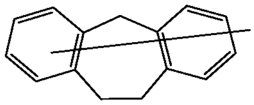
(1-8)

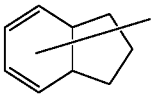
(1-9)
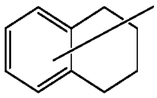
(1-10)
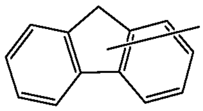
(1-11)
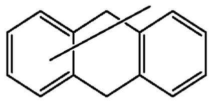
(1-12)

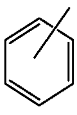
(1-13)
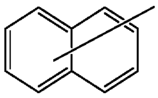
(1-14)
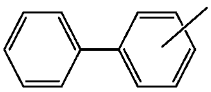
(1-15)
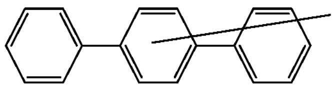
(1-16)

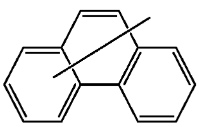
(1-17)
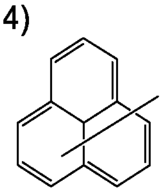
(1-18)
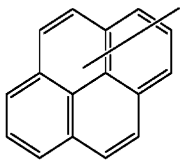
(1-19)
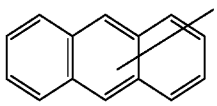
(1-20)
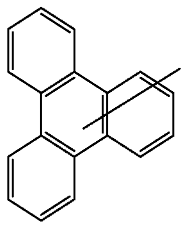
(1-21)

2) Specific examples of a heterocyclic ring group having 2 to 40 carbon atoms having at least one aromatic ring selected from the group consisting of an aromatic hydrocarbon ring having 6 to 30 carbon atoms and an aromatic heterocyclic ring having 2 to 30 carbon atoms include the configurations represented by the following formulas (2-1) to (2-51), and the aromatic heterocyclic ring groups having 2 to 30 carbon atoms represented by formulas (2-12) to (2-51) are preferable.

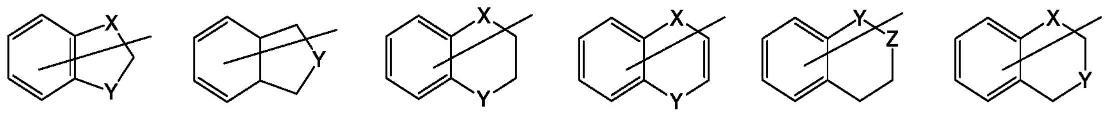

(2-1) (2-2) (2-3) (2-4) (2-5) (2-6)

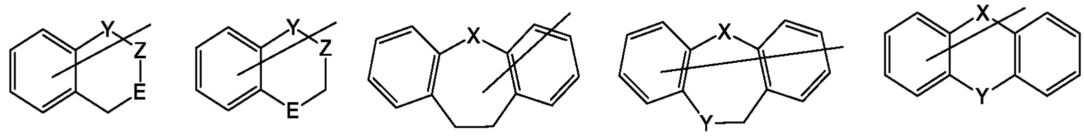

(2-7) (2-8) (2-9) (2-10) (2-11)

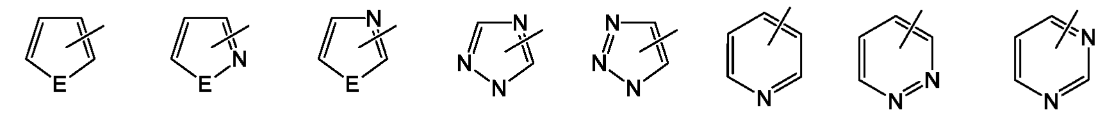

(2-12) (2-13) (2-14) (2-15) (2-16) (2-17) (2-18) (2-19)

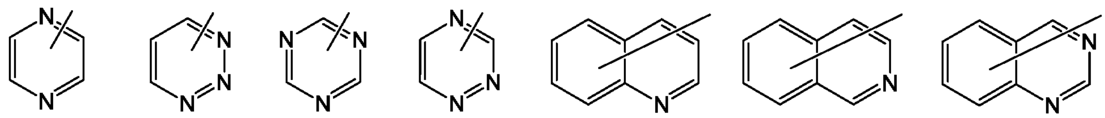

(2-20) (2-21) (2-22) (2-23) (2-24) (2-25) (2-26)

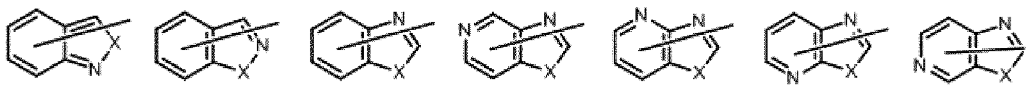

(2-27) (2-28) (2-29) (2-30) (2-31) (2-32) (2-33)

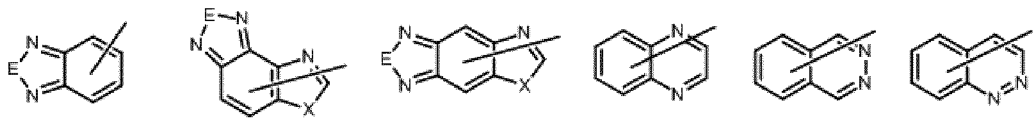

(2-34) (2-35) (2-36) (2-37) (2-38) (2-39)

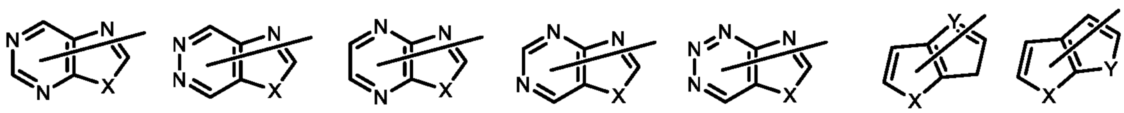

(2-40) (2-41) (2-42) (2-43) (2-44) (2-45) (2-46)

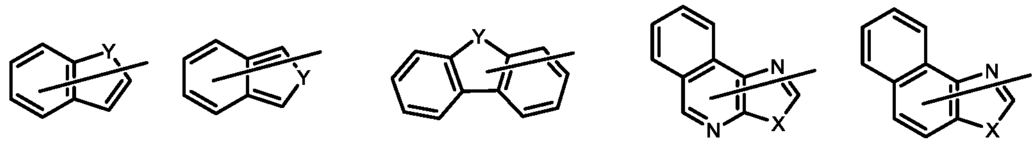

(2-47) (2-48) (2-49) (2-50) (2-51)

where in each formula, X represents $-CH_2-$, $-NR^c-$, an oxygen atom, a sulfur atom, -SO- or $-SO_2-$, Y and Z each independently represent $-NR^c-$, an oxygen atom, a sulfur atom, -SO-, or $-SO_2-$, E represents $-NR^c-$, an oxygen atom or a sulfur atom, and $R^c$ represents a hydrogen atom, or, an alkyl group having 1 to 6 carbon atoms such as a methyl group, an ethyl group, and a propyl group, where in each formula, an oxygen atom, a sulfur atom, -SO-, and $-SO_2-$are not

adjacent to each other.

3) Specific examples of an alkyl group having 1 to 12 carbon atoms which was substituted with at least one of an aromatic hydrocarbon ring group having 6 to 30 carbon atoms and an aromatic heterocyclic ring group having 2 to 30 carbon atoms include the configurations represented by the following formulas (3-1) to (3-8):

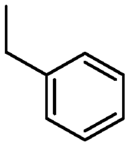
(3-1)

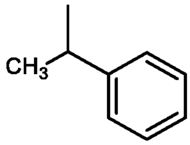
(3-2)

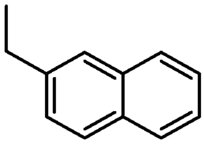
(3-3)

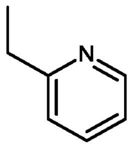
(3-4)

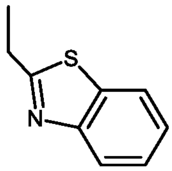
(3-5)

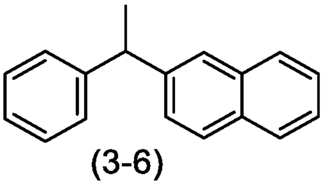
(3-6)

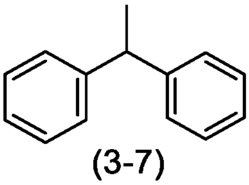
(3-7)

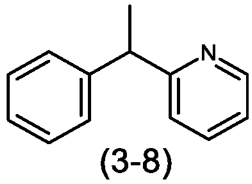
(3-8)

.

4) Specific examples of an alkenyl group having 2 to 12 carbon atoms which was substituted with at least one of an aromatic hydrocarbon ring group having 6 to 30 carbon atoms and an aromatic heterocyclic ring group having 2 to 30 carbon atoms include the configurations represented by the following formulas (4-1) to (4-5):

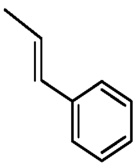
(4-1)

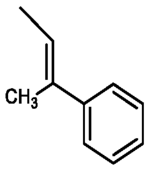
(4-2)

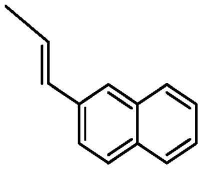
(4-3)

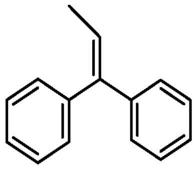
(4-4)

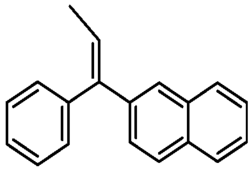
(4-5)

.

5) Specific examples of an alkynyl group having 2 to 12 carbon atoms which was substituted with at least one selected from the group consisting of an aromatic hydrocarbon ring and an aromatic heterocyclic ring include the configurations represented by the following formulas (5-1) to (5-2):

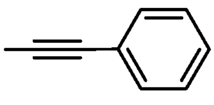
(5-1)

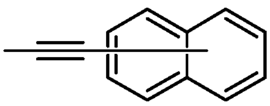
(5-2)

.

Note that the rings of the aforementioned preferred examples of Ax may have one or a plurality of substituents. Moreover, when there is a plurality of substituents, the plurality of substituents may be the same or different. Examples of such a substituent include: a halogen atom such as a fluorine atom and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group; a N-N-dialkylamino group having 1 to 12 carbon atoms such as a dimethylamino group; an alkoxy group having 1 to 6 carbon atoms such as a methoxy group, an ethoxy group, and an isopropoxy group; a nitro group; an aromatic hydrocarbon ring group having 6 to 20 carbon atoms such as a phenyl group and a naphthyl group; $-OCF_3$; $-C(=O)-R^b$; $-O-C(=O)-R^b$; $-C(=O)-O-R^b$; and $-SO_2R^a$.

Here, $R^b$ and $R^a$ are the same as defined above, and preferred examples thereof are also the same as stated above. Thereamong, the substituents which the aforementioned ring of Ax has are preferably a halogen atom, a cyano

group, an alkyl group having 1 to 6 carbon atoms, and, an alkoxy group having 1 to 6 carbon atoms.

Among these described above, Ax is preferably a group represented by an aromatic hydrocarbon ring group having 6 to 30 carbon atoms, an aromatic heterocyclic ring group having 2 to 30 carbon atoms, or the formula (1-9).

Moreover, Ax is more preferably an aromatic hydrocarbon ring group having 6 to 20 carbon atoms, or an aromatic heterocyclic ring group having 4 to 20 carbon atoms, and is even more preferably any of the groups represented by the aforementioned formulas (1-14), (1-20), (2-27) to (2-33), (2-35) to (2-43), (2-50), and (2-51).

Note that as described above, the aforementioned ring may have one or a plurality of substituents. When there is a plurality of substituents, the plurality of substituents may be the same or different. Examples of such a substituent include: a halogen atom such as a fluorine atom and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group and a pentafluoroethyl group; a N-N-dialkylamino group having 1 to 12 carbon atoms such as a dimethylamino group; an alkoxy group having 1 to 6 carbon atoms such as a methoxy group, an ethoxy group, and an isopropoxy group; a nitro group; an aromatic hydrocarbon ring group having 6 to 20 carbon atoms such as a phenyl group and a naphthyl group; -C(=O)-$R^b$; -O-C(=O)-$R^b$; -C(=O)-O-$R^b$; and -$SO_2R^a$.

Here, $R^b$ and $R^a$ are the same as defined above, and preferred examples thereof are also the same as stated above.

Thereamong, the substituent of the aforementioned ring is preferably a halogen atom, a cyano group, an alkyl group having 1 to 6 carbon atoms, and, an alkoxy group having 1 to 6 carbon atoms.

Moreover, Ax is more preferably a group represented by the following formula (V).

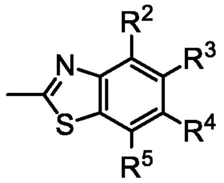

(V)

Here, in formula (V), $R^2$ to $R^5$ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, -$OCF_3$, O-C(=O)-$R^b$, or, -C(=O)-O-$R^b$, and $R^b$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring group having 5 to 18 carbon atoms which may have a substituent. Thereamong, it is preferable that all of $R^2$ to $R^5$ are hydrogen atoms, or at least one among $R^2$ to $R^5$ is an alkoxy group having 1 to 6 carbon atoms which may have a substituent, and the rest are hydrogen atoms.

Moreover, C-$R^2$ to C-$R^5$ may be the same or different, and one or more ring constituents C-$R^2$ to C-$R^5$ may be replaced by a nitrogen atom.

Here, specific examples of the group in which at least one among C-$R^2$ to C-$R^5$ of the group represented by the aforementioned formula (V) is replaced by a nitrogen atom are given below. However, the groups in which at least one among C-$R^2$ to C-$R^5$ is replaced by a nitrogen atom are not limited to these groups.

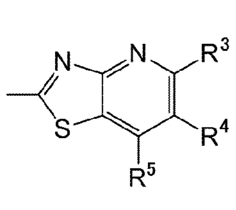
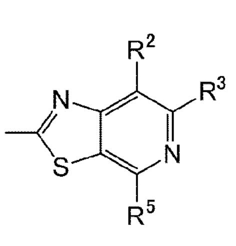
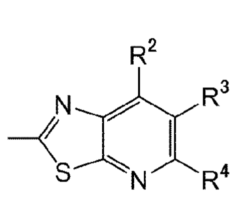
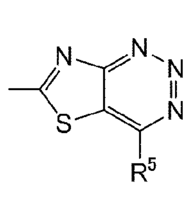

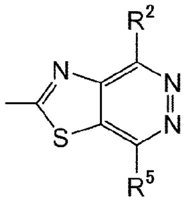
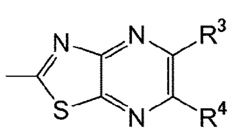
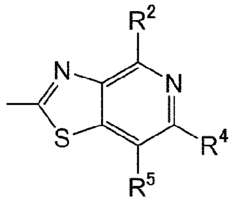
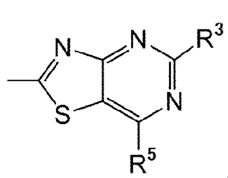

where in each formula, $R^2$ to $R^5$ are the same as defined above, and preferred examples thereof are also the same as stated above.

Further, the organic group having 1 to 30 carbon atoms which may have a substituent of Ay of the groups represented by the aforementioned formulas (II-1) to (II-7) and (ii-1) to (ii-21) is not specifically limited, and examples thereof include an alkyl group having 1 to 20 carbon atoms which may have a substituent, an alkenyl group having 2 to 20 carbon atoms which may have a substituent, an alkynyl group having 2 to 20 carbon atoms which may have a substituent, a cycloalkyl group having 3 to 12 carbon atoms which may have a substituent, $-SO_2R^a$, $-C(=O)-O-R^b$, $-O-C(=O)-R^b$, $-C(=O)-R^b$, $-CS-NH-R^b$, $-NH-C(=O)-O-R^b$, $-O-C(=O)-NH-R^b$, an aromatic hydrocarbon ring group having 6 to 30 carbon atoms which may have a substituent, and an aromatic heterocyclic ring group having 2 to 30 carbon atoms which may have a substituent.

Here, $R^a$ and $R^b$ are the same as defined above, and preferred examples thereof are also the same as stated above.

Note that examples of an alkyl group having 1 to 20 carbon atoms in the case when Ay is an alkyl group having 1 to 20 carbon atoms which may have a substituent, an alkenyl group having 2 to 20 carbon atoms in the case when Ay is an alkenyl group having 2 to 20 carbon atoms which may have a substituent, a cycloalkyl group having 3 to 12 carbon atoms in the case when Ay is a cycloalkyl group having 3 to 12 carbon atoms which may have a substituent include the same groups as those listed as the specific examples of an alkyl group having 1 to 20 carbon atoms in the case when the aforementioned $R^b$ is an alkyl group having 1 to 20 carbon atoms which may have a substituent, an alkenyl group having 2 to 20 carbon atoms in the case when $R^b$ is an alkenyl group having 2 to 20 carbon atoms which may have a substituent, a cycloalkyl group having 3 to 12 carbon atoms in the case when $R^b$ is a cycloalkyl group having 3 to 12 carbon atoms which may have a substituent. Furthermore, the number of carbon atoms of an alkyl group having 1 to 20 carbon atoms which may have a substituent is preferably 1 to 10, the number of carbon atoms of an alkenyl group having 2 to 20 carbon atoms which may have a substituent is preferably 2 to 10, and the number of carbon atoms of a cycloalkyl group having 3 to 12 carbon atoms which may have a substituent is preferably 3 to 10.

Furthermore, examples of an alkynyl group having 2 to 20 carbon atoms which may have a substituent in the case when Ay is an alkynyl group having 2 to 20 carbon atoms which may have a substituent include an ethynyl group, a propynyl group, a 2-propynyl group (propargyl group), a butynyl group, a 2-butynyl group, a 3-butynyl group, a pentinyl group, a 2-pentinyl group, a hexynyl group, a 5-hexynyl group, a heptinyl group, an octinyl group, a 2-octynyl group, a nonanyl group, a decanyl group, and a 7-decanyl group.

Moreover, examples of the substituent in the case when Ay is an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an alkynyl group having 2 to 20 carbon atoms which may have a substituent include: a halogen atom such as a fluorine atom and a chlorine atom; a cyano group; an N-N-dialkylamino group having 2 to 12 carbon atoms such as a dimethylamino group; an alkoxy group having 1 to 20 carbon atoms such as a methoxy group, an ethoxy group, an isopropoxy group, and a butoxy group; an alkoxy group having 1 to 12 carbon atoms substituted with an alkoxy group having 1 to 12 carbon atoms such as a methoxymethoxy group and a methoxyethoxy group; a nitro group; an aromatic hydrocarbon ring group having 6 to 20 carbon atoms such as a phenyl group and a naphthyl group; an aromatic heterocyclic ring group having 2 to 20 carbon atoms such as a triazolyl group, a pyrrolyl group, a furanyl group, and a thiophenyl group; a cycloalkyl group having 3 to 8 carbon atoms such as a cyclopropyl group, a cyclopentyl group and a cyclohexyl group; a cycloalkyloxy group having 3 to 8 carbon atoms such as a cyclopentyloxy group and a cyclohexyloxy group; a cyclic ether group having 2 to 12 carbon atoms such as a tetrahydrofuranyl group, a tetrahydropyranyl group, a dioxolanyl group and a dioxanyl group; an aryloxy group having 6 to 14 carbon atoms such as a phenoxy group and a naphthoxy group; a fluoroalkyl group having 1 to 12 carbon atoms in which at least one hydrogen atom is substituted with a fluorine atom such as a trifluoromethyl group, a pentafluoroethyl group, and $-CH_2CF_3$; a benzofuryl group; a benzopyranyl group; a benzodioxolyl group; a benzodioxanyl group; $-O-C(=O)-R^b$; $-C(=O)-R^b$; $-C(=O)-O-R^b$; $-SO_2R^a$; $-SR^b$; an alkoxy group having 1 to 12 carbon atoms substituted with $-SR^b$; and a hydroxyl group. Here, $R^a$ and $R^b$ are the same as defined above, and preferred examples thereof are also the same as stated above.

Note that an alkyl group having 1 to 20 carbon atoms, an alkenyl group having 2 to 20 carbon atoms, a cycloalkyl group having 3 to 12 carbon atoms, and an alkynyl group having 2 to 20 carbon atoms which may have a substituent of Ay may have a plurality of substituents as described above, and when there is a plurality of substituents, the plurality of substituents may be the same or different.

Further, examples of the aromatic hydrocarbon ring group having 6 to 30 carbon atoms, the aromatic heterocyclic ring group having 2 to 30 carbon atoms, and, these substituents of Ay include the same groups as those listed as the respective examples of the aromatic hydrocarbon ring group, the aromatic heterocyclic ring group, and, these substituents of Ax. The aromatic hydrocarbon ring group having 6 to 30 carbon atoms and the aromatic heterocyclic ring group having 2 to 30 carbon atoms of Ay may have a plurality of substituents selected from those listed above. When the aromatic hydrocarbon ring group and the aromatic heterocyclic ring group of Ay have a plurality of substituents, the plurality of

substituents may be the same or different. Furthermore, the number of carbons atoms of the aforementioned aromatic hydrocarbon ring group of Ay is preferably 6 to 20, more preferably 6 to 18, and even more preferably 6 to 12. Further, the number of carbon atoms of the aforementioned aromatic heterocyclic ring group of Ay is preferably 2 to 20 and more preferably 2 to 18.

Among these described above, Ay is preferably a hydrogen atom, an alkyl group having 1 to 20 carbon atoms which may have a substituent, an alkenyl group having 2 to 20 carbon atoms which may have a substituent, an alkynyl group having 2 to 20 carbon atoms which may have a substituent, a cycloalkyl group having 3 to 12 carbon atoms which may have a substituent, an aromatic hydrocarbon ring group having 6 to 18 carbon atoms which may have a substituent, or an aromatic heterocyclic ring group having 2 to 18 carbon atoms which may have a substituent. Furthermore, Ay is more preferably a hydrogen atom, an alkyl group having 1 to 18 carbon atoms which may have a substituent, an alkenyl group having 2 to 18 carbon atoms which may have a substituent, an alkynyl group having 2 to 18 carbon atoms which may have a substituent, a cycloalkyl group having 3 to 10 carbon atoms which may have a substituent, an aromatic hydrocarbon ring group having 6 to 12 carbon atoms which may have a substituent, or an aromatic heterocyclic ring group having 2 to 18 carbon atoms which may have a substituent. Thereamong, Ay is particularly preferably an alkyl group having 1 to 18 carbon atoms which may have a substituent, and thereamong, an alkyl group having 2 to 12 carbon atoms which may have a substituent is further particularly preferable.

Further, in the aforementioned formula (I-1), Xa represents an organic group having 1 to 20 carbon atoms which may have a substituent, and examples of an organic group having 1 to 20 carbon atoms include an alkylene group having 1 to 18 carbon atoms which may have a substituent, a cyclic aliphatic group having 3 to 18 carbon atoms which may have a substituent, an aromatic hydrocarbon ring group having 6 to 18 carbon atoms which may have a substituent.

Examples of the substituent of Xa include: a halogen atom such as a fluorine atom and a chlorine atom; a cyano group; an alkyl group having 1 to 6 carbon atoms such as a methyl group, an ethyl group, a propyl group, an isopropyl group, a butyl group, a sec-butyl group, and a tertiary butyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group; an N-N-dialkylamino group having 2 to 12 carbon atoms such as a dimethylamino group; an alkoxy group having 1 to 6 carbon atoms such as a methoxy group, an ethoxy group, and an isopropoxy group; a nitro group; an aromatic hydrocarbon ring group having 6 to 20 carbon atoms such as a phenyl group and a naphthyl group; $-OCF_3$; $-C(=O)-R^b$; $-C(=O)-O-R^b$; $-O-C(=O)-R^b$; and $-SO_2R^a$.

$R^a$ represents an alkyl group having 1 to 6 carbon atoms such as a methyl group and an ethyl group, or, an aromatic hydrocarbon ring group having 6 to 20 carbon atoms which may have an alkyl group having 1 to 6 carbon atoms or an alkoxy group having 1 to 6 carbon atoms as a substituent, such as a phenyl group, a 4-methylphenyl group, or a 4-methoxyphenyl group.

$R^b$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring group having 5 to 18 carbon atoms which may have a substituent.

When there is a plurality of substituents, the plurality of substituents may be the same or different from each other.

Examples of the substituent of Xa are preferably, from the viewpoint of solubility improvement, a halogen atom, a cyano group, an alkyl group having 1 to 6 carbon atoms, an alkyl group having 1 to 6 carbon atoms in which at least one hydrogen atom is substituted with a halogen atom, an alkoxy group having 1 to 6 carbon atoms, and a nitro group. When Xa has a plurality of the aforementioned substituents, the substituent may be the same or different.

Xa is preferably a group represented by any of the following formulas (VII-1) to (VII-29), and the groups represented by the following formulas may have the aforementioned substituents.

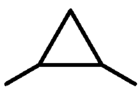
(VII-1)

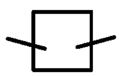
(VII-2)

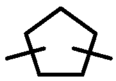
(VII-3)

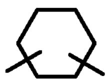
(VII-4)

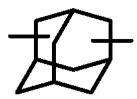
(VII-5)

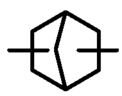
(VII-6)

$-CH_2-$ (VII-7)

$-(CH_2)_2-$ (VII-8)

$-(CH_2)_3-$ (VII-9)

$-(CH_2)_4-$ (VII-10)

$-(CH_2)_5-$ (VII-11)

$-(CH_2)_6-$ (VII-12) $-(CH_2)_7-$ (VII-13) $-(CH_2)_8-$ (VII-14) $-(CH_2)_9-$ (VII-15) $-(CH_2)_{10}-$ (VII-16)

$-(CH_2)_{11}-$ (VII-17) $-(CH_2)_{12}-$ (VII-18) $-(CH_2)_{13}-$ (VII-19) $-(CH_2)_{14}-$ (VII-20) $-(CH_2)_{15}-$ (VII-21)

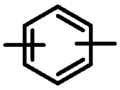
(VII-22)

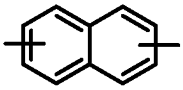
(VII-23)

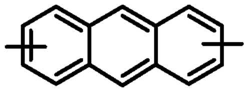
(VII-24)

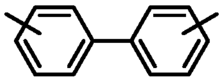
(VII-25)

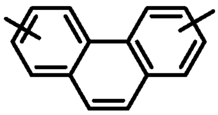
(VII-26)

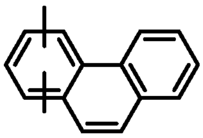
(VII-27)

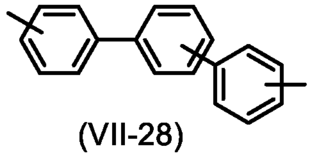
(VII-28)

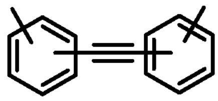
(VII-29)

Further, in the aforementioned formula (I-1), $Z^1$ to $Z^4$ each independently represent a single bond, -O-, -O-$CH_2$-, -$CH_2$-O-, -O-$CH_2$-$CH_2$, -$CH_2$-$CH_2$-O-, -C(=O)-O-, -O-C(=O)-, -C(=O)-S-, -S-C(=O)-, -$NR^{20}$-C(=O)-, -C(=O)-$NR^{20}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-C(=O)-O-, -O-C(=O)-$CH_2$-, -$CH_2$-O-C(=O)-, -C(=O)-O-$CH_2$-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C≡C-$R^{20}$, and $R^{20}$ represents a hydrogen atom or an alkyl group having 1 to 6 carbon atoms.

Thereamong, $Z^1$ and $Z^4$ preferably each independently represent -C(=O)-O- or -O-C(=O)-.

$Z^2$ and $Z^3$ each independently represent preferably -C(=O)-O-, -O-C(=O)-, -C(=O)-S-, -S-C(=O)-, -$NR^{20}$C(=O)-, -C(=O)-$NR^{20}$-, -$CF_2$-O-, -O-$CF_2$-, -$CF_2$-$CF_2$-, -CH=CH-C(=O)-O-, -O-C(=O)-CH=CH-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C=C-, more preferably -C(=O)-O-, -O-C(=O)-, -C(=O)-S-, -S-C(=O)-, -$NR^{20}$C(=O)-, -C(=O)-NR $^{21}$-, particularly preferably -C(=O)-O-, -O-C(=O)-, -$NR^{20}$C(=O)-, or -C(=O)-$NR^{21}$ -, and most preferably -C(=O)-O- or -O-C(=O)-.

Further, in the aforementioned formula (I-1), $G^1$ and $G^2$ each independently represent an organic group of either an alkylene group having 1 to 20 carbon atoms, or, an alkylene group having 3 to 20 carbon atoms in which at least one methylene group (-CH2-) is substituted by -O-or -C(=O)-, and the hydrogen atom included in the organic group of $G^1$ and $G^2$ may be substituted by at least one substituent selected from the group consisting of an alkyl group having 1 to 5 carbon atoms, an alkoxy group having 1 to 5 carbon atoms, and a halogen atom. Note that in the description "an alkylene group having 3 to 20 carbon atoms in which at least one methylene group (-$CH_2$-) is substituted with -O- or -C(=O)-", -O- preferably does not replace the consecutive methylene groups in the alkylene group (i.e., the -O-O- configuration is not formed), and -C(=O)-preferably does not replace the consecutive methylene groups in the alkylene group (i.e., the -C(=O)-C(=O)- configuration is not formed). Furthermore, the methylene group (-$CH_2$-) at each terminal of $G^1$ and $G^2$ is not substituted with -O- or -C(=O)-.

Here, the organic groups of $G^1$ and $G^2$ are preferably an alkylene group having 1 to 20 carbon atoms which may be substituted with a fluorine atom, or, groups represented by -$(CH_2)_j$-C(=O)-O-$(CH_2)_k$- which may be substituted with a fluorine atom (where j and k each represent an integer from 2 to 12, and preferably represent an integer from 2 to 8), more preferably an alkylene group having 2 to 12 carbon atoms which may be substituted with a fluorine atom, even more preferably an unsubstituted alkylene group having 2 to 12 carbon atoms, and particularly preferably a group

represented by $-(CH_2)_1-$ (where 1 represents an integer from 2 to 12, and preferably, represents an integer from 2 to 8).

Further, in the aforementioned formula (I-1), $A^1$ and $A^2$ preferably each independently represent an aromatic group which may have a substituent or an aromatic group having 2 to 20 carbon atoms which may have a substituent.

Specific examples of the aromatic group include: an aromatic hydrocarbon ring group having 6 to 20 carbon atoms such as a 1,2-phenylene group, a 1,3-phenylene group, a 1,4-phenylene group, a 1,4-naphthalene group, a 1,5-naphthylene group, a 2,6-naphthylene group, and a 4,4'-biphenylene group; and an aromatic heterocyclic ring group having 2 to 20 carbon atoms such as a furan-2,5-diyl group, a thiophene-2,5-diyl group, a pyridine-2,5-diyl group, and a pyrazine-2,5-diyl group. Thereamong, the aromatic group is preferably an aromatic hydrocarbon ring group having 6 to 20 carbon atoms, more preferably a phenylene group, and particularly preferably a 1,4-phenylene group represented by the following formula (b).

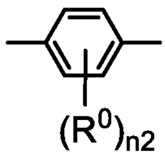
(b)

where $R^0$ and n2 are the same as defined above, and preferred examples thereof are also the same as stated above.

Note that when there is a plurality of $R^0$, each $R^0$ may be the same or different.

Further, in the aforementioned formula (I-1), $B^1$ and $B^2$ preferably each independently represent a cyclic aliphatic group which may have a substituent, or, an aromatic group which may have a substituent, a cyclic aliphatic group having 5 to 20 carbon atoms which may have a substituent, or, an aromatic group having 2 to 20 carbon atoms which may have a substituent.

Here, the aromatic groups of $B^1$ and $B^2$ are the same as the aromatic groups of $A^1$ and $A^2$, and the substituents of the aromatic groups of $B^1$ and $B^2$ are the same as the substituents of the aromatic groups of $A^1$ and $A^2$.

Specific examples of the cyclic aliphatic group include: a cycloalkanediyl group having 5 to 20 carbon atoms such as a cyclopentane-1,3-diyl group, a cyclohexane-1,4-diyl group, a cycloheptane-1,4-diyl group, and a cyclooctane-1,5-diyl group; and a bicycloalkanediyl group having 5 to 20 carbon atoms such as a decahydronaphthalene-1,5-diyl group and a decahydronaphthalene-2,6-diyl group. Thereamong, the cyclic aliphatic group is preferably a cycloalkanediyl group having 5 to 20 carbon atoms which may be substituted, more preferably a cyclohexanediyl group, and particularly preferably a cyclohexane-1,4-diol group represented by the following formula (a). The cyclic aliphatic group may be a trans-isomer represented by formula (a1), a cis-isomer represented by formula (a2), or a mixture of cis- and trans-isomers, but a trans-isomer represented by formula (a1) is more preferable.

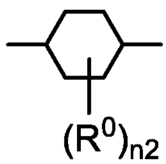
(a)

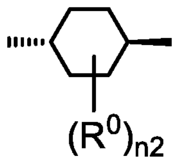
(a1)

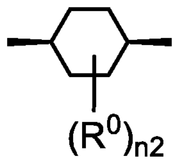
(a2)

where $R^0$ and n2 are the same as defined above, and preferred examples thereof are also the same as stated above.

Note that when there is a plurality of $R^0$, each $R^0$ may be the same or different.

Further, in the aforementioned formula (I-1), $Y^1$ to $Y^4$ each independently represent a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or $-NR^{21}-C(=O)-NR^{22}-$. Here, $R^{21}$ and $R^{22}$ each independently represent a hydrogen atom or an alkyl group having 1 to 6 carbon atoms.

Thereamong, $Y^1$ to $Y^4$ preferably each independently represent -O-, -C(=O)-, -C(=O)-O-, -O-C(=O)-O-, or -O-C(=O)-.

When a plurality of $Y^1$ and $Y^2$ are present, these may be the same or different.

Further, in the aforementioned formula (I-1), one of $P^1$ and $P^2$ represents a hydrogen atom or a polymerizable group, and the other of $P^1$ and $P^2$ represents a polymerizable group. Here, $P^1$ and $P^2$ preferably each independently represent a polymerizable group.

Here, examples of the polymerizable groups of $P^1$ and $P^2$ include groups represented by $CH_2=CR^1-C(=O)-O-$ ($R^1$ represents a hydrogen atom, a methyl group, or a chlorine atom) such as an acryloyloxy group and a methacryloyloxy group, a vinyl group, a vinyl ether group, a p-stilbene group, an acryloyl group, a methacryloyl group, a carboxyl group,

a methyl carbonyl group, a hydroxyl group, an amide group, an alkylamino group having 1 to 4 carbon atoms, an amino group, an epoxy group, an oxetanyl group, an aldehyde group, an isocyanate group, and a thioisocyanate group. Thereamong, as in the following formula (IV), the groups represented by $-CH_2=CR^1-C(=O)-O-$ are preferable, $-CH_2=CH-C(=O)-O-$ (acryloyloxy group) and $-CH_2=C(CH_3)-C(=O)-O-$(methacryloyloxy group) are more preferable, and acryloyloxy group is even more preferable. Note that when two $R^1$ are present in the polymerizable compound (I-1), they may be the same or different. Furthermore, $P^1$ and $P^2$ may be different, but it is preferable that they are the same polymerizable group.

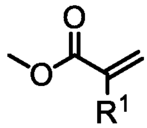
(IV)

where in the formula (IV), $R^1$ represents a hydrogen atom, a methyl group, or a chlorine atom]

Here, in the formula (I-1), p and q each independently represent an integer from 0 to 2, and preferably each independently is 0 or 1, and more preferably 0.

When both of p and q are 1, $B^1$ and $B^2$ preferably each independently represent a cyclic aliphatic group which may have a substituent in the aforementioned formula (I-1), and more preferably a cyclic aliphatic group having 5 to 20 carbon atoms which may have a substituent.

Further, the polymerizable compound (I-1) is not specifically limited, but preferably has a symmetrical structure around Xa (namely, $Z^2$ and $Z^3$, $Ar^0$ and $Ar^1$, $Z^1$ and $Z^4$, $A^1$ and $A^2$, $Y^1$ and $Y^2$, $B^1$ and $B^2$, p and q, $Y^3$ and $Y^4$, $G^1$ and $G^2$, and $P^1$ and $P^2$ are respectively the same).

Here, the polymerizable compound of the present disclosure is preferably a polymerizable compound represented by any of the following formulas (III-1) to (III-6), and more preferably a polymerizable compound represented by any of the following formulas (VI-1) to (VI-3).

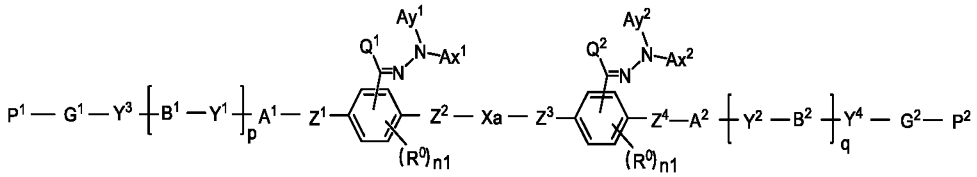
(III-1)

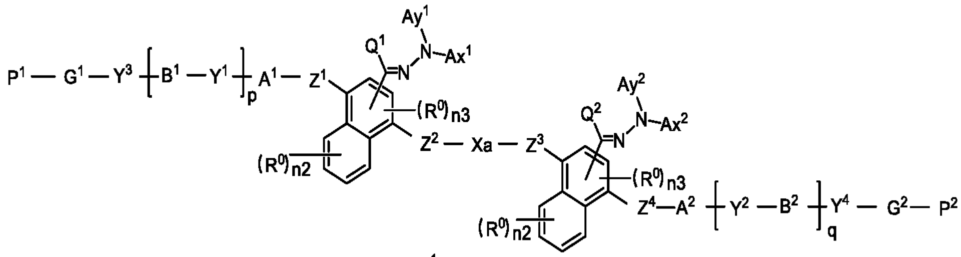
(III-2)

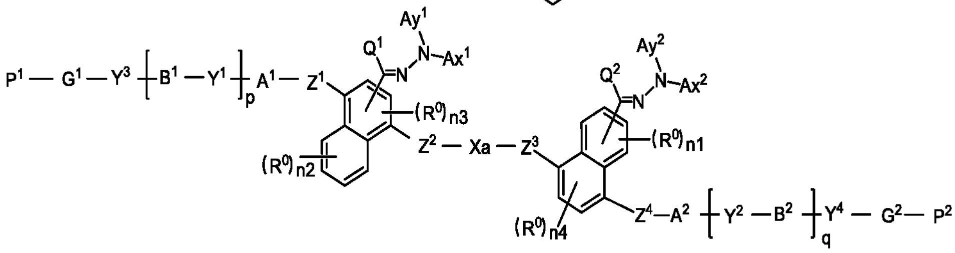
(III-3)

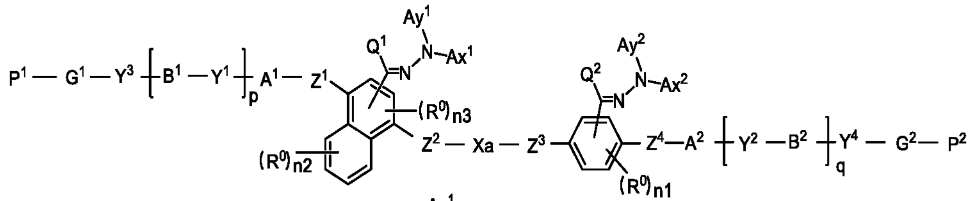
(III-4)

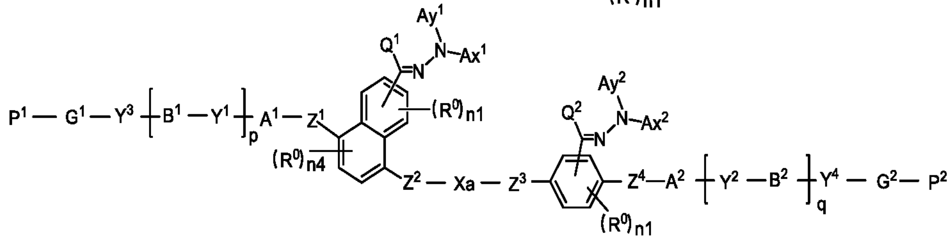
(III-5)

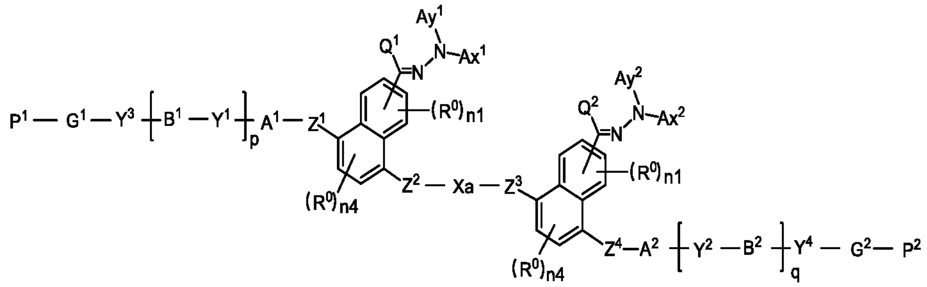
(III-6)

where in the formulas (III-1) to (III-6),
$Z^1$ to $Z^4$, $A^1$, $A^2$, $B^1$, $B^2$, $Y^1$ to $Y^4$, $G^1$, $G^2$, $P^1$, $P^2$, Xa, $R^0$, n1, n2, n3, n4, p, and q are the same as defined above, and preferred examples thereof are also the same as stated above.

Note that n1, n2, n3, and n4 may be the same or different on both sides of Xa.

$Ax^1$ and $Ax^2$ each independently represent an organic group having at least one aromatic ring selected from the group consisting of an aromatic hydrocarbon ring having 6 to 30 carbon atoms and an aromatic heterocyclic ring having 2 to 30 carbon atoms, the aromatic rings of $Ax^1$ and $Ax^2$ may have a substituent, $Ay^1$ and $Ay^2$ each independently represent a hydrogen atom or an organic group having 1 to 30 carbon atoms which may have a substituent, and $Q^1$ and $Q^2$ each independently represent a hydrogen atom or an alkyl group having 1 to 6 carbon atoms.

Specific examples and preferred examples of $Ax^1$, $Ax^2$, $Ay^1$, $Ay^2$, $Q^1$, and $Q^2$ are the same as the specific examples and the preferred examples of Ax, Ay, and Q.

However, when a plurality of $B^1$, $B^2$, $Y^1$, $Y^2$, and $R^0$ are present, these may be the same or different.

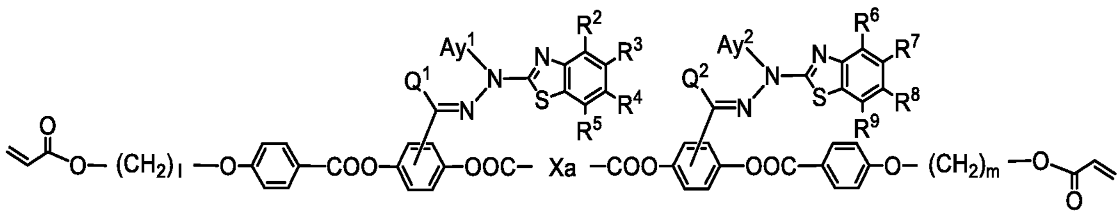
(VI-1)

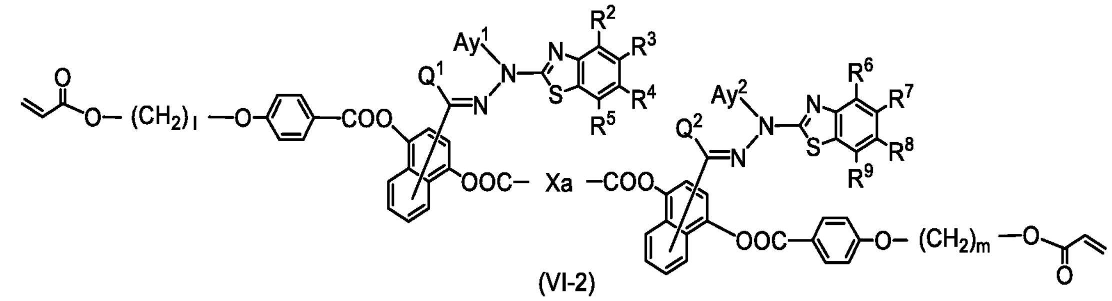

(VI-2)

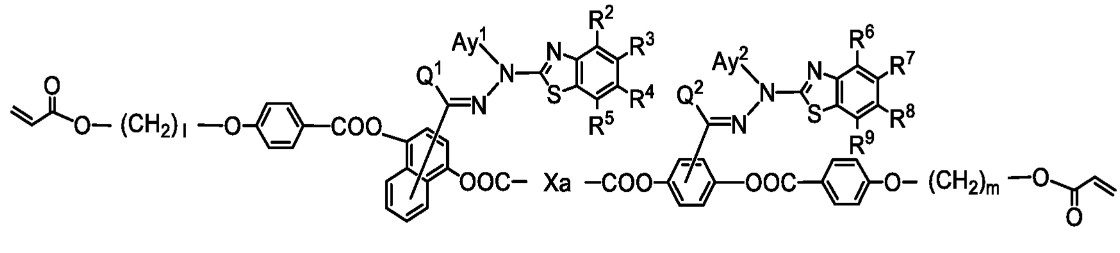

(VI-3)

where in the formulas (VI-1) to (VI-3), $R^2$ to $R^9$ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, $-OCF_3$, $-O-C(=O)-R^b$, or $-C(=O)-O-R^b$, $R^b$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or, an aromatic hydrocarbon ring group having 5 to 18 carbon atoms which may have a substituent, the plurality of $R^2$ to $R^9$ may be the same or different, and one or more ring constituents $C-R^2$ to $C-R^9$ may be replaced by a nitrogen atom.

Specific examples and preferred examples of $R^2$ to $R^9$ are the same as the specific examples and the preferred examples of $R^2$ to $R^5$ of the formula (V).

$Ay^1$, $Ay^2$, $Q^1$ and $Q^2$ are the same as defined above, and preferred examples thereof are also the same as stated above. Further, 1 and m each independently represent an integer from 1 to 18.

The polymerizable compound (I-1) described above may be produced by a combination of known synthesis reactions. That is, it may be synthesized by referring to the methods described in various literature (for example, WO 2012/141245, WO 2012/147904, WO 2014/010325, WO 2013/046781, WO 2013/180217, WO 2014/061709, WO 2014/065176, WO 2014/126113, WO 2015/025793, WO 2015/064698, JP2015-140302 A, WO 2015/129654, WO 2015/141784, WO 2016/159193, WO 2012/169424, WO 2012/176679, WO 2015/122385.

(2-1) Polymerizable liquid crystal mixture

As stated above, the polymerizable liquid crystal mixture of the present disclosure is a mixture containing the aforementioned polymerizable compound (I-1) as a main component, and the mixture and a polymerizable composition containing the mixture can be advantageously used when preparing a polymer, an optical film, and an optically anisotropic body which will be described later. A "main component" means a "component with the highest content ratio in terms of solid content".

When the polymerizable compound (I-1) does not show liquid crystal properties, the mixture can be made to show liquid crystal properties by mixing with a polymerizable compound which exhibits liquid crystal properties.

The mixing ratio of the aforementioned polymerizable compound (I-1) in the polymerizable liquid crystal mixture is preferably more than 50 mass% and less than 100 mass% in terms of solid content, more preferably not less than 55 mass% to less than 100 mass%, and particularly preferably not less than 60 mass% to less than 100 mass%.

The mixing ratio of the polymerizable compound (I-1) can be measured by high-performance liquid chromatography (HPLC).

The components other than the aforementioned polymerizable compound (I-1) in the polymerizable liquid crystal mixture are not specifically limited, examples thereof include a polymerizable compound having a different chemical structure than the aforementioned polymerizable compound (I-1), such as by-products produced when preparing the aforementioned polymerizable compound (I-1), a polymerizable compound represented by the following formula (1-2) (which may be hereinafter referred to as the "polymerizable compound (1-2)"), and a copolymerizable monomer which

is described later, and preferred forms thereof are the same.

In the polymerizable liquid crystal mixture of the present disclosure, as described above, the polymerizable compound (I-1) is included as a main component, but when including the polymerizable compound (I-1) and a polymerizable compound having a different chemical structure than the polymerizable compound (I-1), an area value of the polymerizable compound (I-1) measured by high-performance liquid chromatography (HPLC) is preferably a value greater than 50%, more preferably not less than 55% to less than 100%, and particularly preferably not less than 60% to less than 100%, of a sum of area values of the polymerizable compound (I-1) and the polymerizable compound having a different chemical structure than the polymerizable compound (I-1).

By the area value being a value greater than 50%, it is possible to produce an optical film which has a good balance of lightness and saturation and which can improve reverse wavelength dispersion on the short wavelength side while achieving reverse wavelength dispersion on the longer wavelength side. In addition, by the area value being in a more preferable range or a particularly preferable range, it is possible to produce an optical film which has an even better balance of lightness and saturation and which can further improve reverse wavelength dispersion on the short wavelength side while achieving reverse wavelength dispersion on the longer wavelength side.

(2-2) Polymerizable compound (1-2)

The polymerizable compound (I-2) is represented by the following formula (I-2):

$$P^3-G^3-Y^7\left[B^3-Y^5\right]_{p1}A^3-Z^5-Ar^2-Z^6-A^4\left[Y^6-B^4\right]_{q1}Y^8-G^4-P^4$$

(I-2)

where in the formula (I-2),

$Ar^2$ represents the same as defined above for $Ar^0$ and $Ar^1$, and preferred examples thereof are the same as those of $Ar^0$ and $Ar^1$,
$Z^5$ and $Z^6$ each independently represent the same as defined above for $Z^1$ to $Z^4$, and preferred examples thereof are the same as those of $Z^1$ to $Z^4$,
$A^3$, $A^4$, $B^3$ and $B^4$ each independently represent the same as defined above for $B^1$ and $B^2$, and preferred examples thereof are the same as those of $B^1$ and $B^2$,
$Y^5$ to $Y^8$ each independently represent the same as defined above for $Y^1$ to $Y^4$, and preferred examples thereof are the same as those of $Y^1$ to $Y^4$,
$G^3$ and $G^4$ each independently represent the same as defined above for $G^1$ and $G^2$, and preferred examples thereof are the same as those of $G^1$ and $G^2$,
$P^3$ and $P^4$ each independently represent the same as defined above for $P^1$ and $P^2$, and preferred examples thereof are the same as those of $P^1$ and $P^2$, and
p1 and q1 each independently represent the same as defined above for p and q, and preferred examples thereof are the same as those of p and q,
where when a plurality of $B^3$ to $B^4$ and $Y^5$ to $Y^6$ are present, these may be the same or different.

When the polymerizable compound (I-1) and the polymerizable compound (I-2) are included in the polymerizable liquid crystal mixture of the present disclosure, an area value of the polymerizable compound (I-1) measured by high-performance liquid chromatography (HPLC) is preferably a value greater than 50%, more preferably not less than 55 mass% to less than 100 mass%, and particularly preferably not less than 60 mass% to less than 100 mass%, of a sum of area values of the polymerizable compound (I-1) and the polymerizable compound (I-2).

By the area value being a value greater than 50%, it is possible to produce an optical film which has a better balance of lightness and saturation and which can improve reverse wavelength dispersion on the short wavelength side while achieving reverse wavelength dispersion on the longer wavelength side. In addition, by the area value being in a more preferable range or a particularly preferable range, it is possible to produce an optical film which has an even better balance of lightness and saturation and which can further improve reverse wavelength dispersion on the short wavelength side while achieving reverse wavelength dispersion on the longer wavelength side.

(2-3) Polymerizable composition

The aforementioned polymerizable composition includes at least the polymerizable compound (I-1) and a polymerization initiator, and preferably, comprises the aforementioned polymerizable liquid crystal mixture (a mixture

containing the polymerizable compound (I-1) as a main component), the polymerization initiator, and a solvent.

Note that as described later, the aforementioned polymerizable composition is useful as a material for producing the polymer, the optical film, and the optically anisotropic body of the present disclosure. Moreover, the polymerizable composition of the present disclosure can suitably produce an optical film which has a better balance of lightness and saturation and which can improve reverse wavelength dispersion on the short wavelength side while achieving reverse wavelength dispersion on the longer wavelength side.

Here, the polymerization initiator is blended from the viewpoint of more efficiently performing the polymerization reaction of the polymerizable compound (I-1) contained in the polymerizable composition.

Moreover, examples of the polymerization initiator to be used include a radical polymerization initiator, an anionic initiator, and a cationic polymerization initiator.

Examples of the radical initiator include: a thermal radical generator which is a compound that generates an active species that initiates the polymerization of the polymerizable compound upon heating; and a photo-radical generator which is a compound that generates an active species that can initiate the polymerization of the polymerizable compound upon exposure to exposure light such as visible light, ultraviolet rays (e.g., i-line), far ultraviolet rays, electron beam, and X-rays. However, it is preferable to use the photo-radical generator.

Examples of the photo-radical generator include an acetophenone-based compound, a biimidazole-based compound, a triazine-based compound, an O-acyloxime-based compound, an onium salt-based compound, a benzoin-based compound, a benzophenone-based compound, an $\alpha$-diketone compound, a polynuclear quinone-based compound, a xanthone-based compound, a diazo-based compound, and an imide sulfonate-based compound. These compounds generate either or both of active radicals and an active acid upon exposure. These photo-radical generators may be used either alone or in combination.

Specific examples of the acetophenone-based compound include 2-hydroxy-2-methyl-1-phenylpropan-1-one, 2-methyl-1-[4-(methylthio)phenyl]-2-morpholinopropan-1-one, 2-benzyl-2-dimethylamino-1-(4-morpholinophenyl)butan-1-one, 1-hydroxycyclohexyl phenyl ketone, 2,2-dimethoxy-1,2-diphenylethan-1-one, and 1-[4-(phenylthio)phenyl]-octane-1,2-dione-2-(O-benzoyloxime).

Specific examples of the biimidazole-based compound include 2,2'-bis(2-chlorophenyl)-4,4',5,5'-tetrakis(4-ethoxycarbonylphenyl)-1,2'-biimidazole, 2,2'-bis(2-bromophenyl)-4,4',5,5'-tetrakis(4-ethoxycarbonylphenyl)-1,2'-biimidazole, 2,2'-bis(2-chlorophenyl)-4,4',5,5'-tetraphenyl-1,2'-biimidazole, 2,2'-bis(2,4-dichlorophenyl)-4,4',5,5'-tetraphenyl-1,2'-biimidazole, 2,2'-bis(2,4-dichlorophenyl)-4,4',5,5'-tetraphenyl-1,2'-biimidazole, 2,2'-bis(2,4,6-trichlorophenyl)-4,4',5,5'-tetraphenyl-1,2'-biimidazole, 2,2'-bis(2-bromophenyl)-4,4',5,5'-tetraphenyl-1,2'-biimidazole, 2,2'-bis(2,4-dibromophenyl)-4,4',5,5',2'-biimidazole, and 2,2'-bis(2,4,6-tribromophenyl)-4,4',5,5'-tetraphenyl-1,2'-biimidazole.

Note that, in the present disclosure, when using a biimidazole-based compound as a photoinitiator (photo-radical generator), it is preferable to use a hydrogen donor in combination with the biimidazole-based compound in order to further improve sensitivity.

As used herein, the term "hydrogen donor" refers to a compound which can donate a hydrogen atom to radicals generated by the biimidazole-based compound upon exposure to light. A mercaptan-based compound, an amine-based compound defined below, and the like are preferable as the hydrogen donor.

Examples of the mercaptan-based compound include 2-mercaptobenzothiazole, 2-mercaptobenzoxazole, 2-mercaptobenzimidazole, 2,5-dimercapto-1,3,4-thiadiazole, and 2-mercapto-2,5-dimethylaminopyridine. Examples of the amine-based compound include 4,4'-bis(dimethylamino)benzophenone, 4,4'-bis(diethylamino)benzophenone, 4-diethylaminoacetophenone, 4-dimethylaminopropiophenone, ethyl-4-dimethylaminobenzoate, 4-dimethylaminobenzoic acid, and 4-dimethylaminobenzonitrile.

Specific examples of the triazine-based compound includes triazine-based compounds that include a halomethyl group, such as 2,4,6-tris(trichloromethyl)-s-triazine, 2-methyl-4,6-bis(trichloromethyl)-s-triazine, 2-[2-(5-methylfuran-2-yl)ethenyl]-4,6-bis(trichloromethyl)-s-triazine, 2-[2-furan-2-yl)ethenyl]-4,6-bis(trichloromethyl)-s-triazine, 2-[2-(4-diethylamino-2-methylphenyl)ethenyl]-4,6-bis(trichloromethyl)-s -triazine, 2-[2-(3,4-dimethoxyphenyl)ethenyl]-4,6-bis(trichloromethyl)-s-triazine, 2-(4-methoxyphenyl)-4,6-bis(trichloromethyl)-s-triazine, 2-(4-ethoxystyryl)-4,6-bis(trichloromethyl)-s-triazine, and 2-(4-n-butoxyphenyl)-4,6-bis(trichloromethyl)-s-triazine.

Specific examples of the O-acyloxime-based compound include 1-[4-(phenylthio)phenyl]heptane-1,2-dione-2-(O-benzoyloxime), 1-[4-(phenylthio)phenyl]octane-1,2-dione-2-(O-benzoyloxime), 1-[4-(benzoyl)phenyl]octane-1,2-dione-2-(O-benzoyloxime), 1-[9-ethyl-6-(2-methylbenzoyl)-9H-carbazol-3-yl]ethanone-1-(O-acetyloxime), 1-[9-ethyl-6-(3-methylbenzoyl)-9H-carbazol-3-yl]ethanone-1-(O-acetyloxime), 1-(9-ethyl-6- benzoyl-9H-carbazol-3 -yl)ethanone-1-(O-acetyloxime), ethanone-1-[9-ethyl-6-(2-methyl-4-tetrahydrofuranylbenzoyl)-9H-carbazol-3-yl]-1-(O-acetyloxime), ethanone-1-[9-ethyl-6-(2-methyl-4-tetrahydropyranylbenzoyl)-9H-carbazol-3-yl]-1-(O-acetyloxime), ethanone-1-[9-ethyl-6-(2-methyl-5-tetrahydrofuranylbenzoyl)-9H-carbazol-3-yl]-1-(O-acetyloxime), ethanone-1-[9-ethyl-6-(2-methyl-5-tetrahydropyranylbenzoyl)-9H-carbazol-3-yl]-1-(O-acetyloxime), ethanone-1-[9-ethyl-6- {2-methyl-4-(2,2-dimethyl-1,3-dioxolanyl) benzoyl}-9H-carbazol-3-yl]-l-(O-acetyloxime), ethanone-1-[9-ethyl-6-(2-methyl-4-tetrahydrofuranylmethoxybenzoyl)-9H-carbazol-3-yl]-1-(O-acetyloxime), ethanone-1-[9-ethyl-6-(2-methyl-4-tetrahydropyranylmethoxybenzoyl)-9H-carbazol-3-yl]-1-(O-acetyloxime), ethanone-1-[9-ethyl-6-(2-methyl-5-tetrahydrofuranylmethoxybenzoyl)-9H-carbazol-3-yl]-1- (O-acetyloxime), ethanone-1-[9-ethyl-6-(2-methylbenzoyl)-9H-carbazol-3-yl]-1-(O-acetyloxime), ethanone-1-[9-ethyl-6-(2-methyl-5-tetrahydropyranylmethoxybenzoyl)-9H-carbazol-3-yl]-1-(O-acetyloxime), and ethanone-1-[9-ethyl-6-{2-methyl-4-(2,2-dimethyl-1,3 -dioxolanyl) methoxybenzoyl}-9H-carbazol-3-yl]-1-(O-acetyloxime).

Further, a commercially available product may be used directly as the photo-radical generator. Specific examples of a commercially available product that may be used as the photo-radical generator include: Irgacure 907, Irgacure 184, Irgacure 369, Irgacure 651, Irgacure 819, Irgacure 907, and Irgacure OXE02 (manufactured by BASF); and ADEKA ARKLS N1919T (manufactured by Adeka Corporation).

Examples of the anionic initiator include: an alkyllithium compound; a monolithium salt or a monosodium salt of biphenyl, naphthalene, pyrene, and the like; and a polyfunctional initiator such as a dilithium salt and a trilithium salt.

Further, examples of the cationic polymerization initiator include a proton acid such as sulfuric acid, phosphoric acid, perchloric acid, and trifluoromethanesulfonic acid; a Lewis acid such as boron trifluoride, aluminum chloride, titanium tetrachloride, and tin tetrachloride; and an aromatic onium salt or a combination of an aromatic onium salt and a reducing agent.

These polymerization initiators may be used either alone or in combination.

Note that in the aforementioned polymerizable composition, the blending ratio of the polymerization initiator is normally 0.1 to 30 parts by mass, and preferably 0.5 to 10 parts by mass, based on 100 parts by mass of the polymerizable compound in the polymerizable composition.

Further, a surfactant is preferably added to the aforementioned polymerizable composition in order to adjust the surface tension. The surfactant is not particularly limited, but a nonionic surfactant is normally preferable as the surfactant. Examples of a commercially available product that may be used as the nonionic surfactant include a nonionic surfactant which is a fluorine-containing group, a hydrophilic group, and a lipophilic group-containing oligomer, for example, the SURFLON series (S242, S243, S386, S611, S651, etc.) manufactured by AGC Seimi Chemical Co., Ltd, the Megaface series (F251, F554, F556, F562, RS-75, RS-76-E, etc.) manufactured by DIC Corporation, the Ftargent series (FTX601AD, FTX602A, FTX601ADH2, FTX650A, etc.) manufactured by Neos Co., Ltd. and the like. Further, as the surfactant, one type thereof may be solely used, and two or more types thereof may also be used in combination at any ratio.

Here, in the aforementioned polymerizable composition, the blending ratio of the surfactant is normally 0.01 to 10 parts by mass, and preferably 0.01 to 2 parts by mass, based on 100 parts by mass of the polymerizable compound in the polymerizable composition.

Furthermore, in addition to the polymerizable compound, the polymerization initiator, and the surfactant, other components may be further included to the extent that the effect of the present disclosure is not affected. Examples of the other components include a metal, a metal complex, a dye, a pigment, a fluorescent material, a phosphorescent material, a leveling agent, a thixotropic agent, a gelling agent, a polysaccharide, an ultraviolet absorber, an infrared absorber, an antioxidant, an ion exchange resin, and a metal oxide such as titanium oxide.

Further, examples of the other components include also include other copolymerizable monomers. These are not specifically limited, and examples include 4'-methoxyphenyl 4-(2-methacryloyloxyethyloxy)benzoate, biphenyl 4-(6-methacryloyloxyhexyloxy)benzoate, 4'-cyanobiphenyl 4-(2-acryloyloxyethyloxy)benzoate, 4'-cyanobiphenyl 4-(2-methacryloyloxyethyloxy)benzoate, 3',4'-difluorophenyl 4-(2-methacryloyloxyethyloxy)benzoate, naphthyl 4-(2-methacryloyloxyethyloxy)benzoate, 4-acryloyloxy-4'-decylbiphenyl, 4-acryloyloxy-4'-cyanobiphenyl, 4-(2-acryloyloxyethyloxy)-4'-cyanobiphenyl, 4-(2-methacryloyloxyethyloxy)-4'-methoxybiphenyl, 4-(2-methacryloyloxyethyloxy)-4'-(4"-fluorobenzyloxy)-biphenyl, 4-acryloyloxy-4'-propylcyclohexylphenyl, 4-methacryloyl-4'-butylbicyclohexyl, 4-acryloyl-4'-amyltolane, 4-acryloyl-4'-(3,4-difluorophenyl)bicyclohexyl, (4-amylphenyl) 4-(2-acryloyloxyethyl)benzoate, (4-(4'-propylcyclohexyl)phenyl) 4-(2-acryloyloxyethyl)benzoate, Product name: "LC-242" (BASF), trans-1,4-bis[4-[6-(acryloyloxy)hexyloxy]phenyl]cyclohexane dicarboxylate, and copolymerizable monomers such as the compounds disclosed in JP 2007-002208 A, JP 2009-173893 A, JP 2009-274984 A, JP 2010-030979 A, JP 2010-031223 A, JP 2011-006360 A and JP 2010-24438 A, WO 2012/141245, WO 2012/147904, WO 2012/169424, WO 2012/76679, WO 2013/180217, WO 2014/010325, WO 2014/061709, WO 2014/065176, WO 2014/126113, WO 2015/025793, WO 2015/064698, WO 2015/122384, and WO 2015/122385.

The blending ratio of these other components is normally 0.005 to 50 parts by mass based on 100 parts by mass of the polymerizable compound contained in the polymerizable composition.

The aforementioned polymerizable composition can normally be prepared by mixing and dissolving the polymerizable compound, the polymerization initiator, and a predetermined amount of the other components to be blended according to need in an appropriate solvent.

Examples of the organic solvent used include: ketones such as cyclopentanone, cyclohexanone, and methyl ethyl ketone; acetate esters such as butyl acetate and amyl acetate; halogenated hydrocarbons such as chloroform, dichloromethane, and dichloroethane; and ethers such as 1,4-dioxane, cyclopentyl methyl ether, tetrahydrofuran, tetrahydropyran, and 1,3-dioxolane.

(3) Polymer

The polymer of the present disclosure is obtainable by polymerizing the aforementioned polymerizable compound (I-1), the aforementioned polymerizable liquid crystal mixture, or the aforementioned polymerizable composition.

Here, the term "polymerize" means a chemical reaction in the broad sense including a normal polymerization reaction and a crosslinking reaction.

Moreover, the polymer of the present disclosure normally has a monomer unit (for example, a repeating unit (I-1)') derived from the polymerizable compound (I-1).

The structure of the repeating unit (I-1)' when using the polymerizable compound (I-1) having a polymerizable group represented by $CH_2$=$CR^1$-C(=O)-O- as $P^1$ and $P^2$ is given as one example below.

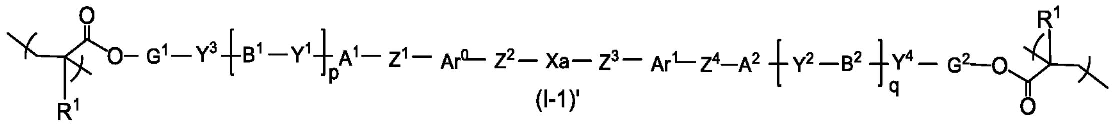

where in the formula (I-1)', $Ar^0$, $Ar^1$, $Z^1$, $Z^2$, $Z^3$, $Z^4$, $A^1$, $A^2$, $B^1$, $B^2$, $Y^1$, $Y^2$, $Y^3$, $Y^4$, $G^1$, $G^2$, $R^1$, p, and q are defined as stated above, and the preferred examples are the same.

Note that the polymer of the present disclosure is prepared using the aforementioned polymerizable compound (I-1), or the aforementioned polymerizable liquid crystal mixture, and thus, can be suitably used as the constituent material of the optical film or the like.

Further, the polymer of the present disclosure is not specifically limited, and can be used in any shape or form according to its intended use, including a film-like shape, a powder form, or a layer made of an aggregation of powder.

Specifically, a film of the polymer can be suitably used as the constituent material of the optical film and the optically anisotropic body which are described later, powders of the polymer can be utilized for paints, anti-forgery items, security items, and the like, and layers made of the polymer powder can be suitably used as the constituent material for the optically anisotropic body.

Moreover, the polymer of the present disclosure can be suitably produced for example by: (α) a method for polymerizing the aforementioned polymerizable compound (I-1), the aforementioned polymerizable liquid crystal mixture, or the aforementioned polymerizable composition, isolating the target polymer, dissolving the polymer in the presence of a suitable organic solvent to prepare a solution, applying the solution on a suitable substrate to form thereon a coating film, and then drying the coating film followed by optional heating; or (β) a method for dissolving the aforementioned polymerizable compound (I-1), the aforementioned polymerizable liquid crystal mixture, or the aforementioned polymerizable composition in an organic solvent, applying the solution on a substrate by a coating method known in the art, and then removing the solvent, and performing a polymerization reaction by heating or actinic radiation and the like. Note that the aforementioned polymerizable compound (I-1) may be polymerized alone.

The organic solvent which can be used for the polymerization by method (α) is not specifically limited as long as it is inert. Examples of the organic solvent include: aromatic hydrocarbons such as toluene, xylene, and mesitylene; ketones such as cyclohexanone, cyclopentanone, and methyl ethyl ketone; acetates such as butyl acetate and amyl acetate; halogenated hydrocarbons such as chloroform, dichloromethane, and dichloroethane; and ethers such as cyclopentyl methyl ether, tetrahydrofuran, and tetrahydropyran.

Thereamong, from the viewpoint of handling capability, organic solvents having a boiling point of 60°C to 250°C are preferable, and those having a boiling point of 60°C to 150°C are more preferable.

Further, examples of organic solvents used to dissolve the isolated polymer in method (α) and organic solvents used in method (β) include: ketone solvents such as acetone, methyl ethyl ketone, methyl isobutyl ketone, cyclopentanone, and cyclohexanone; ester solvents such as butyl acetate and amyl acetate; halogenated hydrocarbon solvents such as dichloromethane, chloroform, and dichloroethane; halogenated hydrocarbon solvents such as dichloromethane, chloroform, and dichloroethane; ether solvents such as tetrahydrofuran, tetrahydropyran, 1,2-dimethoxyethane, 1,4-dioxane, cyclopentyl methyl ether, and 1,3-dioxolane; and aprotic polar solvents such as N,N-dimethylformamide, N,N-dimethylacetamide, dimethyl sulfoxide, γ-butyrolactone, and N-methylpyrrolidone. Thereamong, organic solvents having a boiling point of the solvent of 60°C to 200°C from the viewpoint of handling capability. These solvents can be used alone or in combination.

Substrates made of any organic or inorganic material known and commonly used in the art can be used in the methods (α) and (β). Examples of the organic material include polycycloolefins such as Zeonex® and Zeonor® (Zeonex and Zeonor are registered trademarks in Japan, other countries, or both, manufactured by Zeon Corporation), Arton® (Arton is a registered trademark in Japan, other countries, or both, manufactured by JSR Corporation), and Apel® (Apel is a registered trademark in Japan, other countries, or both, manufactured by Mitsui Chemicals Inc.), polyethylene

terephthalates; polycarbonates; polyimides; polyamides; polymethyl methacrylates; polystyrenes; polyvinyl chlorides; polytetrafluoroethylene, celluloses, cellulose triacetate; and polyethersulfones. Examples of the inorganic material include silicon, glass, and calcite.

Further, the substrate may be a monolayer or a laminate.

The substrate is preferably made of organic material, more preferably a resin film formed to a film shape with an organic material.

Note that the substrate includes those used for the production of an optically anisotropic body which is described later.

Further, methods known in the art can be used for applying the polymer solution on the substrate in the method ($\alpha$) and for applying the solution for the polymerization reaction on the substrate in the method ($\beta$). Specific examples of usable coating methods include curtain coating, extrusion coating, roll coating, spin coating, dip coating, bar coating, spray coating, slide coating, print coating, gravure coating, die coating, and cap coating.

Furthermore, the drying or solvent removal in the methods ($\alpha$) and ($\beta$) can be effected by natural drying, drying by heating, drying under reduced pressure, drying by heating under reduced pressure, or the like.

The drying temperature is not specifically limited as long as the solvent can be removed, but the lower limit temperature is preferably 50°C or more, and more preferably 70°C or more from the viewpoint of stably obtaining a constant temperature.

The upper limit of the drying temperature is preferably 200°C or less, and more preferably 195°C or less from the viewpoint of not adversely affecting the substrate.

Further, examples of the method for polymerizing the aforementioned polymerizable compound (I-1), the aforementioned polymerizable liquid crystal mixture, or the aforementioned polymerizable composition include a method for irradiating with actinic radiation, a thermal polymerization method, and the like. However, the method for irradiating with actinic radiation is preferable as the reaction progresses at room temperature without requiring heating. Thereamong, the method for irradiating with light such as UV light is preferable as the operation is simple.

The temperature for irradiating light such as UV is not specifically limited as long as the liquid crystal phase can be maintained, but the lower limit temperature is preferably 15°C or more, and more preferably 20°C or more from the viewpoint that the photopolymerization can stably progress.

The upper limit of the temperature for irradiating light such as UV is preferably 200°C or less, and more preferably 195°C or less from the viewpoint of not adversely affecting the substrate.

Here, the temperature during the irradiation of light is preferably set to 30°C or less. The irradiation intensity is normally in a range from 1 $W/m^2$ to 10 $kW/m^2$, preferably in a range from 5 $W/m^2$ to 2 $kW/m^2$.

The polymer obtained as described above can be transferred from the substrate for use, removed from the substrate for single use, or used as the constituent material for optical film etc. without being removed from the substrate.

Further, the polymer removed from the substrate can also be made into a powder form by a grinding method known in the art prior to use.

The number-average molecular weight of the polymer of the present disclosure obtainable as described above is preferably 500 to 500,000, more preferably 5,000 to 300,000. When the number-average molecular weight is within these ranges, a high hardness can be obtained and the handling capability is excellent, which is desirable. The number-average molecular weight of the polymer can be determined by gel permeation chromatography (GPC) using monodisperse polystyrene as a standard with tetrahydrofuran as an eluant.

Moreover, the polymer of the present disclosure can obtain an optical film which has a better balance of lightness and saturation and which can improve reverse wavelength dispersion on the short wavelength side while achieving reverse wavelength dispersion on the longer wavelength side.

(4) Optical film

The optical film of the present disclosure is formed using the polymer and/or the polymerizable compound of the present disclosure, and includes a layer having an optical function. An optical function means a simple transmittance, reflection, refraction, birefringence, or the like. Moreover, the optical film of the present disclosure may be an optical film which contains the polymer of the present disclosure as a main constituent material of a layer having an optical function, or an optical film in which a layer having an optical function contains the polymerizable compound of the present disclosure.

Preferably, the optical film which contains the polymer of the present disclosure as a constituent material has an occupancy ratio of the polymer of the present disclosure in excess of 50 mass% when all of the components of the layer having the optical function are 100 mass%. Further, the optical film containing the polymerizable compound of the present disclosure preferably contains 0.01 mass% or more of the polymerizable compound of the present disclosure when all of the components of the layer having the optical function are 100 mass%.

Here, the optical film of the present disclosure may be used in any of the following arrangements: an arrangement (alignment substrate / (alignment film) / optical film) where the optical film remains formed on an alignment substrate

which may have an alignment film, an arrangement (transparent substrate film / optical film) where the optical film has been transferred to a transparent substrate film or the like which is different from the alignment substrate, or, a single optical film form (optical film) when the optical film is self-supportive.

Note that the alignment film and the alignment substrate can use the same substrate and alignment film as the optically anisotropic body which is described later.

Moreover, the optical film of the present disclosure can be produced by (A) a method for applying on an alignment substrate a solution containing the polymerizable compound of the present disclosure or a solution of the polymerizable liquid crystal mixture, drying the resulting coating film, subjecting the film to heat treatment (for alignment of liquid crystals), and irradiation and/or heating treatment (for polymerization), (B) a method for applying on an alignment substrate a solution of a liquid crystal polymer obtainable by polymerization of the polymerizable compound of the present disclosure, or the polymerizable liquid crystal mixture, and optionally drying the resulting coated film, or (C) a method for applying on an alignment substrate a solution containing the polymerizable compound of the present disclosure and resin, and drying the obtained coated film.

The optical film of the present disclosure can be used for an optically anisotropic body, alignment films for liquid crystal display devices, color filters, low-pass filters, polarization prisms, and various optical filters.

Note that the optical film of the present disclosure preferably has a wavelength dispersion ratio as close as possible to an ideal value, which is determined as described below from the retardation at wavelengths of 400 nm, 410 nm, 420 nm, 430 nm, 440 nm, 450 nm, 550 nm, 600 nm, 650 nm, 700 nm, 750 nm, and 800 nm measured by a Mueller Matrix Polarimeter Axoscan. Specifically, the ideal value of the wavelength dispersion ratio at 400 nm is 0.7273, the ideal value of the wavelength dispersion ratio at 410 nm is 0.7455, the ideal value of the wavelength dispersion ratio at 420 nm is 0.7636, the ideal value of the wavelength dispersion ratio at 430 nm is 0.7818, the ideal value of the wavelength dispersion ratio at 440 nm is 0.8000, the ideal value of the wavelength dispersion ratio at 450 nm is 0.8182, the ideal value of the wavelength dispersion ratio at 600 nm is 1.0909, the ideal value of the wavelength dispersion ratio at 650 nm is 1.1818, the ideal value of the wavelength dispersion ratio at 700 nm is 1.2727, the ideal value of the wavelength dispersion ratio at 750 nm is 1.3636, and the ideal value of the wavelength dispersion ratio at 800 nm is 1.4545.

Further, in the optical film of the present disclosure, the wavelength dispersion ratio at 420 nm is preferably 0.60 to 0.82, and the wavelength dispersion ratio at 440 nm is preferably 0.75 to 0.85.

(wavelength dispersion ratio at 400 nm) = (retardation value at 400 nm) / (retardation value at 550 nm)

(wavelength dispersion ratio at 410 nm) = (retardation value at 410 nm) / (retardation value at 550 nm)

(wavelength dispersion ratio at 420 nm) = (retardation value at 420 nm) / (retardation value at 550 nm)

(wavelength dispersion ratio at 430 nm) = (retardation value at 430 nm) / (retardation value at 550 nm)

(wavelength dispersion ratio at 440 nm) = (retardation value at 440 nm) / (retardation value at 550 nm)

(wavelength dispersion ratio at 450 nm) = (retardation value at 450 nm) / (retardation value at 550 nm)

(wavelength dispersion ratio at 600 nm) = (retardation value at 600 nm) / (retardation value at 550 nm)

(wavelength dispersion ratio at 650 nm) = (retardation value at 650 nm) / (retardation value at 550 nm)

(wavelength dispersion ratio at 700 nm) = (retardation value at 700 nm) / (retardation value at 550 nm)

(wavelength dispersion ratio at 750 nm) = (retardation value at 750 nm) / (retardation value at 550 nm)

(wavelength dispersion ratio at 800 nm) = (retardation value at 800 nm) / (retardation value at 550 nm)

In the optical film of the present disclosure, the difference between the retardation where the lightness is the lowest and the retardation where the saturation is the lowest is not specifically limited, and is, for example, 5.5 nm or less, preferably 3.5 nm or less.

(5) Optically anisotropic body

The optically anisotropic body of the present disclosure has a layer which contains the polymer of the present disclosure as a constituent material.

The optically anisotropic body of the present disclosure can be obtained, for example, by forming an alignment film on a substrate and forming a layer (liquid crystal layer) made of the polymer of the present disclosure on the alignment film. Note that the optically anisotropic body of the present disclosure may be obtained by directly forming a layer (liquid crystal layer) made of the polymer of the present disclosure on the substrate, or may consist only of a layer (liquid crystal layer) made of the polymer of the present disclosure.

Note that the layer made of the polymer may be formed of a film-like polymer or may be an aggregate of powdery polymer.

Here, the alignment film is formed on the surface of the substrate to align and regulate the polymerizable compound in one direction in the plane.

The alignment film can be obtained by applying a solution (alignment film composition) containing a polymer such as polyimide, polyvinyl alcohol, polyester, polyarylate, polyamideimide, or polyetherimide in a film-like form on the substrate, drying the film, and rubbing the film in one direction.

The thickness of the alignment film is preferably 0.001 to 5 μm, and more preferably 0.001 to 1.0 μm.

The method of the rubbing treatment is not specifically limited, but, for example, the alignment film may be rubbed in a certain direction using a roll around which a cloth or felt formed of a synthetic fiber such as nylon or a natural fiber such as cotton is wound. The alignment film is preferably washed with isopropyl alcohol or the like after the rubbing treatment in order to remove fine powders (foreign substances) formed during the rubbing treatment to clean the surface of the alignment film.

Further, other than the rubbing treatment, the alignment film can be provided with a function for aligning and regulating in one direction in the plane even by a method for irradiating the surface of the alignment film with polarized UV light.

Examples of substrates on which the alignment film is to be formed include glass substrates and substrates formed of synthetic resin films. Examples of synthetic resins include thermoplastic resins such as acrylic resins, polycarbonate resins, polyethersulfone resins, polyethylene terephthlate resins, polyimide resins, polymethyl methacrylate resins, polysulfone resins, polyarylate resins, polyethylene resins, polystyrene resins, polyvinyl chloride resins, cellulose diacetate, cellulose triacetate, and alicyclic olefin polymers.

Examples of the alicyclic olefin polymers include: cyclic olefin random multi-component copolymers described in JP H05-310845 A and US 5179171 B; hydrogenated polymers described in JP H05-97978 A and US 5202388 B; and thermoplastic dicyclopentadiene open-ring polymers and hydrogenated products thereof described in JP H11-124429 A (WO 99/20676).

In the present disclosure, examples of methods for forming a liquid crystal layer made of the polymer of the present disclosure on the alignment film are the same as the methods described above for the polymer of the present disclosure (the methods (α) and (β)).

The thickness of the resulting liquid crystal layer is not specifically limited, but normally is 1 to 10 μm.

Note that examples of the optically anisotropic body of the present disclosure include, but are not specifically limited to, a retardation plate and a viewing-angle enhancing film.

Note that the optically anisotropic body of the present disclosure preferably has a wavelength dispersion ratio as close as possible to an ideal value, which is determined as described below from the retardation at wavelengths of 400 nm, 410 nm, 420 nm, 430 nm, 440 nm, 450 nm, 550 nm, 600 nm, 650 nm, 700 nm, 750 nm, and 800 nm measured by a Mueller Matrix Polarimeter Axoscan. Specifically, the ideal value of the wavelength dispersion ratio at 400 nm is 0.7273, the ideal value of the wavelength dispersion ratio at 410 nm is 0.7455, the ideal value of the wavelength dispersion ratio at 420 nm is 0.7636, the ideal value of the wavelength dispersion ratio at 430 nm is 0.7818, the ideal value of the wavelength dispersion ratio at 440 nm is 0.8000, the ideal value of the wavelength dispersion ratio at 450 nm is 0.8182, the ideal value of the wavelength dispersion ratio at 600 nm is 1.0909, the ideal value of the wavelength dispersion ratio at 650 nm is 1.1818, the ideal value of the wavelength dispersion ratio at 700 nm is 1.2727, the ideal value of the wavelength dispersion ratio at 750 nm is 1.3636, and the ideal value of the wavelength dispersion ratio at 800 nm is 1.4545.

Further, in the optically anisotropic body of the present disclosure, the wavelength dispersion ratio at 420 nm is preferably 0.60 to 0.82, and the wavelength dispersion ratio at 440 nm is preferably 0.75 to 0.85.

(wavelength dispersion ratio at 400 nm) = (retardation value at 400 nm)/(retardation value at 550 nm)

(wavelength dispersion ratio at 410 nm) = (retardation value at 410 nm) / (retardation value at 550 nm)

(wavelength dispersion ratio at 420 nm) = (retardation value at 420 nm) / (retardation value at 550 nm)

(wavelength dispersion ratio at 430 nm) = (retardation value at 430 nm) / (retardation value at 550 nm)

(wavelength dispersion ratio at 440 nm) = (retardation value at 440 nm) / (retardation value at 550 nm)

(wavelength dispersion ratio at 450 nm) = (retardation value at 450 nm) / (retardation value at 550 nm)

(wavelength dispersion ratio at 600 nm) = (retardation value at 600 nm) / (retardation value at 550 nm)

(wavelength dispersion ratio at 650 nm) = (retardation value at 650 nm) / (retardation value at 550 nm)

(wavelength dispersion ratio at 700 nm) = (retardation value at 700 nm) / (retardation value at 550 nm)

(wavelength dispersion ratio at 750 nm) = (retardation value at 750 nm) / (retardation value at 550 nm)

(wavelength dispersion ratio at 800 nm) = (retardation value at 800 nm) / (retardation value at 550 nm)

In the optically anisotropic body of the present disclosure, the difference between the retardation where the lightness is the lowest and the retardation where the saturation is the lowest is not specifically limited, and is, for example, 5.5 nm or less, preferably 3.5 nm or less, more preferably 2.5 nm or less, and particularly preferably 0.5 nm or less.

(6) Polarizing plate and the like

The polarizing plate of the present disclosure includes the optically anisotropic body of the present disclosure and a polarizing film.
A specific example of the polarizing plate of the present disclosure is obtained by laminating the optically anisotropic body of the present disclosure on a polarizing film either directly or with other layer(s) (a glass plate, etc) interposed therebetween.
The production method of the polarizing film is not specifically limited. Examples of the method for producing a PVA polarizing film include: a method wherein iodine ions are adsorbed onto a PVA film followed by uniaxial stretching of the PVA film; a method wherein a PVA film is uniaxially stretched followed by adsorption of iodine ions; a method wherein adsorption of iodine ions to a PVA film and uniaxial stretching are simultaneously performed; a method wherein a PVA film is dyed with dichroic dye followed by uniaxial stretching; a method wherein a PVA film is uniaxially stretched followed by dying with dichroic dye; and a method wherein dying of a PVA film with dichroic dye and uniaxial stretching are simultaneously performed. Further, examples of methods of manufacturing a polyene polarizing film include known methods in the art such as a method wherein a PVA film is uniaxially stretched followed by heating and dehydration in the presence of a dehydration catalyst, and a method wherein a polyvinyl chloride film is uniaxially stretched followed by heating and dehydration in the presence of a dechlorination catalyst.
In the polarizing plate of the present disclosure, the polarizing film and optically anisotropic body of the present disclosure may be bonded with an adhesive layer consisting of an adhesive (including tackifier). The average thickness of the adhesive layer is normally 0.01 μm to 30 μm, preferably 0.1 μm to 15 μm. The adhesive layer preferably has a tensile fracture strength of 40 MPa or less as measured in accordance with JIS K7113.
Examples of adhesives for the adhesive layer include acrylic adhesives, urethane adhesives, polyester adhesives, polyvinyl alcohol adhesives, polyolefin adhesives, modified polyolefin adhesives, polyvinyl alkyl ether adhesives, rubber adhesives, vinyl chloride-vinyl acetate adhesives, styrene butadiene styrene copolymer (SBS copolymer) adhesives, and their hydrogenated product (SEBS copolymer) adhesives, ethylene adhesives such as ethylene-vinyl acetate copolymers and ethylene-styrene copolymers, and acrylic acid ester adhesives such as ethylene-methyl methacrylate copolymer, ethylene-methyl acrylate copolymer, ethylene-ethyl methacrylate copolymer, and ethylene-ethyl acrylate copolymer.
The polarizing plate of the present disclosure uses the optically anisotropic body of the present disclosure, and thus, has a better balance of lightness and saturation, and can improve reverse wavelength dispersion on the short wavelength side while achieving reverse wavelength dispersion on the longer wavelength side.
Further, a display device having a panel and an antireflection film can be preferably produced using the polarizing plate of the present disclosure. Examples of the panel include a liquid crystal panel and an organic electroluminescence panel. Examples of the display device include a flat panel display device having a polarizing plate and a liquid crystal panel, and an organic electroluminescence display device having a liquid crystal panel and an organic electroluminescence panel.

(7) Compound

The compound of the present disclosure is a compound represented by any of the following formulas (XI-1) to (XI-6). Hereinbelow, the compound represented by the following formula (XI-1) may be referred to as "compound (XI-1)", the compound represented by the following formula (XI-2) as "compound (XI-2)", the compound represented by the following formula (XI-3) as "compound (XI-3)", the compound represented by the following formula (XI-4) as "compound (XI-4)", the compound represented by the following formula (XI-5) as "compound (XI-5)", and the compound represented by the following formula (XI-6) as "compound (XI-6)".

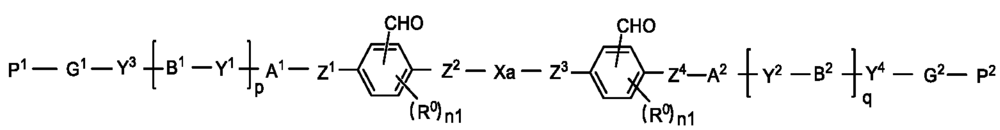

(XI-1)

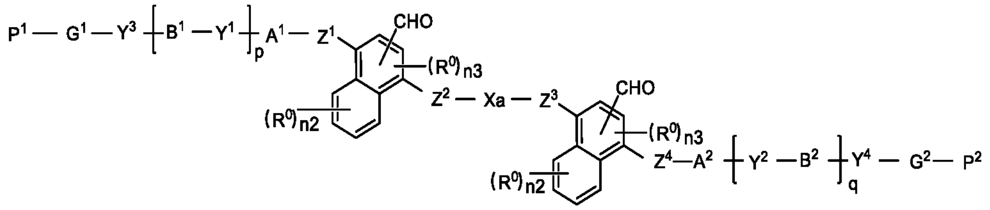

(XI-2)

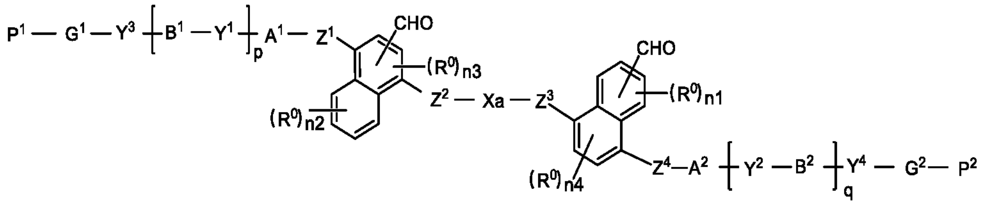

(XI-3)

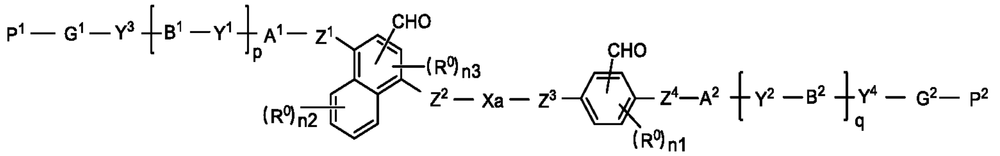

(XI-4)

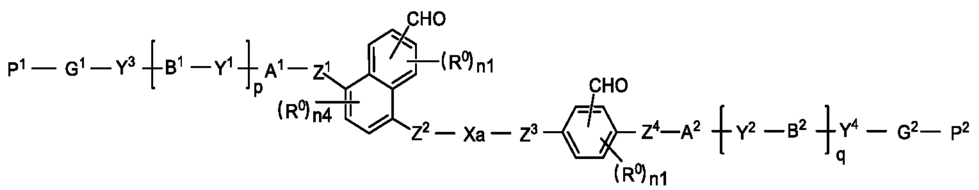

(XI-5)

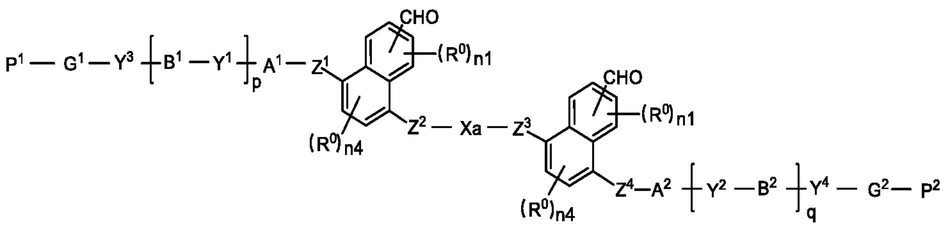

(XI-6)

In formulas (XI-1) to (XI-6), Xa, $Z^1$ to $Z^4$, $A^1$, $A^2$, $B^1$, $B^2$, $Y^1$ to $Y^4$, $G^1$, $G^2$, $P^1$, $P^2$, p, q, $R^0$, and n1, n2, n3, and n4 are the same as defined above, and preferred examples thereof are also the same as stated above.

However, when a plurality of $R^0$, $B^1$, $B^2$, $Y^1$, and $Y^2$ are present, these may be the same or different.

The compound represented by formulas (XI-1) to (XI-6) described above may be produced by a combination of known synthesis reactions using the compounds represented by the aforementioned formulas (X-1) to (X-6) as the material. That is, they may be synthesized by referring to the methods described in various literature (for example, WO 2012/141245, WO 2012/147904, WO 2014/010325, WO 2013/046781, WO 2013/180217, WO 2014/061709, WO 2014/065176, WO 2014/126113, WO 2015/025793, WO 2015/064698, JP 2015-140302 A, WO 2015/129654, WO 2015/141784, WO 2016/159193, WO 2012/169424, WO 2012/176679, WO 2015/122385.

Furthermore, among the compounds represented by formula (XI-1), the compound represented by the following formula (XII-1) is preferable. Further, among the compounds represented by the formulas (XI-2), (XI-3), and (XI-6), the compound represented by the following formula (XII-2) is preferable. Further, among the compounds represented by the formulas (XI-4) and (XI-5), the compound represented by the following formula (XII-3) is preferable.

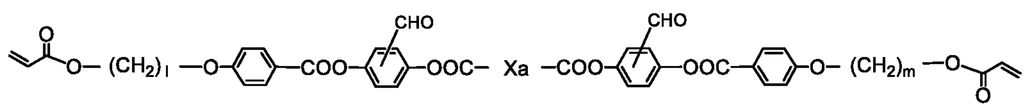

(XII-1)

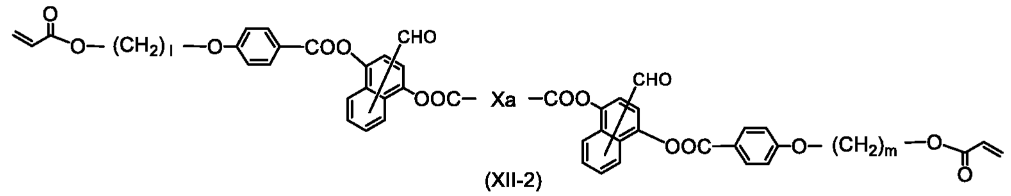

(XII-2)

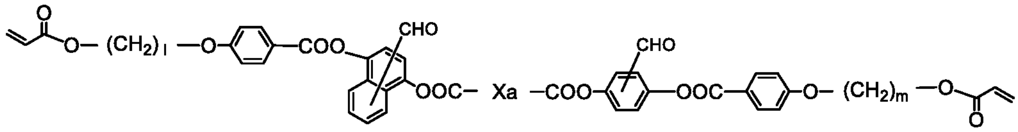

(XII-3)

In the formulas (XII-1) to (XII-3), Xa is the same as defined above, and the preferred examples are the same. 1 and m each independently represent an integer from 1 to 18.

Moreover, the compounds represented by formulas (XII-1) to (XII-3) described above may be produced by a combination of known synthesis reactions. That is, they may be synthesized by referring to the methods described in various literature (for example, WO 2012/141245, WO 2012/147904, WO 2014/010325, WO 2013/046781, WO 2013/180217, WO 2014/061709, WO 2014/065176, WO 2014/126113, WO 2015/025793, WO 2015/064698, JP 2015-140302 A, WO 2015/129654, WO 2015/141784, WO 2016/159193, WO 2012/169424, WO 2012/176679, WO 2015/122385.

The aforementioned polymerizable compound (I-1) can be obtained from the compounds represented by the formulas (XII-1) to (XII-3) by utilizing a CHO portion in a known synthesis reaction in the formulas (XII-1) to (XII-3).

EXAMPLES

The present disclosure will be described below in detail with reference to examples. However, the present disclosure is not limited to the following examples.

(Synthesis Example 1: Synthesis of Compound 1 (Example of the compound represented by the formula (VI-1))

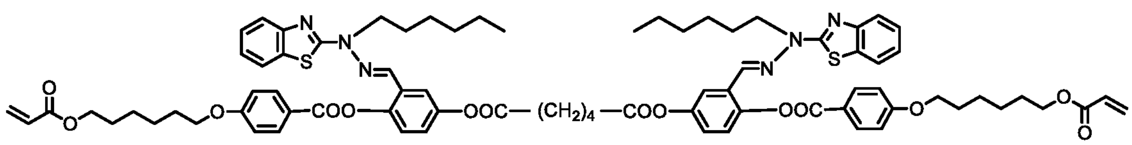

... Compound 1

<Step 1: Synthesis of Intermediate A>

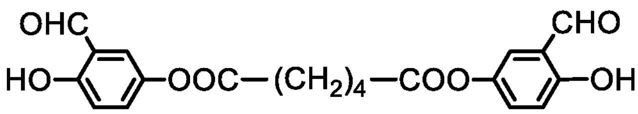

...Intermediate A

A three-necked reactor equipped with a thermometer was charged with 10 g (68.4 mmol) of adipic acid, 18.9 g (136.9 mmol) of 2,5-dihydroxybenzaldehyde, 836 mg (6.84 mmol) of N-N-dimethylaminopyridine, and 250 ml of chloroform under a nitrogen stream. The solution was placed in an ice bath and cooled to 0°C, and then, 20.7 g (164.3 mmol) of N-N'-diisopropylcarbodiimide was added thereto. Then, the solution was stirred at 25°C for 20 hours. After completion of the reaction, the resulting precipitate was filtered. The obtained filtered matter was charged in 500 ml of methanol,

and was stirred and washed for 1 hour at room temperature. Filtration was performed again, and the filtered matter washed in 500 ml of methanol to obtain 16 g of Intermediate A as a white solid. The yield was 62.8 mol%. The structure of Intermediate A was identified by $^1$H-NMR. The $^1$H-NMR spectral data is presented below.

$^1$H-NMR (500 MHz, DMSO-$d_6$, TMS, δppm): 10.84 (s, 2H), 10.25 (s, 2H), 7.35 (d, 2H, J = 3.0 Hz), 7.29 (dd, 2H, J = 3.0 Hz, 9.0 Hz), 7.02 (d, 2H, J = 9.0 Hz), 2.65-2.60 (m, 4H), 1.75-1.69 (m, 4H).

<Step 2: Synthesis of Intermediate B (Example of the compound represented by the formula (XII-1))>

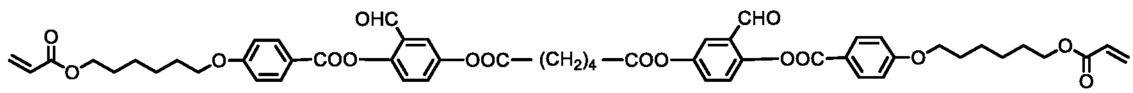

...Intermediate B

5.0 g (12.9 mmol) of Intermediate A obtained in Step 1, 9.45 g (32.3 mmol) of 4-(6-acryloyl-hex-1-yloxy)benzoic acid (manufactured by DKSH), and 15.9 mg (0.13 mmol) of N-N-dimethylaminopyridine were added to 200 ml of N-methylpyrrolidone in a three-necked reactor equipped with a thermometer under a nitrogen stream. 7.4 g (38.8 mmol) of 1-ethyl-3-(3-dimethylaminopropyl)carbodiimidehydrochloride was added thereto while gently stirring at 25 °C. Then, the solution was stirred at 25°C for 15 hours to perform the reaction. After completion of the reaction, 1 liter of distilled water and 100 ml of saturated saline solution were added to the obtained reaction solution, followed by extraction twice with 500 ml of ethyl acetate. The obtained organic layer was dried with anhydrous sodium sulfate, and the sodium sulfate was filtered off. After 250 ml of the solvent was distilled off with a rotary evaporator, the obtained organic layer was gradually dropped into 2 liters of methanol. The precipitated solid was collected by filtration. The obtained solid was vacuum dried to obtain 8.59 g of Intermediate B as a white solid. The yield was 72.3 mol%. The structure of Intermediate B was identified by $^1$H-NMR. The $^1$H-NMR spectral data is presented below.

$^1$H-NMR (500 MHz, $CDCl_3$ , TMS, δppm): 10.19 (s, 2H), 8.16 (d, 4H, J = 9.0 Hz), 7.68 (d, 2H, J = 3.0 Hz), 7.42 (dd, 2H, J = 3.0 Hz, 9.0 Hz), 7.35 (d, 2H, J = 9.0 Hz), 6.99 (d, 4H, J = 9.0 Hz), 6.41 (dd, 2H, J = 1.5 Hz, 17.0 Hz), 6.13 (dd, 2H, J = 10.5 Hz, 17.0 Hz), 5.83 (dd, 2H, J = 1.5 Hz, 10.5 Hz), 4.19 (t, 4H, J = 6.5 Hz), 4.07 (t, 4H, J = 6.5 Hz), 2.70-2.68 (m, 4H), 1.94-1.83 (m, 8H), 1.76-1.71 (m, 4H), 1.59-1.45 (m, 8H).

<Step 3: Synthesis of Intermediate C>

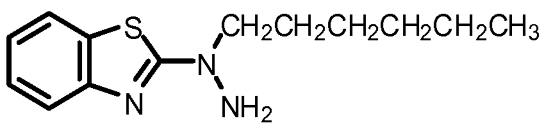

...Intermediate C

2.00 g (12.1 mmol) of 2-hydrazinobenzothiazole was dissolved in 20 ml of dimethylformamide in a four-necked reactor equipped with a thermometer under a nitrogen stream. 8.36 g (60.5 mmol) of potassium carbonate and 3.08 g (14.5 mmol) of 1-iodohexane were added to this solution, which was stirred at 50°C for 7 hours. After completion of the reaction, the reaction solution was cooled to 20°C, the reaction solution was charged in 200 ml of water, and extracted with 300 ml of ethyl acetate. The ethyl acetate layer was dried with anhydrous sodium sulfate. After the sodium sulfate was filtered off, and ethyl acetate was evaporated under reduced pressure using a rotary evaporator to obtain a yellow solid. The yellow solid was purified by silica gel column chromatography (hexane:ethyl acetate = 75:25 (volume ratio)) to obtain 2.10 g of Intermediate C as a white solid. The yield was 69.6 mol%. The structure of Intermediate C was identified by $^1$H-NMR. The $^1$H-NMR spectral data is presented below.

$^1$H-NMR (500 MHz, $CDCl_3$, TMS, δppm): 7.60 (dd, 1H, J = 1.0, 8.0 Hz), 7.53 (dd, 1H, J = 1.0, 8.0 Hz), 7.27 (ddd, 1H, J = 1.0, 8.0, 8.0 Hz), 7.06 (ddd, 1H, J = 1.0, 8.0, 8.0 Hz), 4.22 (s, 2H), 3.74 (t, 2H, J = 7.5 Hz), 1.69-1.76 (m, 2H), 1.29-1.42 (m, 6H), 0.89 (t, 3H, J = 7.0 Hz).

<Step 5: Synthesis of Compound 1 (Example of the compound represented by the formula (VI-1))>

3.0 g (3.21 mmol) of Intermediate B synthesized in Step 2, 2.0 g (8.03 mmol) of Intermediate C synthesized in Step 3, and 74 mg (0.32 mmol) of (±)-10-camphorsulfonic acid were added to a mixed solution of tetrahydrofuran 100

ml and 10 ml of ethanol in a four-necked reactor equipped with a thermometer under a nitrogen stream. The solution was stirred at 50°C for 2 hours. After completion of the reaction, the reaction solution was cooled, and charged into 500 ml of a 10 mass% sodium bicarbonate water, followed by extraction twice with 300 ml of ethyl acetate. The organic layer was collected, and dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 3.06 g of Compound 1 as a light yellow solid. The yield was 68.3 mol%. The structure of the target product (Compound 1) was identified by $^1$H-NMR. The $^1$H-NMR spectral data is presented below.

$^1$H-NMR (500 MHz, $CDCl_3$ , TMS, δppm): 8.15 (d, 4H, J = 9.0 Hz), 7.78 (d, 2H, J = 3.0 Hz), 7.61 (d, 2H, J = 8.0 Hz), 7.54-7.52 (m, 4H), 7.29-7.25 (m, 2H), 7.23 (d, 2H, J = 9.0 Hz), 7.16 (dd, 2H, J = 3.0 Hz, 9.0 Hz), 7.07 (ddd, 2H, J = 1.0 Hz, 8.0 Hz, 8.0 Hz), 7.00 (d, 4H, J = 9.0 Hz), 6.42 (dd, 2H, J = 1.5 Hz, 17.5 Hz), 6.14 (dd, 2H, J = 10.5 Hz, 17.5 Hz), 5.84 (dd, 2H, J = 1.5 Hz, 10.5 Hz), 4.19 (t, 4H, J = 6.5 Hz), 4.06 (t, 4H, J = 6.5 Hz), 4.02 (t, 4H, J = 7.5 Hz), 2.77-2.75 (m, 4H), 2.02-2.01 (m, 4H), 1.89-1.85 (m, 4H), 1.77-1.71 (m, 4H), 1.57-1.46 (m, 12H), 1.16-1.03 (m, 12H), 0.77 (t, 6H, J = 7.0 Hz).

(Synthesis Example 2: Synthesis of Compound 2 (Another example of the compound represented by the formula (VI-1)))

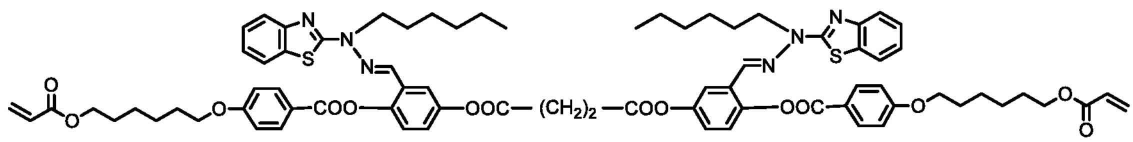

...Compound 2

<Step 1: Synthesis of Intermediate D (Example of the compound represented by the formula (X-1))>

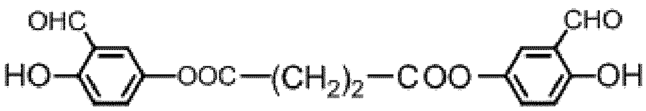

...Intermediate D

10 g (84.7 mmol) of succinic acid, 23.4 g (169.4 mmol) of 2,5-dihydroxybenzaldehyde, and 1.04 g (8.5 mmol) of N-N-dimethylaminopyridine were added to 250 ml of chloroform in a three-necked reactor equipped with a thermometer under a nitrogen stream. The solution was placed in an ice bath and cooled to 0°C, and then, 25.7 g (203.3 mmol) of N-N'-diisopropylcarbodiimide was added to the solution. Then, the solution was stirred at 25°C for 20 hours to perform the reaction. After completion of the reaction, the resulting precipitate was filtered. The obtained filtered matter was charged into 500 ml of methanol, and was stirred and washed for 1 hour at room temperature. The filtration was performed again, and the filtered matter washed in 500 ml of methanol to obtain 19.6 g of Intermediate D as a white solid. The yield was 64.6 mol%. The structure of Intermediate D was identified by $^1$H-NMR. The $^1$H-NMR spectral data is presented below.

$^1$H-NMR (500 MHz, $CDCl_3$, TMS, δppm): 10.93 (s, 2H), 9.85 (s, 2H), 7.34 (d, 2H, J = 2.5 Hz), 7.27 (dd, 2H, J = 2.5 Hz, 9.0 Hz), 7.01 (d, 2H, J = 9.0 Hz), 3.01 (s, 4H).

<Step 2: Synthesis of Intermediate E (Another example of the compound represented by the formula (XII-1))>

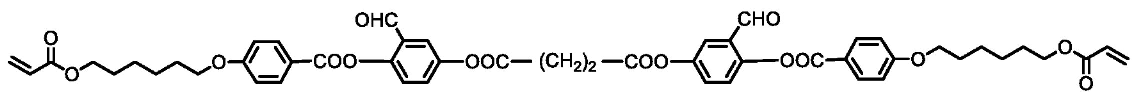

...Intermediate E

5.0 g (14.0 mmol) of intermediate D synthesized in Step 1, 10.2 g (34.9 mmol) of 4-(6-acryloyl-hex-1-yloxy)benzoic acid (manufactured by DKSH), and 17.1 mg (0.14 mmol) of N-N-dimethylaminopyridine were added to 200 ml of N-methylpyrrolidone in a three-necked reactor equipped with a thermometer under a nitrogen stream. 8.0 g (41.9 mmol) of 1-ethyl-3-(3-dimethylaminopropyl)carbodiimide hydrochloride was added thereto while gently stirring at 25°C. Then, the solution was stirred at 25°C for 12 hours to perform the reaction. After completion of the reaction, 1 liter of distilled water and 100 ml of saturated saline solution were added to the obtained reaction solution, followed by extraction twice with 250 ml of chloroform. The obtained organic layer was dried with anhydrous sodium sulfate, and the sodium sulfate was filtered off. After 250 ml of the solvent was distilled off with a rotary evaporator, the obtained residue was purified by silica gel column chromatography (chloroform:tetrahydrofuran = 90:10 (volume ratio)) to obtain 8.8 g of Intermediate E as a white solid. The yield was 69.3 mol%. The structure of Intermediate E was identified by $^1$H-NMR. The $^1$H-NMR spectral data is presented below.

$^1$H-NMR (400 MHz, $CDCl_3$ , TMS, δppm): 10.19 (s, 2H), 8.16 (d, 4H, J = 8.8 Hz), 7.70 (d, 2H, J = 2.8 Hz), 7.44 (dd, 2H, J = 2.8 Hz, 8.8 Hz), 7.36 (d, 2H, J = 8.8 Hz), 6.99 (d, 4H, J = 8.8 Hz), 6.41 (dd, 2H, J = 1.6 Hz, 17.6 Hz), 6.13 (dd, 2H, J = 10.4 Hz, 17.6 Hz), 5.83 (dd, 2H, J = 1.6 Hz, 10.4 Hz), 4.19 (t, 4H, J = 6.4 Hz), 4.07 (t, 4H, J = 6.4 Hz), 3.05 (s, 4H), 1.89-1.82 (m, 4H), 1.77-1.70 (m, 4H), 1.59-1.43 (m, 4H), 1.32-1.25 (m, 4H).

<Step 3: Synthesis of Compound 2 (Another example of the compound represented by the formula (VI-1))>

3.0 g (3.31 mmol) of Intermediate E synthesized in Step 2, 2.06 g (8.28 mmol) of Intermediate C synthesized in the same manner as Step 3 of Synthesis example 1, 76 mg (0.33 mmol) of (±)-10-camphorsulfonic acid were added to a mixed solution of 100 ml of tetrahydrofuran and 10 ml of ethanol in a four-necked reactor equipped with a thermometer under a nitrogen stream. The solution was stirred at 50°C for 2 hours. After completion of the reaction, the reaction solution was cooled, and charged into 500 ml of a 10 mass% sodium bicarbonate water, followed by extraction twice with 300 ml of ethyl acetate. The organic layer was collected, and dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 2.99 g of Compound 2 as a light yellow solid. The yield was 66 mol%. The structure of the target product (Compound 2) was identified by $^1$H-NMR. The $^1$H-NMR spectral data is presented below.

$^1$H-NMR (500 MHz, $CDCl_3$ , TMS, δppm): 8.11 (d, 4H, J = 9.0 Hz), 7.89 (d, 2H, J = 2.5 Hz), 7.61 (d, 2H, J = 8.0 Hz), 7.45 (dd, 2H, J = 0.5 Hz, 8.0 Hz), 7.40 (s, 2H), 7.29-7.24 (m, 4H), 7.18 (dd, 2H, J = 2.5 Hz, 8.0 Hz), 7.08 (ddd, 2H, J = 1.0 Hz, 8.0 Hz, 8.0 Hz), 6.99 (d, 4H, J = 9.0 Hz), 6.42 (dd, 2H, J = 1.5 Hz, 17.5 Hz), 6.14 (dd, 2H, J = 10.5 Hz, 17.5 Hz), 5.84 (dd, 2H, J = 1.5 Hz, 10.5 Hz), 4.20 (t, 4H, J = 6.5 Hz), 4.06 (t, 4H, J = 6.5 Hz), 3.83 (t, 4H, J = 7.5 Hz), 3.13 (s, 4H), 1.89-1.84 (m, 4H), 1.77-1.71 (m, 4H), 1.59-1.38 (m, 12H), 1.11-0.99 (m, 12H), 0.76 (t, 6H, J = 7.5 Hz).

(Synthesis Example 3: Synthesis of Compound 3 (Still another example of the compound represented by the formula (VI-1)))

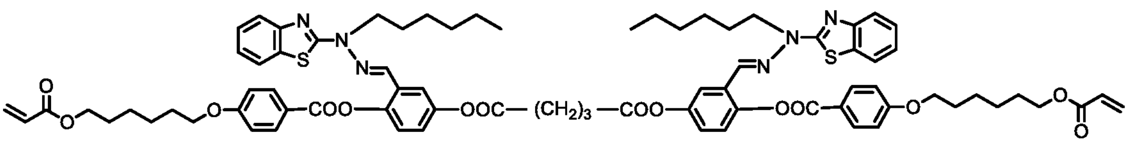

... Compound 3

<Step 1: Synthesis of Intermediate F (Another example of the compound represented by the formula (X-1))>

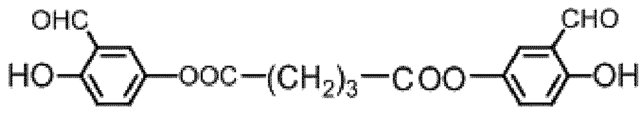

...Intermediate F

10 g (75.7 mmol) of glutaric acid, 20.9 g (151.4 mmol) of 2,5-dihydroxybenzaldehyde, and 928 mg (7.6 mmol) of N-N-dimethylaminopyridine were added to 250 ml of chloroform in a three-necked reactor equipped with a thermometer under a nitrogen stream. The solution was placed in an ice bath and cooled to 0°C, and then, 22.9 g (181.7 mmol) of N-N'-diisopropylcarbodiimide was added thereto. Then, the solution was stirred at 25°C for 20 hours. After completion of the reaction, the concentration of the reaction solution was adjusted by evaporating the solvent by the rotary evaporator. 500 ml of methanol was added to the reaction solution to precipitate a solid, and the produced solid was filtered off. The obtained filtered matter was charged in 500 ml of methanol, and was stirred and washed for 1 hour at room temperature. Filtration was performed again, and the filtered matter washed in 500 ml of methanol to obtain 18.3 g of Intermediate F as a white solid. The yield was 64.9 mol%. The structure of Intermediate F was identified by $^1$H-NMR. The $^1$H-NMR spectral data is presented below.

$^1$H-NMR (500 MHz, $CDCl_3$ , TMS, δppm): 10.93 (brs, 2H), 9.85 (s, 1H), 9.85 (s, 1H), 7.34 (d, 2H, J = 3.0 Hz), 7.28-7.25 (m, 2H), 7.01 (d, 2H, J = 9.0 Hz), 2.75 (t, 4H, J = 7.5 Hz), 2.21 (quin, 2H, J = 7.0 Hz).

<Step 2: Synthesis of Intermediate G (Still another example of the compound represented by the formula (XII-1))>

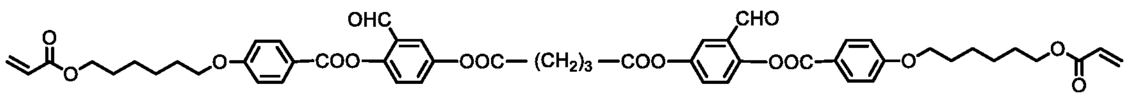

...Intermediate G

5.0 g (13.4 mmol) of Intermediate F synthesized in Step 1, 9.8 g (33.57 mmol) of 4-(6-acryloyl-hex-1-yloxy)benzoic acid (manufactured by DKSH), and 16.4 mg (0.13 mmol) of N-N-dimethylaminopyridine were added to 200 ml of N-methylpyrrolidone in a three-necked reactor equipped with a thermometer under a nitrogen stream. 7.7 g (40.28 mmol) of 1-ethyl-3-(3-dimethylaminopropyl)carbodiimide hydrochloride was added thereto while gently stirring at 25 °C. Then, the solution was stirred at 25°C for 12 hours to perform the reaction. After completion of the reaction, 1 liter of distilled water and 100 ml of saturated saline solution were added to the obtained reaction solution, followed by extraction twice with 250 ml of chloroform. The obtained organic layer was dried with anhydrous sodium sulfate, and the sodium sulfate was filtered off. After 250 ml of the solvent was distilled off with a rotary evaporator, the obtained residue was purified by silica gel column chromatography (chloroform:tetrahydrofuran = 90:10 (volume ratio)) to obtain 7.7 g of Intermediate G as a white solid. The yield was 62.5 mol%. The structure of the Intermediate G was identified by $^1$H-NMR. The $^1$H-NMR spectral data is presented below.

$^1$H-NMR (500 MHz, $CDCl_3$ , TMS, δppm): 10.19 (s, 2H), 8.16 (d, 4H, J = 9.0 Hz), 7.69 (d, 2H, J = 2.5 Hz), 7.43 (dd, 2H, J = 2.5 Hz, 9.0 Hz), 7.36 (d, 2H, J = 9.0 Hz), 6.99 (d, 4H, J = 9.0 Hz), 6.41 (dd, 2H, J = 1.5 Hz, 17.5 Hz), 6.13 (dd, 2H, J = 10.5 Hz, 17.5 Hz), 5.83 (dd, 2H, J = 1.5 Hz, 17.5 Hz), 4.19 (t, 4H, J = 6.5 Hz), 4.07 (t, 4H, J = 6.5 Hz), 2.79 (t, 4H, J = 7.5 Hz), 2.23 (quin, 2H, J = 7.5 Hz), 1.88-1.83 (m, 4H), 1.76-1.71 (m, 4H), 1.59-1.45 (m, 8H).

<Step 3: Synthesis of Compound 3 (Still another example of the compound represented by the formula (VI-1))>

3.0 g (3.26 mmol) of Intermediate G synthesized om Step 2, 1.06 g (4.24 mmol) of Intermediate C synthesized in the same manner as Step 3 of Synthesis Example 1, and 77 mg (0.33 mmol) of (±)-10-camphorsulfonic acid were added to a mixed solution of 100 ml of tetrahydrofuran and 10 ml of ethanol in a four-necked reactor equipped with a thermometer, under a nitrogen stream. The solution was stirred at 50°C for 2 hours. After completion of the reaction, the reaction solution was cooled, and charged into 500 ml of a 10 mass% sodium bicarbonate water, followed by extraction twice with 300 ml of ethyl acetate. The organic layer was collected, and dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 2.9 g of Compound 3 as a light yellow solid. The yield was 64.8 mol%. The structure of the target product (Compound 3) was identified by $^1$H-NMR. The $^1$H-NMR spectral data is presented below.

$^1$H-NMR (500 MHz, $CDCl_3$ , TMS, δppm): 8.17 (d, 4H, J = 9.0 Hz), 7.81 (d, 2H, J = 3.0 Hz), 7.63-7.61 (m, 4H), 7.58 (dd, 2H, J = 0.5 Hz, 8.0 Hz), 7.30-7.23 (m, 4H), 7.19 (dd, 2H, J = 3.0 Hz, 9.0 Hz), 7.09 (ddd, 2H, J = 1.0 Hz, 8.0 Hz, 8.0 Hz), 7.00 (d, 4H, J = 9.0 Hz), 6.42 (dd, 2H, J = 1.5 Hz, 17.5 Hz), 6.14 (dd, 2H, J = 10.5 Hz, 17.5 Hz), 5.83 (dd, 2H, J = 1.5 Hz, 10.5 Hz), 4.19 (t, 4H, J = 6.5 Hz), 4.09-4.05 (m, 8H), 2.86 (t, 4H, J = 7.5 Hz), 2.32 (quin, 2H, J = 7.5 Hz), 1.89-1.83 (m, 4H), 1.77-1.71 (m, 4H), 1.58-1.45 (m, 12H), 1.17-1.04 (m, 12H), 0.77 (t, 6H, J = 7.0 Hz).

(Synthesis Example 4: Synthesis of Compound 4 (Still another example of the compound represented by the formula (VI-1)))

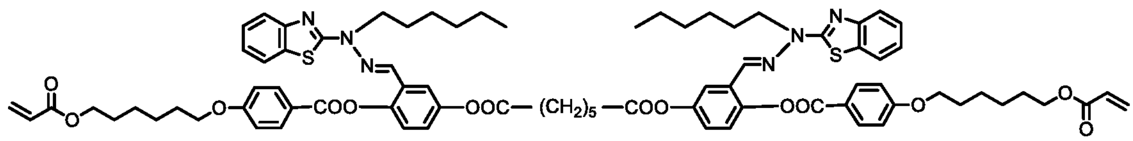

... Compound 4

<Step 1: Synthesis of Intermediate H (Still another example of the compound represented by the formula (X-1))>

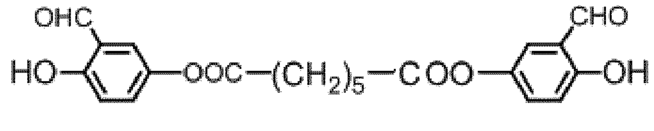

...Intermediate H

10 g (62.4 mmol) of pimelic acid, 17.2 g (124.9 mmol) of 2,5-dihydroxybenzaldehyde, and 757 mg (6.2 mmol) of N-N-dimethylaminopyridine were added to 250 ml of chloroform in a three-necked reactor equipped with a thermometer under a nitrogen stream. The solution was placed in an ice bath and cooled to 0°C, and then, 18.9 g (149.9 mmol) of N-N'-diisopropylcarbodiimide was added thereto. Then, the solution was stirred at 25°C for 20 hours. After completion of the reaction, 500 ml of distilled water and 100 ml of saturated saline solution were added to the obtained reaction solution, followed by extraction twice with 300 ml of chloroform. The organic layer was collected, and dried with anhydrous sodium sulfate, and the sodium sulfate was filtered off. The concentration of the obtained organic layer was adjusted by evaporating the solvent using the rotary evaporator. 500 ml of methanol was added to the solution to precipitate a solid, and the produced solid was filtered off. The obtained filtered matter was charged into 500 ml of methanol, and was stirred and washed for 1 hour at room temperature. The filtration was performed again, and the filtered matter washed in 500 ml of methanol to obtain 16.7 g of Intermediate H as a white solid. The yield was 66.7 mol%. The structure of Intermediate H was identified by $^1$H-NMR. The $^1$H-NMR spectral data is presented below.

$^1$H-NMR (500 MHz, $CDCl_3$, TMS, δppm): 10.91 (s, 2H), 9.83 (s, 2H), 7.32 (d, 2H, J = 3.0 Hz), 7.24 (dd, 2H, J = 3.0 Hz, 9.0 Hz), 7.00 (d, 2H, J = 9.0 Hz), 2.62 (t, 4H, J = 7.5 Hz), 1.86-1.80 (m, 4H), 1.59-1.53 (m, 2H).

<Step 2: Synthesis of Intermediate I (Still another example of the compound represented by the formula (XII-1))>

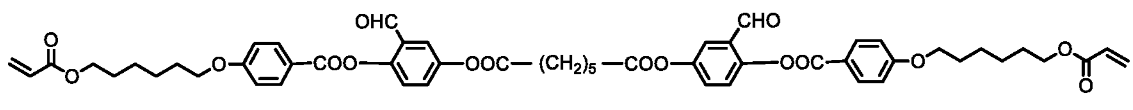

...Intermediate I

5.0 g (12.5 mmol) of Intermediate H synthesized in Step 1, 9.1 g (31.22 mmol) of 4-(6-acryloyl-hex-1-yloxy)benzoic acid (manufactured by DKSH), and 16.4 mg (0.13 mmol)of N-N-dimethylaminopyridine were added to 200 ml of N-methylpyrrolidone in a three-necked reactor equipped with a thermometer under a nitrogen stream. 7.2 g (37.46 mmol) of 1-ethyl-3-(3-dimethylaminopropyl)carbodiimide hydrochloride was added thereto while gently stirring at 25°C. Then, the solution was stirred at 25°C for 12 hours to perform the reaction. After completion of the reaction, 1 liter of distilled water and 100 ml of saturated saline solution were added to the obtained reaction solution, followed by extraction twice with 250 ml of chloroform. The obtained organic layer was dried with anhydrous sodium sulfate, and the sodium sulfate was filtered off. After 250 ml of the solvent was distilled off with a rotary evaporator, the obtained organic layer was gradually dropped into 2 liters of methanol. The precipitated solid was collected by filtration. The obtained solid was

vacuum dried to obtain 8.3 g of Intermediate I as a white solid. The yield was 70.2 mol%. The structure of Intermediate I was identified by $^1$H-NMR. The $^1$H-NMR spectral data is presented below.

$^1$H-NMR (500 MHz, $CDCl_3$ , TMS, δppm): 10.19 (s, 2H), 8.15 (d, 4H, J = 9.0 Hz), 7.67 (d, 2H, J = 3.0 Hz), 7.41 (dd, 2H, J = 3.0 Hz, 9.0 Hz), 7.35 (d, 2H, J = 9.0 Hz), 6.99 (d, 4H, J = 9.0 Hz), 6.41 (dd, 2H, J = 1.5 Hz, 17.5 Hz), 6.13 (dd, 2H, J = 10.5 Hz, 17.5 Hz), 5.83 (dd, 2H, J = 1.5 Hz, 10.5 Hz), 4.19 (t, 4H, J = 6.5 Hz), 4.07 (t, 4H, J = 6.5 Hz), 2.65 (t, 4H, J = 7.5 Hz), 1.88-1.82 (m, 8H), 1.76-1.71 (m, 4H), 1.61-1.45 (m, 10H).

<Step 3: Synthesis of Compound 4 (Still another example of the compound represented by the formula (VI-1))>

3.0 g (3.16 mmol) of Intermediate I synthesized in Step 2, 1.02 g (4.11 mmol) of Intermediate C synthesized in the same manner as Step 3 of Synthesis Example 1, and 74 mg (0.32 mmol) of (±)-10-camphorsulfonic acid were added to a mixed solution of 100 ml of tetrahydrofuran and 10 ml of ethanol in a four-necked reactor equipped with a thermometer, under a nitrogen stream. The solution was stirred at 50°C for 2 hours. After completion of the reaction, the reaction solution was cooled, and charged into 500 ml of a 10 mass% sodium bicarbonate water, followed by extraction twice with 300 ml of ethyl acetate. The organic layer was collected, and dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 2.65 g of Compound 4 as a light yellow solid. The yield was 59.4 mol%. The structure of the target product (Compound 4) was identified by $^1$H-NMR. The $^1$H-NMR spectral data is presented below.

$^1$H-NMR (500 MHz, $CDCl_3$ , TMS, δppm): 8.16 (d, 4H, J = 9.0 Hz), 7.77 (d, 2H, J = 3.0 Hz), 7.66 (s, 2H), 7.63-7.61 (m, 4H), 7.31-7.28 (m, 2H), 7.22 (d, 2H, J = 8.5 Hz), 7.14 (dd, 2H, J = 3.0 Hz, 9.0 Hz), 7.12-7.09 (m, 2H), 6.99 (d, 4H, J = 9.0 Hz), 6.42 (dd, 2H, J = 1.5 Hz, 17.5 Hz), 6.13 (dd, 2H, J = 10.5 Hz, 17.5 Hz), 5.83 (dd, 2H, J = 1.5 Hz, 10.5 Hz), 4.19 (t, 4H, J = 6.5 Hz), 4.13 (t, 4H, J = 7.5 Hz), 4.06 (t, 4H, J = 6.5 Hz), 2.72 (t, 4H, J = 7.5 Hz), 1.96-1.83 (m, 8H), 1.77-1.63 (m, 6H), 1.57-1.45 (m, 12H), 1.19-1.07 (m, 12H), 0.78 (t, 6H, J = 6.5 Hz).

(Synthesis Example 5: Synthesis of Compound 5 (Still another example of the compound represented by the formula (VI-1)))

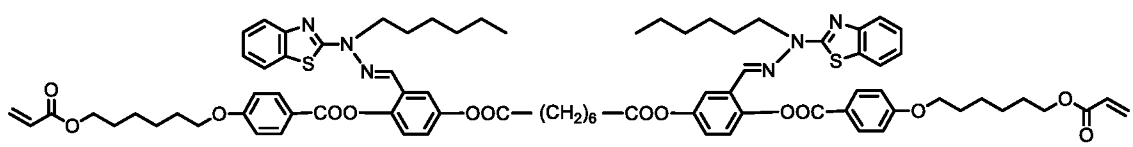

...Compound 5

<Step 1: Synthesis of Intermediate J (Still another example of the compound represented by the formula (X-1))>

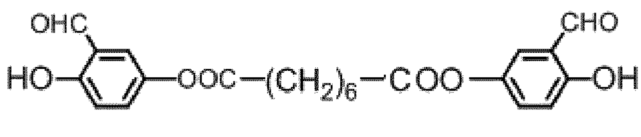

...Intermediate J

10 g (57.4 mmol) of 1,6-hexanedicarboxylic acid, 15.9 g (114.8 mmol) of 2,5-dihydroxybenzaldehyde, and 696 mg (5.7 mmol) of N-N-dimethylaminopyridine were added to 250 ml of chloroform in a three-necked reactor equipped with a thermometer under a nitrogen stream. The solution was placed in an ice bath and cooled to 0°C, and then, 17.4 g (137.8 mmol) of N-N'-diisopropylcarbodiimide was added thereto. Then, the solution was stirred at 25°C for 20 hours. after completion of the reaction, 500 ml of distilled water and 100 ml of saturated saline solution were added to the obtained reaction solution, followed by extraction twice with 300 ml of chloroform. The organic layer was collected, and dried with anhydrous sodium sulfate, and the sodium sulfate was filtered off. The concentration of the obtained organic layer was adjusted by evaporating the solvent using a rotary evaporator. 500 ml of methanol was added to the solution

to precipitate a solid, and the produced solid was filtered off. The obtained filtered matter was charged into 500 ml of methanol, and was stirred and washed for 1 hour at room temperature. The filtration was performed again, and the filtered matter washed in 500 ml of methanol to obtain 13.8 g of Intermediate J as a gray solid. The yield was 58.2 mol%. The structure of Intermediate J was identified by $^1$H-NMR. The $^1$H-NMR spectral data is presented below.

$^1$H-NMR (500 MHz, $CDCl_3$, TMS, δppm): 10.91 (s, 2H), 9.85 (s, 1H), 9.85 (s, 1H), 7.32 (d, 2H, J = 2.5 Hz), 7.24 (dd, 2H, J = 2.5 Hz, 9.0 Hz), 7.00 (d, 2H, J = 9.0 Hz), 2.59 (t, 4H, J = 7.5 Hz), 1.81-1.78 (m, 4H), 1.51-1.48 (m, 4H).

<Step 2: Synthesis of Intermediate K (Still another example of the compound represented by the formula (XII-1))>

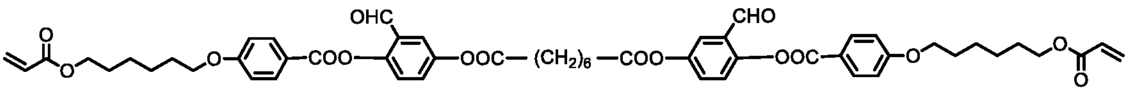

...Intermediate K

5.0 g (12.1 mmol) of Intermediate J synthesized in Step 1, 8.82 g (30.2 mmol) of 4-(6-acryloyl-hex-1-yloxy)benzoic acid (manufactured by DKSH), and 14.7 mg (0.12 mmol) of N-N-dimethylaminopyridine were added to 200 ml of N-methylpyrrolidone in a three-necked reactor equipped with a thermometer under a nitrogen stream. 6.95 g (36.2 mmol) of 1-ethyl-3-(3-dimethylaminopropyl)carbodiimide hydrochloride was added thereto while gently stirring at 25 °C. Then, the solution was stirred at 25°C for 12 hours to perform the reaction. After completion of the reaction, 1 liter of distilled water and 100 ml of saturated saline solution were added to the obtained reaction solution, followed by extraction twice with 250 ml of chloroform. The obtained organic layer was dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 8.0 g of Intermediate K as a white solid. The yield was 68.8 mol%. The structure of Intermediate K was identified by $^1$H-NMR. The $^1$H-NMR spectral data is presented below.

$^1$H-NMR (400 MHz, $CDCl_3$ , TMS, δppm): 10.19 (s, 2H), 8.16 (d, 4H, J = 8.8 Hz), 7.67 (d, 2H, J = 2.8 Hz), 7.41 (dd, 2H, J = 2.8 Hz, 8.8 Hz), 7.35 (dd, 2H, J = 8.8 Hz), 6.99 (d, 4H, J = 8.8 Hz), 6.41 (dd, 2H, J = 1.6 Hz, 17.6 Hz), 6.13 (dd, 2H, J = 10.4 Hz, 17.6 Hz), 5.83 (dd, 2H, J = 1.6 Hz, 10.4 Hz), 4.19 (t, 4H, J = 6.4 Hz), 4.07 (t, 4H, J = 6.4 Hz), 2.62 (t, 4H, J = 7.2 Hz), 1.89-1.70 (m, 12H), 1.57-1.44 (m, 12H).

<Step 3: Synthesis of Compound 5 (Still another example of the compound represented by the formula (VI-1))>

3.0 g (3.12 mmol) of Intermediate K synthesized in Step 2, 1.01 g (4.05 mmol) of Intermediate C synthesized in the same manner as Step 3 of Synthesis Example 1, and 72 mg (0.31 mmol) of (±)-10-camphorsulfonic acid were added to a mixed solution of 100 ml of tetrahydrofuran and 10 ml of ethanol in a four-necked reactor equipped with a thermometer, under a nitrogen stream. The solution was stirred at 50°C for 2 hours. After completion of the reaction, the reaction solution was cooled, and charged into 500 ml of a 10 mass% sodium bicarbonate water, followed by extraction twice with 300 ml of ethyl acetate. The organic layer was collected, and dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 2.7 g of Compound 5 as a light yellow solid. The yield was 60.8 mol%. The structure of the target product (Compound 5) was identified by $^1$H-NMR. The $^1$H-NMR spectral data is presented below.

$^1$H-NMR (500 MHz, $CDCl_3$ , TMS, δppm): 8.16 (d, 4H, J = 9.0 Hz), 7.77 (d, 2H, J = 3.0 Hz), 7.68 (s, 2H), 7.64-7.62 (m, 4H), 7.32-7.28 (m, 2H), 7.23 (d, 2H, J = 9.0 Hz), 7.15-7.10 (m, 4H), 6.99 (d, 4H, J = 9.0 Hz), 6.41 (dd, 2H, J = 1.5 Hz, 17.5 Hz), 6.13 (dd, 2H, J = 10.5 Hz, 17.5 Hz), 5.83 (dd, 2H, J = 1.5 Hz, 10.5 Hz), 4.19 (t, 4H, J = 6.5 Hz), 4.14 (t, 4H, J = 7.5 Hz), 4.06 (t, 4H, J = 6.5 Hz), 2.68 (t, 4H, J = 7.5 Hz), 1.90-1.83 (m, 8H), 1.77-1.71 (m, 4H), 1.59-1.45 (m, 16H), 1.19-1.08 (m, 12H), 0.78 (t, 6H, J = 6.5 Hz).

(Synthesis Example 6: Synthesis of Compound 6 (Still another example of the compound represented by the formula (VI-1)))

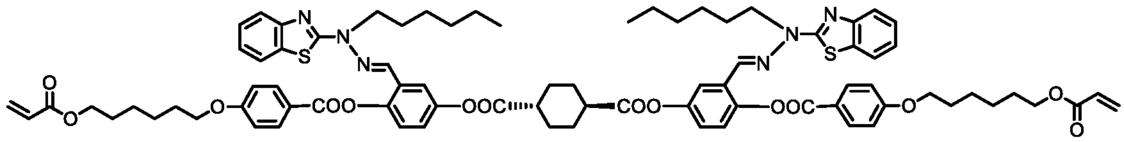

...Compound 6

<Step 1: Synthesis of Intermediate L (Still another example of the compound represented by the formula (X-1))>

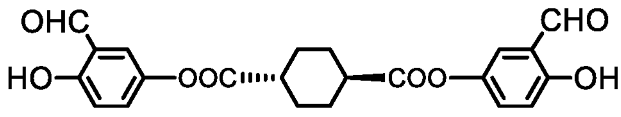

...Intermediate L

10 g (58.1 mmol) of trans-1,4-cyclohexanedicarboxylic acid, 16.0 g (116 mmol) of 2,5-dihydroxybenzaldehyde, and 710 mg (5.8 mmol) of N-N-dimethylaminopyridine were added to 350 ml of chloroform in a three-necked reactor equipped with a thermometer under a nitrogen stream. 17.6 g (139 mmol) of N-N'-diisopropylcarbodiimide was gradually dropped therein at 15°C while stirring vigorously. Then, the solution was stirred at 25°C for 6 hours to perform the reaction. After completion of the reaction, the resulting precipitate was filtered. The obtained filtered matter was washed with 500 ml of methanol. Furthermore, the obtained filtered matter was purified by silica gel column chromatography (chloroform: ethyl acetate = 90:10 (volume ratio)) to obtain 18 g of Intermediate L as a white solid. The yield was 75.1 mol%. The structure of Intermediate L was identified by $^1$H-NMR. The $^1$H-NMR spectral data is presented below.

$^1$H-NMR (500 MHz, DMSO-$d_6$, TMS, δppm): 10.78 (s, 2H), 10.26 (s, 2H), 7.34 (d, 2H, J = 3.0 Hz), 7.29 (dd, 2H, J = 3.0 Hz, 9.0 Hz), 7.03 (d, 2H, J = 9.0 Hz), 2.65-2.58 (m, 2H), 2.18-2.12 (m, 4H), 1.62-1.52 (m, 4H).

<Step 2: Synthesis of Intermediate M (Still another example of the compound represented by the formula (XII-1))>

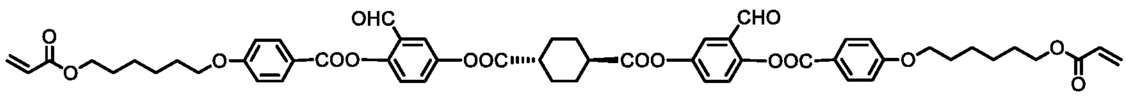

...Intermediate M

5.0 g (12.1 mmol) of Intermediate L synthesized in Step 1, 8.77 g (30 mmol) of 4-(6-acryloyl-hex-1-yloxy)benzoic acid (manufactured by DKSH), and 14.7 mg (0.12 mmol) of N-N-dimethylaminopyridine were added to 200 ml of N-methylpyrrolidone in a three-necked reactor equipped with a thermometer under a nitrogen stream. 6.9 g (36 mmol) of 1-ethyl-3-(3-dimethylaminopropyl)carbodiimide hydrochloride was added thereto while gently stirring at 25°C. Then, the solution was stirred at 25°C for 12 hours to perform the reaction. After completion of the reaction, 1 liter of distilled water and 100 ml of saturated saline solution were added to the obtained reaction solution, followed by extraction twice with 250 ml of chloroform. The obtained organic layer was dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 6.45 g of Intermediate M as a white solid. The yield was 55.5 mol%. The structure of Intermediate M was identified by $^1$H-NMR. The $^1$H-NMR spectral data is presented below.

$^1$H-NMR (500 MHz, DMSO-$d_6$, TMS, δppm): 10.07 (s, 2H), 8.12 (d, 4H, J = 9.0 Hz), 7.72 (d, 2H, J = 3.0 Hz), 7.59 (dd, 2H, J = 3.0 Hz, 9.0 Hz), 7.54 (d, 2H, J = 9.0 Hz), 7.14 (d, 4H, J = 9.0 Hz), 6.33 (dd, 2H, J = 1.5 Hz, 17.5 Hz), 6.18 (dd, 2H, J = 10.5 Hz, 17.5 Hz), 5.94 (dd, 2H, J = 1.5 Hz, 10.5 Hz), 4.12 (t, 4H, J = 7.0 Hz), 4.11 (t, 4H, J = 6.5 Hz), 2.75-2.69 (m, 2H), 2.25-2.18 (m, 4H), 1.80-1.74 (m, 4H), 1.68-1.60 (m, 8H), 1.50-1.39 (m, 8H).

<Step 3: Synthesis of Compound 6 (Still another example of the compound represented by the formula (VI-1))>

3.0 g (3.12 mmol) of Intermediate M synthesized in Step 2, 1.01 g (4.05 mmol) of Intermediate C synthesized in the same manner as Step 3 of Synthesis Example 1, and 72 mg (0.31 mmol) of (±)-10-camphorsulfonic acid were added to a mixed solution of 100 ml of tetrahydrofuran and 10 ml of ethanol in a four-necked reactor equipped with a thermometer, under a nitrogen stream. The solution was stirred at 50°C for 2 hours. After completion of the reaction, the reaction solution was cooled, and charged into 500 ml of a 10 mass% sodium bicarbonate water, followed by extraction twice with 300 ml of ethyl acetate. The organic layer was collected, and dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 2.58 g of Compound 6 as a light yellow solid. The yield was 58.1 mol%. The structure of the target product (Compound 6) was identified by $^1$H-NMR. The $^1$H-NMR spectral data is presented below.

$^1$H-NMR (500 MHz, $CDCl_3$ , TMS, δppm): 8.18 (d, 4H, J = 9.0 Hz), 7.79 (d, 2H, J = 2.5 Hz), 7.74 (s, 2H), 7.69-7.64 (m, 4H), 7.34-7.31 (m, 2H), 7.27-7.25 (m, 2H), 7.18-7.14 (m, 4H), 7.00 (d, 4H, J = 9.0 Hz), 6.42 (dd, 2H, J = 1.5 Hz, 17.5 Hz), 6.14 (dd, 2H, J = 10.5 Hz, 17.5 Hz), 5.84 (dd, 2H, J = 1.5 Hz, 10.5 Hz), 4.21-4.17 (m, 8H), 4.06 (t, 4H, J = 6.5 Hz), 2.75-2.68 (m, 2H), 2.42-2.40 (m, 4H), 1.89-1.85 (m, 4H), 1.80-1.73 (m, 8H), 1.63-1.46 (m, 12H), 1.22-1.10 (m, 12H), 0.79 (t, 6H, J = 6.5 Hz).

(Synthesis Example 7: Synthesis of Compound 7 (Still another example of a compound represented by formula (VI-1)))

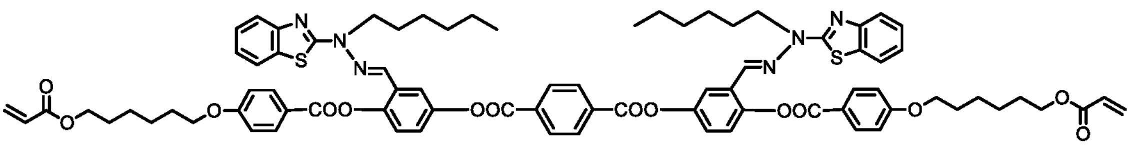

...Compound 7

<Step 1: Synthesis of Intermediate N (Still another example of a compound represented by the formula (X-1))>

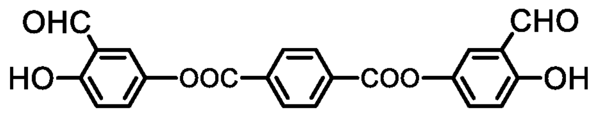

...Intermediate N

10 g (60.2 mmol) of terephthalic acid, 16.6 g (120 mmol) of 2,5-dihydroxybenzaldehyde, and 735 mg (6.0 mmol) of N-N-dimethylaminopyridine were added to 300 ml of chloroform in a three-necked reactor equipped with a thermometer under a nitrogen stream. 18.2 g (144.5 mmol) of N-N'-diisopropylcarbodiimide was gradually dropped therein at 15°C while stirring vigorously. Then, the solution was stirred at 25°C for 12 hours to perform the reaction. After completion of the reaction, the resulting precipitate was filtered. The obtained filtered matter was charged into 500 ml of methanol, and was stirred and washed for 1 hour at room temperature. The filtration was performed again, and the filtered matter washed in 500 ml of methanol to obtain 12.3 g of Intermediate N of a light yellow solid. The yield was 50.3 mol%. The structure of Intermediate N was identified by $^1$H-NMR. The $^1$H-NMR spectral data is presented below.

$^1$H-NMR (500 MHz, DMSO-$d_6$, TMS, δppm): 10.88 (s, 2H), 10.30 (s, 2H), 8.31 (s, 4H), 7.58 (d, 2H, J = 3.0 Hz), 7.52 (dd, 2H, J = 3.0 Hz, 9.0 Hz), 7.10 (d, 2H, J = 9.0 Hz).

<Step 2: Synthesis of Intermediate O (Still another example of the compound represented by the formula (XII-1))>

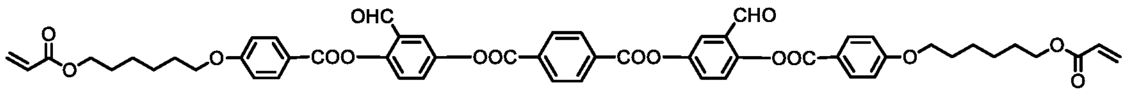

...Intermediate O

5.0 g (12.3 mmol) of Intermediate N synthesized in Step, 9.0 g (30.8 mmol) of 4-(6-acryloyl-hex-1-yloxy)benzoic acid (manufactured by DKSH), and 14.7 mg (0.12 mmol) of N-N-dimethylaminopyridine were added to 200 ml of N-methylpyrrolidone in a three-necked reactor equipped with a thermometer under a nitrogen stream. 7.09 g (37 mmol) of 1-ethyl-3-(3-dimethylaminopropyl)carbodiimide hydrochloride was added thereto while gently stirring at 25 °C. Then, the solution was stirred at 25°C for 12 hours to perform the reaction. After completion of the reaction, 1 liter of distilled water and 100 ml of saturated saline solution were added to the obtained reaction solution, followed by extraction twice with 250 ml of chloroform. The obtained organic layer was dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 7.07 g of Intermediate O as a white solid. The yield was 60.2 mol%. The structure of Intermediate O was identified by $^1$H-NMR. The $^1$H-NMR spectral data is presented below.

$^1$H-NMR (500 MHz, $CDCl_3$, TMS, δppm): 10.24 (s, 2H), 8.37 (s, 4H), 8.19 (d, 4H, J = 9.0 Hz), 7.86 (d, 2H, J = 3.0 Hz), 7.59 (dd, 2H, J = 3.0 Hz, 9.0 Hz), 7.45 (d, 2H, J = 9.0 Hz), 7.01 (d, 4H, J = 9.0 Hz), 6.41 (dd, 2H, J = 1.5 Hz, 17.0 Hz), 6.13 (dd, 2H, J = 10.5 Hz, 17.0 Hz), 5.83 (dd, 2H, J = 1.5 Hz, 10.5 Hz), 4.19 (t, 4H, J = 6.5 Hz), 4.08 (t, 4H, J = 6.5 Hz), 1.89-1.84 (m, 4H), 1.77-1.71 (m, 4H), 1.58-1.45 (m, 8H).

<Step 3: Synthesis of Compound 7 (Still another example of the compound represented by the formula (VI-1))>

3.0 g (3.14 mmol) of Intermediate O synthesized in Step 2, 1.02 g (4.08 mmol) of intermediate C synthesized in the same manner as Step 3 of Synthesis Example 1, and 72 mg (0.31 mmol) of (±)-10-camphorsulfonic acid was added to a mixed solution of 100 ml of tetrahydrofuran and 10 ml of ethanol in a four-necked reactor equipped with a thermometer, under a nitrogen stream. The solution was stirred at 50°C for 2 hours. After completion of the reaction, the reaction solution was cooled, and charged into 500 ml of a 10 mass% sodium bicarbonate water, followed by extraction twice with 300 ml of ethyl acetate. The organic layer was collected, and dried with anhydrous sodium sulfate, and the sodium sulfate was filtered off. After the solvent was removed using a rotary evaporator, the obtained residue was purified by silica gel column chromatography (chloroform:tetrahydrofuran = 90:10 (volume ratio)) to obtain 2.32 g of Compound 7 as a light yellow solid. The yield was 52.1 mol%. The structure of the target product (Compound 7) was identified by $^1$H-NMR. The $^1$H-NMR spectral data is presented below.

$^1$H-NMR (500 MHz, $CDCl_3$, TMS, δppm): 8.44 (s, 4H), 8.20 (d, 4H, J = 9.0 Hz), 7.97 (dd, 2H, J = 1.5 Hz, 1.5 Hz), 7.78 (s, 2H), 7.65-7.63 (m, 4H), 7.34 (d, 4H, J = 1.5 Hz), 7.33-7.30 (m, 2H), 7.13 (ddd, 2H, J = 1.0 Hz, 7.5 Hz, 7.5 Hz), 7.02 (d, 4H, J = 9.0 Hz), 6.42 (dd, 2H, J = 1.5 Hz, 17.5 Hz), 6.14 (dd, 2H, J = 10.5 Hz, 17.5 Hz), 5.84 (dd, 2H, J = 1.5 Hz, 10.5 Hz), 4.20 (t, 8H, J = 6.5 Hz), 4.07 (t, 4H, J = 6.5 Hz), 1.90-1.84 (m, 4H), 1.77-1.72 (m, 4H), 1.64-1.46 (m, 12H), 1.23-1.10 (m, 12H), 0.80 (t, 6H, J = 6.5 Hz).

(Comparative Synthesis Example 1: Synthesis of Compound X (Example of the polymerizable compound represented by the formula (I-2)))

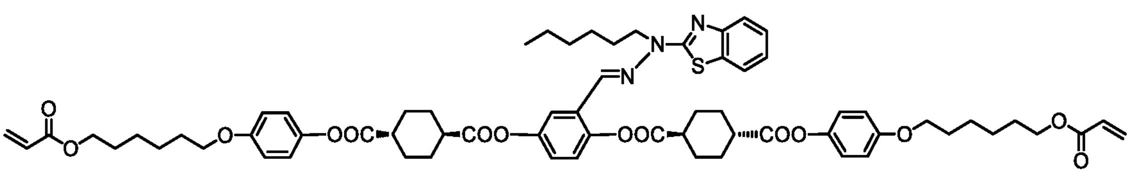

...Compound X

<Step 1: Synthesis of Intermediate P>

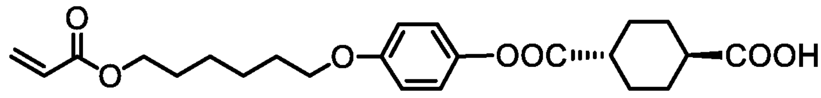

...Intermediate P

17.98 g (104.42 mmol) of trans-1,4-cyclohexanedicarboxylic acid and 180 ml of tetrahydrofuran (THF) were charged into a three-necked reactor equipped with a thermometer, under a nitrogen stream. After 6.58 g (57.43 mmol) of methanesulfonyl chloride was added therein, the reactor was immersed in a water bath to adjust the temperature of the reaction solution to 20°C. Next, 6.34 g (62.65 mmol) of triethylamine was added dropwise to the reaction solution over 10 minutes while maintaining the temperature of the reaction solution at 20 to 30°C. After the dropwise addition, the solution was stirred at 25 °C for 2 hours.

After 0.64 g (5.22 mmol) of 4-(dimethylamino)pyridine and 13.80 g (52.21 mmol) of 4-(6-acryloyloxy-hex-1-yloxy)phenol (manufactured by DKSH) were added to the obtained reaction solution, the reactor was again immersed in a water bath to adjust the temperature of the reaction solution to 15°C. 6.34 g (62.65 mmol) of triethylamine was added dropwise to the reaction solution over 10 minutes while maintaining the temperature of the reaction solution at 20 to 30°C. After the dropwise addition, the solution was stirred at 25°C for 2 hours. After completion of the reaction, 1,000 ml of distilled water and 100 ml of a saturated sodium chloride solution were added to the reaction solution, followed by extraction twice with 400 ml of ethyl acetate. The organic layer was collected, and dried with anhydrous sodium sulfate, and the sodium sulfate was filtered off. After the solvent was evaporated from the filtrate using a rotary evaporator, the obtained residue was purified by silica gel column chromatography (THF: toluene = 1:9 (volume ratio)) to obtain 14.11 g of Intermediate P as a white solid. The yield was 65 mol%. The structure of Intermediate P was identified by $^1$H-NMR. The $^1$H-NMR spectral data is presented below.

$^1$H-NMR (500 MHz, DMSO-$d_6$, TMS, δppm): 12.12 (s, 1H), 6.99 (d, 2H, J = 9.0 Hz), 6.92 (d, 2H, J = 9.0 Hz), 6.32 (dd, 1H, J = 1.5 Hz, 17.5 Hz), 6.17 (dd, 1H, J = 10.0 Hz, 17.5 Hz), 5.93 (dd, 1H, J = 1.5 Hz, 10.0 Hz), 4.11 (t, 2H, J = 6.5 Hz), 3.94 (t, 2H, J = 6.5 Hz), 2.48-2.56 (m, 1H), 2.18-2.26 (m, 1H), 2.04-2.10 (m, 2H), 1.93-2.00 (m, 2H), 1.59-1.75 (m, 4H), 1.35-1.52 (m, 8H).

<Step 2: Synthesis of Intermediate X1>

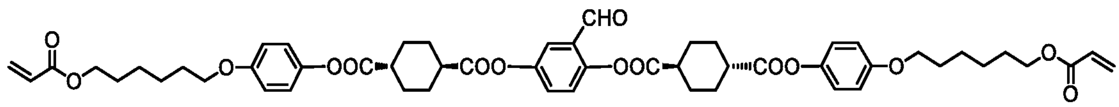

...Intermediate X1

4.00 g (9.56 mmol) of Intermediate P synthesized in Step 1 and 60 ml of THF were charged into a three-necked reactor equipped with a thermometer, under a nitrogen stream to prepare a uniform solution. After 1.12 g (9.78 mmol) of methanesulfonyl chloride was added thereto, the reactor was immersed in a water bath to adjust the temperature of the reaction solution to 20°C. Next, 1.01 g (9.99 mmol) of triethylamine was added dropwise to the reaction solution over 5 minutes while maintaining the temperature of the reaction solution at 20 to 30°C. After the dropwise addition, the solution was stirred at 25°C for an additional 2 hours. After 0.11 g (0.87 mmol) of 4-(dimethylamino)pyridine, and, 0.60 g (4.35 mmol) of 2,5-dihydroxybenzaldehyde was added to the obtained reaction solution, the reactor was again immersed in a water bath to adjust the temperature of the reaction solution to 15°C. 1.10 g (10.87 mmol) of triethylamine was added dropwise to the reaction solution over 5 minutes while maintaining the temperature of the reaction solution at 20 to 30°C, After the dropwise addition, the solution was stirred at 25°C for an additional 2 hours. After completion of the reaction, 400 ml of distilled water and 50 ml of saturated saline solution were added to the reaction solution, followed by extraction twice with 750 ml of ethyl acetate. The organic layer was collected, and dried with anhydrous sodium sulfate, and the sodium sulfate was filtered off. After the solvent was evaporated from the filtrate using a rotary evaporator, the obtained residue was dissolved in 100 ml of THF. 500 ml of methanol was added to the solution to precipitate crystals, which were filtered off. The obtained crystals were washed with methanol, and dried under vacuum to obtain 2.51 g of Intermediate XI as a white solid. The yield was 62 mol%. The structure of Intermediate XI was identified by $^1$H-NMR. The $^1$H-NMR spectral data is presented below.

$^1$H-NMR (500 MHz, DMSO-$d_6$, TMS, δppm): 10.02 (s, 1H), 7.67 (d, 1H, J = 3.0 Hz), 7.55 (dd, 1H, J = 3.0 Hz, 8.5 Hz), 7.38 (d, 1H, J = 8.5 Hz), 6.99-7.04 (m, 4H), 6.91-6.96 (m, 4H), 6.32 (dd, 2H, J = 1.5 Hz, 17.5 Hz), 6.17 (dd, 2H, J = 10.0 Hz, 17.5 Hz), 5.93 (dd, 2H, J = 1.5 Hz, 10.0 Hz), 4.11 (t, 4H, J = 6.5 Hz), 3.95 (t, 4H, J = 6.5 Hz), 2.56-2.81 (m,

4H), 2.10-2.26 (m, 8H), 1.50-1.76 (m, 16H), 1.33-1.49 (m, 8H).

<Step 3: Synthesis of Compound X (Example of the polymerizable compound represented by the formula (I-2))>

697 mg (2.37 mmol) of Intermediate C synthesized in the same manner as Step 3 of Synthesis Example 1, and 2.00 g (2.13 mmol) of Intermediate XI synthesized in Step 2 were dissolved in 35 ml of chloroform in a four-necked reactor equipped with a thermometer, under a nitrogen stream. 49 mg (0.21 mmol) of (±)-10-camphorsulfonic acid was added to the solution, and the solution was stirred at 50°C for 3 hours. After completion of the reaction, the reaction solution was charged into a mixed water of 100 ml of water and 50 ml of 5% aqueous solution of sodium hydrogen carbonate, followed by extraction with 250 ml of ethyl acetate. The ethyl acetate layer was dried with anhydrous sodium sulfate. After the sodium sulfate was filtered off, the ethyl acetate was evaporated under reduced pressure using a rotary evaporator to obtain a white solid. The white solid was purified by silica gel column chromatography (toluene: ethyl acetate = 88:12 (volume ratio)) to obtain 2.33 g of Compound X as a white solid. The yield was 93.5 mol%. The structure of the target product (Compound X) was identified by $^1$H-NMR. The $^1$H-NMR spectral data is presented below.

$^1$H-NMR (400 MHz, $CDCl_3$, TMS, δppm): 7.75 (d, 1H, J = 2.5 Hz), 7.67-7.70 (m, 3H), 7.34 (ddd, 1H, J = 1.0 Hz, 7.0 Hz, 7.5 Hz), 7.17 (ddd, 1H, J = 1.0 Hz, 7.5 Hz, 7.5 Hz), 7.12 (d, 1H, J = 9.0 Hz), 7.10 (dd, 1H, J = 2.5 Hz, 9.0 Hz), 6.99 (d, 2H, J = 9.0 Hz), 6.98 (d, 2H, J = 9.0 Hz), 6.88 (d, 4H, J = 9.0 Hz), 6.40 (dd, 2H, J = 1.5 Hz, 17.0 Hz), 6.13 (dd, 2H, J = 10.5 Hz, 17.5 Hz), 5.82 (dd, 2H, J = 1.5 Hz, 10.5 Hz), 4.30 (t, 2H, J = 8.0 Hz), 4.18 (t, 4H, J = 6.5 Hz), 3.95 (t, 4H, J = 6.5 Hz), 2.58-2.70 (m, 4H), 2.31-2.35 (m, 8H), 1.66-1.82 (m, 18H), 1.31-1.54 (m, 14H), 0.90 (t, 3H, J = 7.0 Hz).

<Measurement of Phase Transition Temperature>

5 mg of each of Compounds 1 to 7 and Compound X was weighed, and placed in a solid state between two glass substrates provided with a polyimide alignment film subjected to a rubbing treatment (manufactured by E.H.C Co., Ltd., Product name: Alignment Treatment Glass Substrate). The substrates were placed on a hot plate, heated from 50°C to 200°C, and cooled to 50°C. A change in the structure during a change in the temperature was observed using a polarizing microscope ("ECLIPSE LV100 POL" manufactured by Nikon Corporation).

The measured phase transition temperatures are listed in the following Table 1.

In Table 1, "C" refers to "crystal", "N" refers to "nematic", and "I" refers to "isotropic". Here, the term "crystal" means that the test compound was in a solid phase, the term "nematic" means that the test compound was in a nematic liquid crystal phase, and the term "isotropic" means that the test compound was in an isotropic liquid phase.

Table 1-1

**Table 1**

| Compound Number | Phase transition temperature |
|---|---|
| Compound 1 | $\mathrm{C} \underset{50^\circ\mathrm{C}\text{ or less}}{\overset{138^\circ\mathrm{C}}{\rightleftarrows}} \mathrm{I}$ |
| Compound 2 | $\mathrm{C} \underset{100^\circ\mathrm{C}}{\overset{139^\circ\mathrm{C}}{\rightleftarrows}} \mathrm{I}$ |
| Compound 3 | $\mathrm{C} \underset{50^\circ\mathrm{C}\text{ or less}}{\overset{72^\circ\mathrm{C}}{\rightleftarrows}} \mathrm{I}$ |
| Compound 4 | $\mathrm{C} \underset{50^\circ\mathrm{C}\text{ or less}}{\overset{85^\circ\mathrm{C}}{\rightleftarrows}} \mathrm{I}$ |

(continued)

| Compound Number | Phase transition temperature |
|---|---|
| Compound 5 | $C \underset{63^{\circ}C}{\overset{96^{\circ}C}{\rightleftarrows}} I$ |
| Compound 6 | $C \underset{50^{\circ}C\ \text{or less}}{\overset{185^{\circ}C}{\rightleftarrows}} N \xrightarrow{200^{\circ}C\ \text{or more}} I$ |
| Compound 7 | $C \underset{120^{\circ}C}{\overset{192^{\circ}C}{\rightleftarrows}} N \xrightarrow{200^{\circ}C\ \text{or more}} I$ |
| Compound X | $C \underset{50^{\circ}C\ \text{or less}}{\overset{96^{\circ}C}{\rightleftarrows}} N \xrightarrow{200^{\circ}C\ \text{or more}} I$ |

<Preparation of Polymerizable Liquid Crystal Composition>

(Examples 1 to 5)

0.5 g of each of Compounds 1 to 5 obtained in Synthesis Examples 1 to 5, 0.5 g of Compound X obtained in Comparative Synthesis Example 1, 43 mg of a photopolymerization initiator ADEKA ARKLS N-1919T (manufactured by ADEKA Corporation), and 300 mg of a mixed solvent (mixing ratio (mass ratio): cyclopentanone / 1,3-dioxolane = 4/6) of cyclopentanone and 1,3-dioxolane containing 1 mass% of surfactant MEGAFACE F-562 (manufactured by DIC Corporation) were prepared separately, and dissolved in a mixed solvent of 2.05 g of 1,3-dioxolane and 1.37 g of cyclopentanone. The solutions were filtered through disposable filters having a pore size of 0.45 μm to prepare Polymerizable Compositions 1 to 5.

(Example 6)

1.0 g of Compound 6 obtained in Synthesis Example 6, 43 mg of a photopolymerization initiator ADEKA ARKLS N-1919T (manufactured by ADEKA Corporation), and 300 mg of a mixed solvent (mixing ratio (mass ratio): cyclopentanone / 1,3-dioxolane = 4/6) of cyclopentanone and 1,3-dioxolane containing 1 mass% of surfactant MEGAFACE F-562 (manufactured by DIC Corporation) were prepared separately, and dissolved in a mixed solvent of 2.05 g of 1,3-dioxolane and 1.37 g of cyclopentanone. The solution was filtered through a disposable filter having a pore size of 0.45 μm to prepare a Polymerizable composition 6.

(Example 7)

1.0 g of Compound 7 obtained in Synthesis Example 7, 43 mg of a photopolymerization initiator ADEKA ARKLS N-1919T (manufactured by ADEKA Corporation), and 300 mg of a mixed solvent (mixing ratio (mass ratio): cyclopentanone / 1,3-dioxolane = 4/6) of cyclopentanone and 1,3-dioxolane containing 1 mass% of surfactant MEGAFACE F-562 (manufactured by DIC Corporation) were prepared separately, and dissolved in a mixed solvent of 2.4 g of 1,3-dioxolane, 1.6 g of cyclopentanone, and 8 g of chloroform. The solution was filtered through a disposable filter having a pore size of 0.45 μm to prepare a Polymerizable composition 7.

(Comparative example 1)

1.0 g of Compound X obtained in Comparative Synthesis Example 1, 43 mg of a photopolymerization initiator ADEKA ARKLS N-1919T (manufactured by ADEKA Corporation), and 300 mg of a mixed solvent (mixing ratio (mass ratio): cyclopentanone / 1,3-dioxolane = 4/6) of cyclopentanone and 1,3-dioxolane containing 1 mass% of surfactant MEGAFACE F-562 (manufactured by DIC Corporation) were prepared separately, and dissolved in a mixed solvent of 2.05 g of 1,3-dioxolane and 1.37 g of cyclopentanone. The solution was filtered through a disposable filter having a pore

size of 0.45 μm to prepare a Polymerizable Composition 1r.

<Analysis by High-Performance Liquid Chromatography (HPLC) of Polymerizable Liquid Crystal Composition>

1) Preparation of analysis sample

0.1 g of each of Polymerizable Compositions 1 to 5 prepared in Examples 1 to 5 was sampled. Moreover, 10 g of tetrahydrofuran was added to each sample, and each sample was diluted. The samples were analyzed by high-performance liquid chromatography (HPLC) under the following conditions. The area ratios of each of Compounds 1 to 5 and Compound X were analyzed and are summarized in Table 1-2.

Note that the analysis conditions of the high-performance liquid chromatography (HPLC) are not specifically limited as long as there is the condition which makes it possible to separate the polymerizable compound represented by the formula (I-1) and the polymerizable compound having a different chemical structure than (I-1).

Table 1-2

| Polymerizable Composition | Polymerizable compound (Area % by HPLC analysis) | |
|---|---|---|
| 1 | Compound 1 (70.0526) | Compound X (29.9474) |
| 2 | Compound 2 (67.0737) | Compound X (32.9263) |
| 3 | Compound 3 (71.1923) | Compound X (28.8077) |
| 4 | Compound 4 (70.6034) | Compound X (29.3966) |
| 5 | Compound 5 (68.1006) | Compound X (31.8994) |

2) High-performance liquid chromatography (HPLC) analysis condition

High-performance liquid chromatography (HPLC) device: Agilent 1200 Series (manufactured by Agilent Technologies).
Column: ZORBAX Eclipse XDB-C18, 4.6 mm × 100 mm, 1.8 μm (manufactured by Agilent Technologies).
Flow rate: 1.0 ml/min.
Injection amount: 1.2 μl.
Detection: UV254 nm.
Mobile phase conditions: Listed in the following Table 1-3.

Table 1-3

| | Mobile phase composition (volume ratio) | | |
|---|---|---|---|
| Time (min) | Acetonitrile | Water | Tetrahydrofuran |
| 0 | 85 | 15 | 0 |
| 15 | 100 | 0 | 0 |
| 30 | 85 | 0 | 15 |

Polymerizable compounds 1 to 5 were verified to contain more than 50% of Compound 1 to 5 by the area value according to the analysis by high-performance liquid chromatography (HPLC).

<Evaluation of optical property>

(i) Formation of liquid crystal layer using polymerizable composition

Each of the Polymerizable Compositions 1 to 7 and 1r obtained as stated above was applied to a polyimide alignment film subjected to a rubbing treatment (manufactured by E.H.C Co., Ltd., Product name: Alignment Treatment Glass Substrate using a #6 or #8 wire bar as listed in the following Table 2-1 to obtain the coating film. The obtained coating films were dried for 1 minute at the temperatures listed in Table 2-1, and subjected to an alignment treatment for 1 minute at the temperatures listed in Table 1 to form a liquid crystal layer.

(ii) Formation of the optically anisotropic body

UV rays were applied to the coated surface side of the liquid crystal layer produced as stated above at a dose of 2000 mJ/cm$^2$ at the temperature as listed in the following Table 2-1 to effect polymerization to obtain an optically anisotropic body with a transparent glass substrate which is the wavelength dispersion measurement sample. Here, the film thickness of the optically anisotropic body (liquid crystal polymer film) was measured by scratching the optically anisotropic body of the optically anisotropic body with a transparent glass substrate with a needle, and the step height was measured by DEKTAK 150 surface profilometer (manufactured by ULVAC, Inc). The results are listed in Table 2-1.

(iii) Measurement of retardation

The retardation between wavelengths of 400 nm and 800 nm was measured using the sample obtained in the aforementioned (ii) utilizing a Mueller Matrix Polarimeter Axoscan (manufactured by Axometrics Inc). The retardation at a wavelength of 550 nm is listed in Table 2-1.

(iv) Evaluation of wavelength dispersion

The wavelength dispersion was evaluated based on the wavelength dispersion ratio that was calculated as described below using the measured retardation. The results are listed in Table 2-2.

(wavelength dispersion ratio at 400 nm) = (retardation value at 400 nm) / (retardation value at 550 nm)

(wavelength dispersion ratio at 410 nm) = (retardation value at 410 nm) / (retardation value at 550 nm)

(wavelength dispersion ratio at 420 nm) = (retardation value at 420 nm) / (retardation value at 550 nm)

(wavelength dispersion ratio at 430 nm) = (retardation value at 430 nm) / (retardation value at 550 nm)

(wavelength dispersion ratio at 440 nm) = (retardation value at 440 nm) / (retardation value at 550 nm)

(wavelength dispersion ratio at 450 nm) = (retardation value at 450 nm) / (retardation value at 550 nm)

(wavelength dispersion ratio at 600 nm) = (retardation value at 600 nm) / (retardation value at 550 nm)

(wavelength dispersion ratio at 650 nm) = (retardation value at 650 nm) / (retardation value at 550 nm)

(wavelength dispersion ratio at 700 nm) = (retardation value at 700 nm) / (retardation value at 550 nm)

(wavelength dispersion ratio at 750 nm) = (retardation value at 750 nm) / (retardation value at 550 nm)

(wavelength dispersion ratio at 800 nm) = (retardation value at 800 nm) / (retardation value at 550 nm)

<Measurement of Difference between Retardation where Lightness is the Lowest and Retardation where Saturation is the Lowest>

The difference between the retardation where the lightness is the lowest and the retardation where the saturation is the lowest was obtained by performing a simulation using the retardation measured in the aforementioned (iii).

The simulation was implemented assuming an optical system in which a linear polarizer / a liquid crystal polymer film / an ideal mirror were laminated in this order, as illustrated in FIG. 1. Here, the angle formed by the absorption axis of the linear polarizer and the slow axis of the liquid crystal polymer film was assumed to be 45°.

<<STEP. 1>>

The retardation Re (λ) at each wavelength λ (nm) measured in (ii) was used to calculate the value of the wavelength λ = wavelength dispersion from 380 nm to 780 nm: Re (λ) / Re (550) at 5 nm intervals. The wavelengths for which direct data could not be measured were calculated by linear interpolation from the data of two nearby points.

The values of the wavelength dispersion were used to calculate the retardation value of each wavelength λ (nm):

Re1 (λ) = Re1 (550) × Re (λ) / Re (550) when the wavelength λ = retardation value at 550 nm was set to Re1 (550).

<<STEP. 2>>

In the optical system of FIG. 1, the reflectance R (λ) of the light at a wavelength λ (nm) incident from a normal direction of the linear polarizer is represented by (Formula 1).

$$R(\lambda)=\frac{1}{8}\left[\left|T_A\left\{\left(T_A+T_B\right)e^{-2i\delta(\lambda)}+\left(T_A-T_B\right)\right\}\right|^2+\left|T_B\left\{\left(T_A+T_B\right)e^{-2i\delta(\lambda)}+\left(T_B-T_A\right)\right\}\right|^2\right] \quad \text{... (Formula 1)}$$

Here, $T_A$ is a value represented by the transmittance of the absorption axis direction of the linear polarizer, $T_B$ is a value represented by the transmittance in a direction perpendicular to the absorption axis direction of the linear polarizer, e is a value represented by a natural logarithm, i is a value represented by an imaginary unit, and δ (λ) is a value represented by 2πRe (λ)/λ where the in-plane retardation at a wavelength λ (nm)of the liquid crystal polymer film is Re (λ). $T_A$ and $T_B$ are values which are dependent upon the wavelength λ (nm), and the values of $T_A$ and $T_B$ used in the simulation are listed in Table 3. Further, the parentheses represented by || mean the calculation of the absolute value.

The retardation value Re1 (λ) of each wavelength λ obtained by (Formula 1) and STEP. 1 were used to calculate the retardation R (λ) of wavelength λ = from 380 nm to 780 nm at 5 nm intervals.

<<STEP. 3>>

The retardation R (λ) obtained in STEP. 2 was used in the following (Formula 2) to (Formula 4) to calculate the tristimulus values X, Y, and Z.

$$X=\sum_{n=0}^{80} S(380+5n)*R(380+5n)*x(380+5n)$$ ... (Formula 2)

$$Y=\sum_{n=0}^{80} S(380+5n)*R(380+5n)*y(380+5n)$$ ... (Formula 3)

$$Z=\sum_{n=0}^{80} S(380+5n)*R(380+5n)*y(380+5n)$$ ... (Formula 4)

Here, S (λ) is the spectrum of the light source, and the simulation used D65 light source values. Further, x (λ), y (λ) and z (λ) represent color-matching functions.

<<STEP. 4>>

The tristimulus values X, Y, and Z calculated in STEP. 3 were used to calculate the lightness L*, a*, and b* of the CIE 1976 L*a*b* color space. The following (Formula 5) to (Formula 7) were used for the calculation.

$$L^*=116f\,(Y/Yn)-16$$ ... (Formula 5)

$$a^*=500\left\{f\left(\frac{X}{X_n}\right)-f\left(\frac{Y}{Y_n}\right)\right\}$$ ... (Formula 6)

$$b^*=200\left\{f\left(\frac{Y}{Y_n}\right)-f\left(\frac{Z}{Z_n}\right)\right\}$$ ... (Formula 7)

Here, $X_n$, $Y_n$, and $Z_n$ are the respective tristimulus values calculated by (Formula 8) to (Formula 10)

$$X_n=\sum_{n=0}^{80} S(380+5n)*x(380+5n)$$ ... (Formula 8)

$$Y_n=\sum_{n=0}^{80} S(380+5n)*y(380+5n)$$ ... (Formula 9)

$$Z_n=\sum_{n=0}^{80} S(380+5n)*z(380+5n)$$ ... (Formula 10)

Further, f ($X/X_n$), f ($Y/Y_n$), and f ($Z/Z_n$) are respectively represented by (Formula 11-1) to (Formula 13-2).

$$f\left(\frac{X}{X_n}\right)=\left(\frac{X}{X_n}\right)^{1/3} \qquad \frac{X}{X_n}>0.008856$$

... (Formula 11-1)

$$f\left(\frac{X}{X_n}\right)=7.787\left(\frac{X}{X_n}\right)+\frac{16}{116} \qquad \frac{X}{X_n}\leqq 0.008856$$

... (Formula 11-2)

$$f\left(\frac{Y}{Y_n}\right)=\left(\frac{Y}{Y_n}\right)^{1/3} \qquad \frac{Y}{Y_n}>0.008856$$

... (Formula 12-1)

$$f\left(\frac{Y}{Y_n}\right)=7.787\left(\frac{Y}{Y_n}\right)+\frac{16}{116} \qquad \frac{Y}{Y_n}\leqq 0.008856$$

... (Formula 12-2)

$$f\left(\frac{Z}{Z_n}\right)=\left(\frac{Z}{Z_n}\right)^{1/3} \qquad \frac{Z}{Z_n}>0.008856$$

... (Formula 13-1)

$$f\left(\frac{Z}{Z_n}\right)=7.787\left(\frac{Z}{Z_n}\right)+\frac{16}{116} \qquad \frac{Z}{Z_n}\leqq 0.008856$$

... (Formula 13-2)

Furthermore, the saturation C* was calculated from the obtained values of a* and b* using (Formula 14).

$$C^*=\sqrt{(a^*)^2+(b^*)^2}$$

... (Formula 14)

<<STEP. 5>>

STEP. 1 to STEP. 4 were repeated every 0.1 nm between Retardation Re1 (550) = 110 to 180 nm to calculate the lightness L* and the saturation C* at each retardation. Moreover, the retardation Re1 (550) where the lightness L* and the saturation C* are the lowest were respectively obtained, and the difference examined.

Note that it is preferable that the difference between the retardation where the lightness is the lowest and the retardation where the saturation is the lowest is small.

The difference between the retardation where the lightness is the lowest and the retardation where the saturation is the lowest is listed in Table 4.

It is understood from Table 2-2 that in the optically anisotropic bodies formed using Examples 1 to 7, i.e., Polymerizable Compositions 1 to 7 containing Compounds 1 to 7, the divergence from the ideal value of the wavelength dispersion ratio was small on the short side of the wavelength 400 nm to 450 nm, and thus, an improvement was seen.

Further, it is understood from Table 2-2 that the divergence from the ideal value of the wavelength dispersion

ratio is made to a minimum value on the longer wavelength side.

Furthermore, it is understood from Table 4 that the difference between the retardation where the lightness is the lowest and the retardation where the saturation is the lowest becomes smaller, and thus, an improvement is seen.

As stated above, it is understood that in Examples 1 to 7, the production of an optically anisotropic body capable of more preferable polarization conversion than Comparative Example 1 becomes possible in the region from 400 nm to 800 nm.

Table 2-1

| | Polymerizable Composition | Polymerizable Compound (Use ratio; mass%) | | Coated wire bar | Drying temperature (°C) | Alignment treatment temperature (°C) | Exposure temperature (°C) | Film thickness (μm) | Phase difference (nm) at 550 nm |
|---|---|---|---|---|---|---|---|---|---|
| Example 1 | 1 | Compound 1 (50) | Compound X (50) | #6 | 145 | 23 | 23 | 2.13 | 138.94 |
| Example 2 | 2 | Compound 2 (50) | Compound X (50) | #6 | 140 | 23 | 23 | 2.05 | 122.01 |
| Example 3 | 3 | Compound 3 (50) | Compound X (50) | #6 | 120 | 23 | 23 | 2.07 | 113.67 |
| Example 4 | 4 | Compound 4 (50) | Compound X (50) | #6 | 120 | 23 | 23 | 2.18 | 113.78 |
| Example 5 | 5 | Compound 5 (50) | Compound X (50) | #6 | 120 | 23 | 23 | 2.11 | 133.68 |
| Example 6 | 6 | Compound 6 (100) | - | #6 | 190 | 23 | 23 | 1.96 | 141.45 |
| Example 7 | 7 | Compound 7 (100) | - | #8 | 195 | 130 | 130 | 1.50 | 124.96 |
| Comparative Example 1 | 1r | Compound X (100) | - | #6 | 110 | 23 | 23 | 1.91 | 141.14 |

Table 2-2

| | Polymerizable Composition | Wavelength dispersion ratio | | | | | | Wavelength dispersion ratio | | | | |
|---|---|---|---|---|---|---|---|---|---|---|---|---|
| | | 400 nm | 410 nm | 420 nm | 430 nm | 440 nm | 450 nm | 600 nm | 650 nm | 700 nm | 750 nm | 800 nm |
| Example 1 | 1 | 0.3135 | 0.5414 | 0.6755 | 0.7583 | 0.8107 | 0.8592 | 1.0175 | 1.0271 | 1.0327 | 1.0379 | 1.0400 |
| Example 2 | 2 | 0.4031 | 0.6059 | 0.7349 | 0.8017 | 0.8447 | 0.8680 | 1.0085 | 1.0160 | 1.0205 | 1.0241 | 1.0252 |
| Example 3 | 3 | 0.4421 | 0.6060 | 0.7220 | 0.7994 | 0.8455 | 0.8748 | 1.0068 | 1.0154 | 1.0185 | 1.0207 | 1.0225 |
| Example 4 | 4 | 0.4027 | 0.6044 | 0.7232 | 0.7942 | 0.8322 | 0.8767 | 1.0100 | 1.0192 | 1.0223 | 1.0257 | 1.0274 |
| Example 5 | 5 | 0.3071 | 0.5302 | 0.6795 | 0.7662 | 0.8262 | 0.8614 | 1.0143 | 1.0239 | 1.0260 | 1.0307 | 1.0322 |
| Example 6 | 6 | 0.1859 | 0.4449 | 0.6104 | 0.7085 | 0.7721 | 0.8165 | 1.0198 | 1.0320 | 1.0375 | 1.0420 | 1.0463 |
| Example 7 | 7 | 0.2963 | 0.5077 | 0.6690 | 0.7719 | 0.8256 | 0.8571 | 1.0138 | 1.0221 | 1.0283 | 1.0336 | 1.0380 |
| Comparative Example 1 | 1r | 0.0914 | 0.3787 | 0.5654 | 0.6711 | 0.7299 | 0.7788 | 1.0242 | 1.0368 | 1.0429 | 1.0481 | 1.0509 |
| Ideal value | | 0.7273 | 0.7455 | 0.7636 | 0.7818 | 0.8000 | 0.8182 | 1.0909 | 1.1818 | 1.2727 | 1.3636 | 1.4545 |

Table 3

| Waveleneth | TA | TB |
|---|---|---|
| | | |
| 380 | 0.016996 | 0.205748 |
| 385 | 0.023216 | 0.311129 |
| 390 | 0.03146 | 0.465522 |
| 395 | 0.032793 | 0.577067 |
| 400 | 0.034147 | 0.71151 |
| 405 | 0.03108,7 | 0.766128 |
| 410 | 0.028366 | 0.823453 |
| 415 | 0.025277 | 0.84332 |
| 420 | 0.022587 | 0.863176 |
| 425 | 0.020339 | 0.872752 |
| 430 | 0.01836 | 0.882208 |
| 435 | 0.016727 | 0.888847 |
| 440 | 0.015271 | 0.895383 |
| 445 | 0.013854 | 0.900307 |
| 450 | 0.012596 | 0.905148 |
| 455 | 0.011561 | 0.908662 |
| 460 | 0.01063 | 0.912112 |
| 465 | 0.,009964 | 0.915249 |
| 470 | 0.009352 | 0.91833 |
| 475 | 0.009226 | 0.922235 |
| 480 | 0.009105 | 0.926015 |
| 485 | 0.009358 | 0.928826 |
| 490 | 0.009613 | 0.931529 |
| 495 | 0.010215 | 0.932406 |
| 500 | 0.010842 | 0.933267 |
| 505 | 0.011298 | 0.935613 |
| 510 | 0.011764 | 0.931918 |
| 515 | 0.011436 | 0.93906 |
| 520 | 0.011124 | 0.940182 |
| 525 | 0.01019 | 0.939532 |
| 530 | 0.00935 | 0.938894 |
| 535 | 0.008265 | 0.940612 |
| 540 | 0.007323 | 0.942301 |
| 545 | 0.006235 | 0.940588 |
| 550 | 0.005325 | 0.938909 |
| 555 | 0.004749 | 0.940509 |

(continued)

| Waveleneth | TA | TB |
|---|---|---|
| 560 | 0.004244 | 0.942082 |
| 565 | 0.003883 | 0.940297 |
| 570 | 0.003559 | 0.938548 |
| 575 | 0.003441 | 0.939953 |
| 580 | 0.003329 | 0.941337 |
| | | |
| 585 | 0.003122 | 0.940007 |
| 590 | 0.002931 | 0.938701 |
| 595 | 0.002928 | 0.94066 |
| 600 | 0.002925 | 0.94259 |
| 605 | 0.002795 | 0.941493 |
| 610 | 0.002672 | 0.940415 |
| 615 | 0.002823 | 0.942363 |
| 620 | 0.00298 | 0.944324 |
| 625 | 0.003099 | 0.944175 |
| 630 | 0.003218 | 0.944029 |
| 635 | 0.003742 | 0.944596 |
| 640 | 0.00434 | 0.945156 |
| 645 | 0.005054 | 0.946915 |
| 650 | 0.005873 | 0.94865 |
| 655 | 0.006997 | 0.947883 |
| 660 | 0.009315 | 0.947128 |
| 665 | 0.010112 | 0.949429 |
| 670 | 0.012261 | 0.951701 |
| 675 | 0.015358 | 0.953001 |
| 680 | 0.019173 | 0.954284 |
| 685 | 0.023786 | 0.953604 |
| 690 | 0.029417 | 0.952935 |
| 695 | 0.036584 | 0.954836 |
| 700 | 0.045356 | 0.956715 |
| 705 | 0.056037 | 0.958176 |
| 710 | 0.069028 | 0.959618 |
| 715 | 0.083532 | 0.958562 |
| 720 | 0.100816 | 0.957521 |
| 725 | 0.121266 | 0.959663 |
| 730 | 0.145495 | 0.961781 |
| 735 | 0.171937 | 0.963267 |
| 740 | 0.202727 | 0.964736 |

(continued)

| Waveleneth | TA | TB |
|---|---|---|
| 745 | 0.232634 | 0.963594 |
| 750 | 0.266464 | 0.96245 |
| 755 | 0.300545 | 0.962311 |
| 760 | 0.338449 | 0.962175 |
| 765 | 0.37645 | 0.969031 |
| 770 | 0.419141 | 0.975845 |
| 775 | 0.454473 | 0.979681 |
| 780 | 0.493434 | 0.983482 |

Table 4

| | Amount of deviation (nm) | Retardation where lightness L* is lowest (nm) | Retardation where saturation C* is lowest (nm) |
|---|---|---|---|
| Example 1 | 2.5 | 137.2 | 139.7 |
| Example 2 | 0.0 | 137.7 | 137.7 |
| Example 3 | 0.5 | 137.9 | 138.4 |
| Example 4 | 0.0 | 137.7 | 137.7 |
| Example 5 | 3.4 | 137.1 | 140.5 |
| Example 6 | 3.9 | 136.6 | 140.5 |
| Example 7 | 2.5 | 137.3 | 139.8 |
| Comparative Example 1 | 5.8 | 140.9 | 146.7 |

**Claims**

**1.** A polymerizable compound represented by formula (I-1):

$$P^1-G^1-Y^3\left[B^1-Y^1\right]_pA^1-Z^1-Ar^0-Z^2-Xa-Z^3-Ar^1-Z^4-A^2\left[Y^2-B^2\right]_qY^4-G^2-P^2$$

**(I-1)**

where in the formula (I-1),

$Ar^0$ represents an aromatic hydrocarbon ring group having at least $D^0$ as a substituent, or, an aromatic heterocyclic ring group having at least $D^0$ as a substituent,
$Ar^1$ represents an aromatic hydrocarbon ring group having at least $D^1$ as a substituent, or, an aromatic heterocyclic ring group having at least $D^1$ as a substituent,
$D^0$ and $D^1$ each independently represent an organic group having 1 to 67 carbon atoms and having at least one aromatic ring selected from the group consisting of an aromatic hydrocarbon ring and an aromatic heterocyclic ring,
Xa represents an organic group having 1 to 20 carbon atoms which may have a substituent,
$Z^1$ to $Z^4$ each independently represent a single bond, -O-, $-O-CH_2-$, $-CH_2-O-$, $-O-CH_2-CH_2$, $-CH_2-CH_2-O-$, -C(=O)-O-, -O-C(=O)-, -C(=O)-S-, -S-C(=O)-, $-NR^{20}-C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-C(=O)-O-$, $-O-C(=O)-CH_2-$, $-CH_2-O-C(=O)-$, $-C(=O)-O-CH_2-$, $-CH_2-CH_2-C(=O)-O-$, $-O-C(=O)-CH_2-CH_2-$, $-CH_2-CH_2-O-C(=O)-$, $-C(=O)-O-CH_2-CH_2-$,

-CH=CH-, -N=CH-, -CH=N-, -N=C($CH_3$)-, -C($CH_3$)=N-, -N=N-, or -C=C-, and $R^{20}$ represents a hydrogen atom or an alkyl group having 1 to 6 carbon atoms,
$A^1$ and $A^2$ each independently represent an aromatic group which may have a substituent,
$B^1$ and $B^2$ each independently represent a cyclic aliphatic group which may have a substituent, or, an aromatic group which may have a substituent,
$Y^1$ to $Y^4$ each independently represent a single bond, -O-, -C(=O)-, -C(=O)-O-, -O-C(=O)-, -$NR^{21}$-C(=O)-, -C(=O)-$NR^{21}$-, -O-C(=O)-O-, -NR $^{21}$-C (=O)-O-, -O-C(=O)-$NR^{21}$-, or, -$NR^{21}$-C(=O)-NR $^{22}$-, and $R^{21}$ and $R^{22}$ each independently represent a hydrogen atom or an alkyl group having 1 to 6 carbon atoms,
$G^1$ and $G^2$ each independently represent an organic group of either an alkylene group having 1 to 20 carbon atoms, or, an alkylene group having 3 to 20 carbon atoms in which at least one methylene group (-$CH_2$-) is substituted with -O- or -C(=O)-, and the hydrogen atom included in the organic group of $G^1$ and $G^2$ may be substituted with an alkyl group having 1 to 5 carbon atoms, an alkoxy group having 1 to 5 carbon atoms, or, a halogen atom, where the methylene group (-$CH_2$-) at each terminal of $G^1$ and $G^2$ is not substituted with -O- or -C(=O)-,
one of $P^1$ and $P^2$ represents a hydrogen atom or a polymerizable group, and the other of $P^1$ and $P^2$ represents a polymerizable group, and
p and q each independently represent an integer from 0 to 2,
where when a plurality of $B^1$, $B^2$, $Y^1$, and $Y^2$ are present, these may be the same or different.

**2.** The polymerizable compound according to claim 1, wherein each of $Ar^0$ and $Ar^1$ is independently represented by any of the following formulas (II-1) to (II-7):

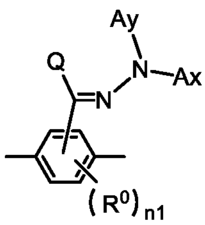
(II-1)

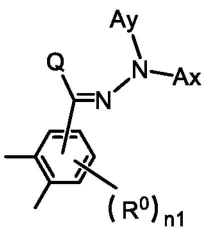
(II-2)

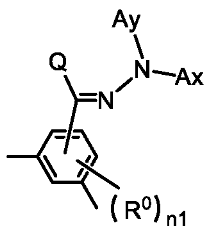
(II-3)

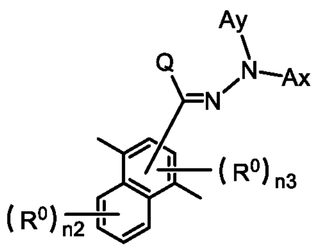
(II-4)

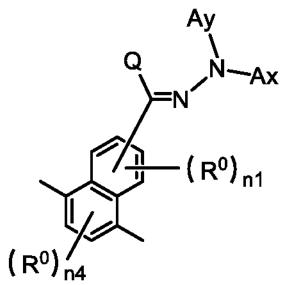
(II-5)

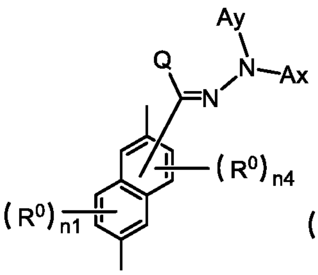
(II-6)

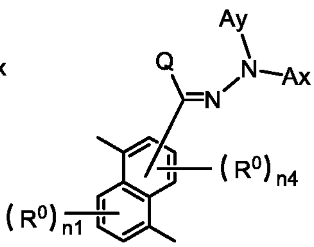
(II-7)

where in the formulas (II-1) to (II-7),

Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring of Ax may have a substituent,
Ay represents a hydrogen atom or an organic group having 1 to 30 carbon atoms which may have a substituent,
Q represents a hydrogen atom or an alkyl group having 1 to 6 carbon atoms, and
$R^0$ represents a halogen atom, a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N-N-dialkylamino group having 2 to 12 carbon atoms, an alkoxy group having 1 to 6 carbon atoms, a nitro group, -C(=O)-$R^a$, -O-C(=O)-$R^a$, -C(=O)-O-$R^a$, or -$SO_2R^a$, $R^a$ represents an alkyl group having 1 to 6 carbon atoms, or, an aromatic hydrocarbon ring group having 6 to 20 carbon atoms

which may have an alkyl group having 1 to 6 carbon atoms or an alkoxy group having 1 to 6 carbon atoms as a substituent, and n1 represents an integer from 0 to 3, n2 represents an integer from 0 to 4, n3 is 0 or 1, and n4 represents an integer from 0 to 2,
where when a plurality of $R^0$ are present, these may be the same or different.

**3.** The polymerizable compound according to claim 2, wherein the polymerizable compound is represented by any of the following formulas (III-1) to (III-6):

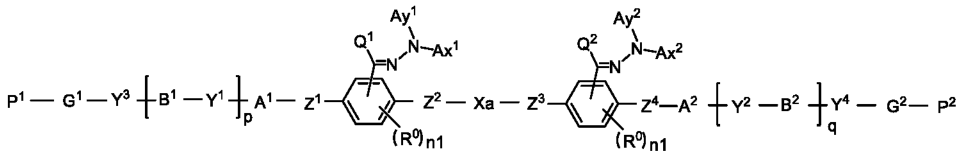
(III-1)

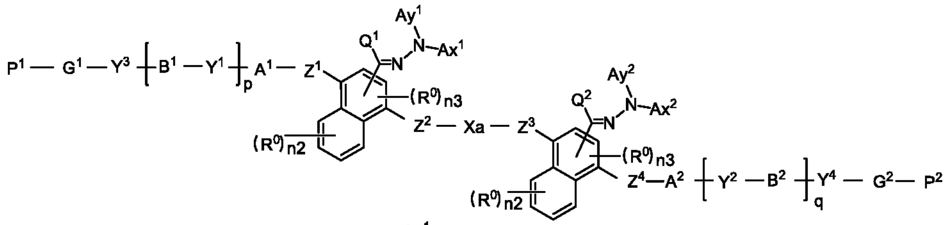
(III-2)

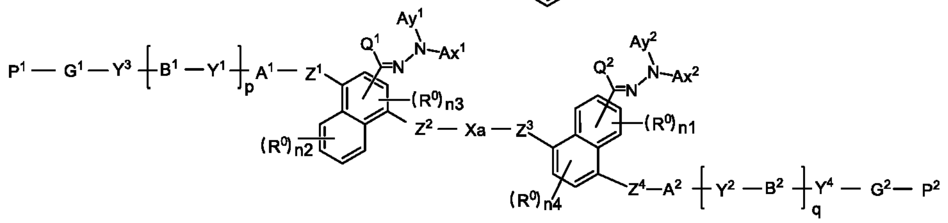
(III-3)

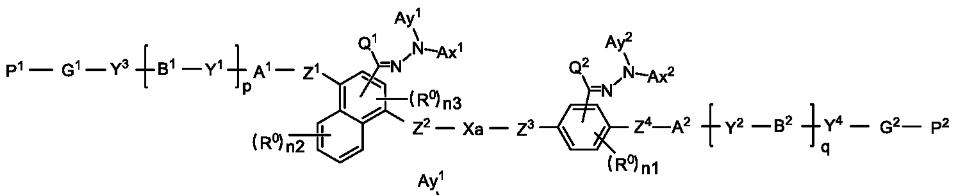
(III-4)

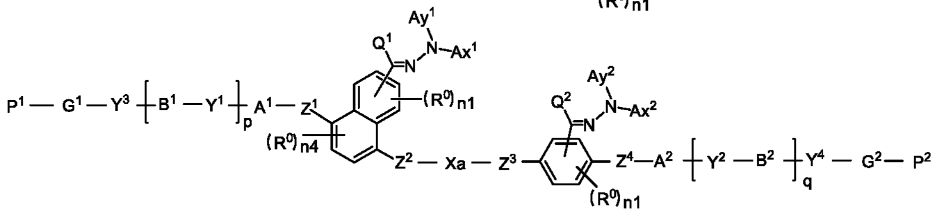
(III-5)

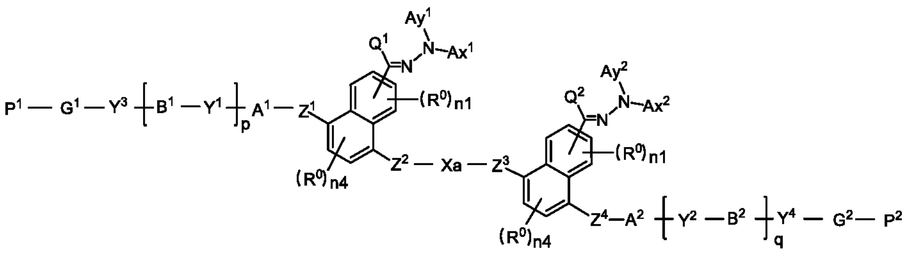
(III-6)

where in the formulas (III-1) to (III-6),

$Z^1$ to $Z^4$, $A^1$, $A^2$, $B^1$, $B^2$, $Y^1$ to $Y^4$, $G^1$, $G^2$, $P^1$, $P^2$, Xa, $R^0$, n1, n2, n3, n4, p, and q are the same as defined in the preceding claim,
$Ax^1$ and $Ax^2$ each independently represent an organic group having at least one aromatic ring selected from the group consisting of an aromatic hydrocarbon ring having 6 to 30 carbon atoms and an aromatic heterocyclic

ring having 2 to 30 carbon atoms, and the aromatic rings of $Ax^1$ and $Ax^2$ may have a substituent,
$Ay^1$ and $Ay^2$ each independently represent a hydrogen atom or an organic group having 1 to 30 carbon atoms which may have a substituent, and
$Q^1$ and $Q^2$ each independently represent a hydrogen atom or an alkyl group having 1 to 6 carbon atoms,
where when a plurality of $B^1$, $B^2$, $Y^1$, $Y^2$, and $R^0$ are present, these may be the same or different.

**4.** The polymerizable compound according to claim 3, wherein $Ay^1$ and $Ay^2$ each independently represent a hydrogen atom, an alkyl group having 1 to 20 carbon atoms which may have a substituent, an alkenyl group having 2 to 20 carbon atoms which may have a substituent, an alkynyl group having 2 to 20 carbon atoms which may have a substituent, a cycloalkyl group having 3 to 12 carbon atoms which may have a substituent, an aromatic hydrocarbon ring group having 6 to 30 carbon atoms which may have a substituent, or, an aromatic heterocyclic ring group having 2 to 30 carbon atoms which may have a substituent.

**5.** The polymerizable compound according to claim 3 or 4, wherein $Ax^1$ and $Ax^2$ are each independently represented by the following formula (V):

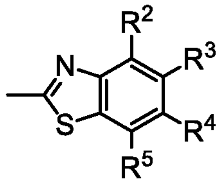
(V)

where in the formula (V), $R^2$ to $R^5$ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, $-OCF_3$, O-C(=O)$R^b$, or -C(=O)-O-$R^b$,
$R^b$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or, an aromatic hydrocarbon ring group having 5 to 18 carbon atoms which may have a substituent, and
each of $R^2$ to $R^5$ may be the same or different, and one or more ring constituents C-$R^2$ to C-$R^5$ may be replaced by a nitrogen atom.

**6.** The polymerizable compound according to any one of claims 1 to 5, wherein $P^1$ and $P^2$ are each independently represented by the following formula (IV).

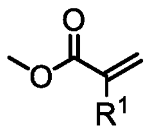
(IV)

where in the formula (IV), $R^1$ represents a hydrogen atom, a methyl group, or a chlorine atom.

**7.** The polymerizable compound according to any one of claims 1 to 6, wherein p and q are both 0.

**8.** The polymerizable compound according to any one of claims 1 to 6, wherein p and q are both 1, and, $B^1$ and $B^2$ each independently represent a cyclic aliphatic group which may have a substituent.

**9.** The polymerizable compound according to any one of claims 1 to 7, wherein the polymerizable compound represented by the formula (I-1) is represented by any of the following formulas (VI-1) to (VI-3):

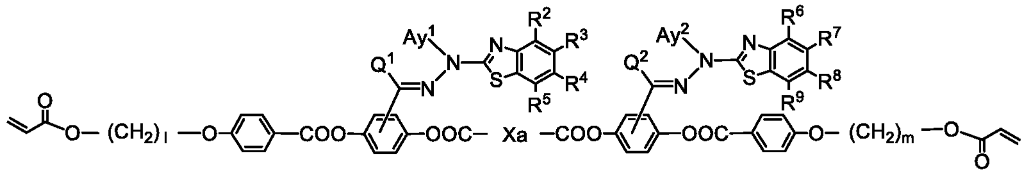

(VI-1)

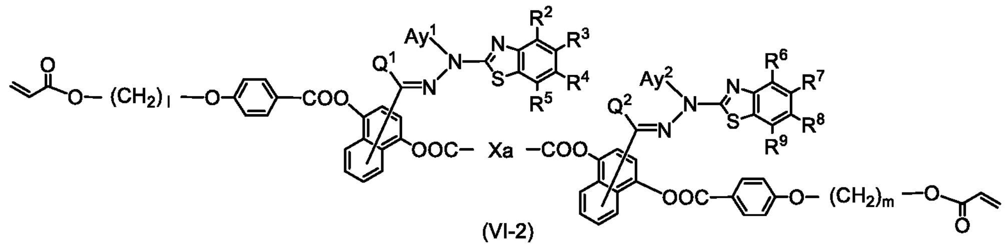

(VI-2)

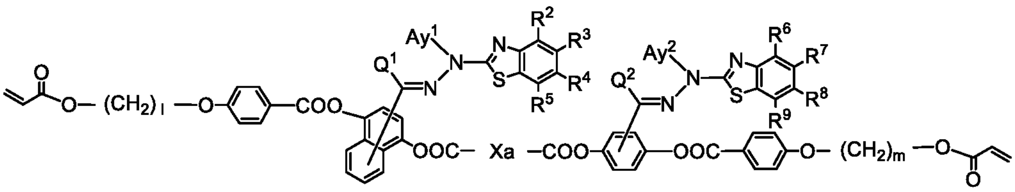

(VI-3)

where in the formulas (VI-1) to (VI-3),

Xa is the same as defined in the preceding claims,
$R^2$ to $R^9$ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, $-OCF_3$, $-O-C(=O)-R^b$, or $-C(=O)-O-R^b$,
$R^b$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or, an aromatic hydrocarbon ring group having 5 to 18 carbon atoms which may have a substituent,
one or more ring constituents $C-R^2$ to $C-R^9$ may be replaced by a nitrogen atom,
$Ay^1$ and $Ay^2$ each independently represent a hydrogen atom or an organic group having 1 to 30 carbon atoms which may have a substituent,
$Q^1$ and $Q^2$ each independently represent a hydrogen atom or an alkyl group having 1 to 6 carbon atoms, and
1 and m each independently represent an integer from 1 to 18.

**10.** The polymerizable compound according to any one of claims 1 to 9, wherein Xa is represented by any of the following formulas (VII-1) to (VII-29) :

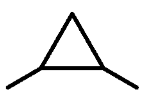 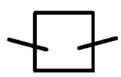 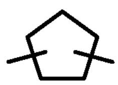 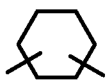 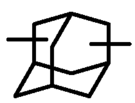 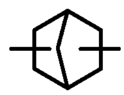

(VII-1) (VII-2) (VII-3) (VII-4) (VII-5) (VII-6)

$-CH_2-$ $-(CH_2)_2-$ $-(CH_2)_3-$ $-(CH_2)_4-$ $-(CH_2)_5-$

(VII-7) (VII-8) (VII-9) (VII-10) (VII-11)

$-(CH_2)_6-$ (VII-12) $-(CH_2)_7-$ (VII-13) $-(CH_2)_8-$ (VII-14) $-(CH_2)_9-$ (VII-15) $-(CH_2)_{10}-$ (VII-16)

$-(CH_2)_{11}-$ (VII-17) $-(CH_2)_{12}-$ (VII-18) $-(CH_2)_{13}-$ (VII-19) $-(CH_2)_{14}-$ (VII-20) $-(CH_2)_{15}-$ (VII-21)

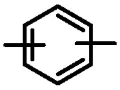
(VII-22)
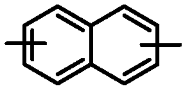
(VII-23)
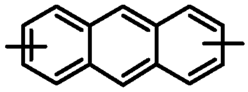
(VII-24)
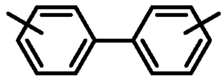
(VII-25)

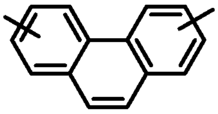
(VII-26)
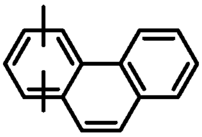
(VII-27)
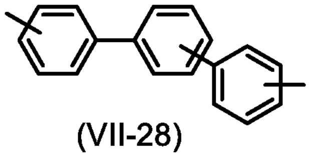
(VII-28)

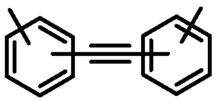
(VII-29)

**11.** A polymerizable liquid crystal mixture comprising the polymerizable compound according to any one of claims 1 to 10 as a main component.

**12.** The polymerizable liquid crystal mixture according to claim 11 comprising the polymerizable compound according to any one of claims 1 to 10 and a polymerizable compound having a different chemical structure than the following formula (I-1), wherein
an area value of the polymerizable compound according to any one of claims 1 to 10 measured by high-performance liquid chromatography (HPLC) is a value greater than 50% of a sum of area values of the polymerizable compound according to any one of claims 1 to 10 and the polymerizable compound having a different chemical structure than the formula (I-1):

$$P^1-G^1-Y^3\left[B^1-Y^1\right]_p A^1-Z^1-Ar^0-Z^2-Xa-Z^3-Ar^1-Z^4-A^2\left[Y^2-B^2\right]_q Y^4-G^2-P^2$$

(I-1)

where in the formula (I-1),

$Ar^0$ represents an aromatic hydrocarbon ring group having at least $D^0$ as a substituent, or, an aromatic heterocyclic ring group having at least $D^0$ as a substituent,
$Ar^1$ represents an aromatic hydrocarbon ring group having at least $D^1$ as a substituent, or, an aromatic heterocyclic ring group having at least $D^1$ as a substituent,
$D^0$ and $D^1$ each independently represent an organic group having 1 to 67 carbon atoms and having at least one aromatic ring selected from the group consisting of an aromatic hydrocarbon ring and an aromatic heterocyclic ring,
Xa represents an organic group having 1 to 20 carbon atoms which may have a substituent,
$Z^1$ to $Z^4$ each independently represent a single bond, -O-, $-O-CH_2-$, $-CH_2-O-$, $-O-CH_2-CH_2$, $-CH_2-CH_2-O-$, -C(=O)-O-, -O-C(=O)-, -C(=O)-S-, -S-C(=O)-, $-NR^{20}-C(=O)-$, $-C(=O)-NR^{20}-$, $-CF_2-O-$, $-O-CF_2-$, $-CH_2-CH_2-$,

$-CF_2-CF_2-$, $-O-CH_2-CH_2-O-$, $-CH=CH-C(=O)-O-$, $-O-C(=O)-CH=CH-$, $-CH_2-C(=O)-O-$, $-O-C(=O)-CH_2-$, $-CH_2-O-C(=O)-$, $-C(=O)-O-CH_2-$, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-, and $R^{20}$ represents a hydrogen atom or an alkyl group having 1 to 6 carbon atoms,

$A^1$ and $A^2$ each independently represent an aromatic group which may have a substituent,

$B^1$ and $B^2$ each independently represent a cyclic aliphatic group which may have a substituent, or, an aromatic group which may have a substituent,

$Y^1$ to $Y^4$ each independently represent a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or, $-NR^{21}-C(=O)-NR^{22}-$, and $R^{21}$ and $R^{22}$ each independently represent a hydrogen atom or an alkyl group having 1 to 6 carbon atoms,

$G^1$ and $G^2$ each independently represent an organic group of either an alkylene group having 1 to 20 carbon atoms, or, an alkylene group having 3 to 20 carbon atoms in which at least one methylene group ($-CH_2-$) is substituted with -O- or -C(=O)-, and the hydrogen atom included in the organic group of $G^1$ and $G^2$ may be substituted with an alkyl group having 1 to 5 carbon atoms, an alkoxy group having 1 to 5 carbon atoms, or, a halogen atom, where the methylene group ($-CH_2-$) at each terminal of $G^1$ and $G^2$ is not substituted with -O- or -C(=O)-,

one of $P^1$ and $P^2$ represents a hydrogen atom or a polymerizable group, and the other of $P^1$ and $P^2$ represents a polymerizable group, and

p and q each independently represent an integer from 0 to 2,

where when a plurality of $B^1$, $B^2$, $Y^1$, and $Y^2$ are present, these may be the same or different.

**13.** The polymerizable liquid crystal mixture according to claim 11 or 12 comprising the polymerizable compound according to any one of claims 1 to 10, and a polymerizable compound represented by the following formula (1-2), wherein an area value of the polymerizable compound according to any one of claims 1 to 10 measured by high-performance liquid chromatography (HPLC) is a value greater than 50% of a sum of area values of the polymerizable compound according to any one of claims 1 to 10 and the polymerizable compound represented by the formula (I-2):

$$P^3-G^3-Y^7-\left[B^3-Y^5\right]_{p1}-A^3-Z^5-Ar^2-Z^6-A^4-\left[Y^6-B^4\right]_{q1}-Y^8-G^4-P^4$$

(I-2)

where in the formula (I-2),

$Ar^2$ represents an aromatic hydrocarbon ring group having at least $D^2$ as a substituent, or, an aromatic heterocyclic ring group having at least $D^2$ as a substituent,

$D^2$ represents an organic group having 1 to 67 carbon atoms and having at least one aromatic ring selected from the group consisting of an aromatic hydrocarbon ring and an aromatic heterocyclic ring,

$Z^5$ and $Z^6$ each independently represent a single bond, -O-, $-O-CH_2-$, $-CH_2-O-$, $-O-CH_2-CH_2$, $-CH_2-CH_2-O-$, -C(=O)-O-, -O-C(=O)-, -C(=O)-S-, -S-C(=O)-, $-NR^{20}-C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, $-CH=CH-C(=O)-O-$, $-O-C(=O)-CH=CH-$, $-CH_2-C(=O)-O-$, $-O-C(=O)-CH_2-$, $-CH_2-O-C(=O)-$, $-C(=O)-O-CH_2-$, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-, and $R^{20}$ represents a hydrogen atom or an alkyl group having 1 to 6 carbon atoms,

$A^3$, $A^4$, $B^3$ and $B^4$ each independently represent a cyclic aliphatic group which may have a substituent, or, an aromatic group which may have a substituent,

$Y^5$ to $Y^8$ each independently represent a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or , $-NR^{21}-C(=O)-NR^{22}-$, and $R^{21}$ and $R^{22}$ each independently represent a hydrogen atom or an alkyl group having 1 to 6 carbon atoms,

$G^3$ and $G^4$ each independently represent an organic group of either an alkylene group having 1 to 20 carbon atoms, or, an alkylene group having 3 to 20 carbon atoms in which at least one methylene group ($-CH_2-$) is substituted with -O- or -C(=O)-, and the hydrogen atom included in the organic group of $G^3$ and $G^4$ may be substituted with an alkyl group having 1 to 5 carbon atoms, an alkoxy group having 1 to 5 carbon atoms, or, a halogen atom,

one of $P^3$ and $P^4$ represents a hydrogen atom or a polymerizable group, and the other of $P^3$ and $P^4$ represents a polymerizable group, and

p1 and q1 each independently represent an integer from 0 to 2,

where when a plurality of $B^3$, $B^4$, $Y^5$, and $Y^6$ are present, these may be the same or different.

**14.** The polymerizable liquid crystal mixture according to claim 13, wherein $Ar^2$ is represented by any of the following formulas (II-1) to (II-7):

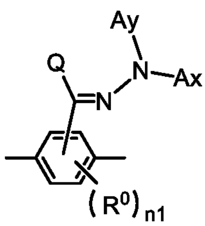
(II-1)

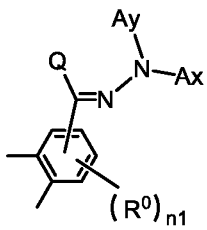
(II-2)

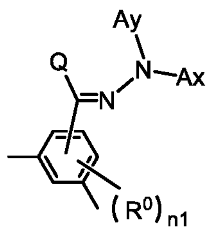
(II-3)

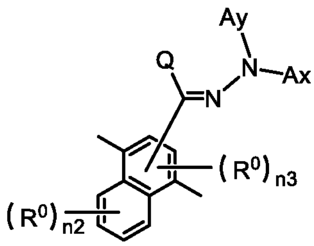
(II-4)

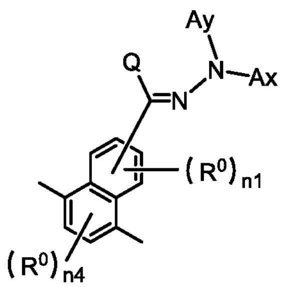
(II-5)

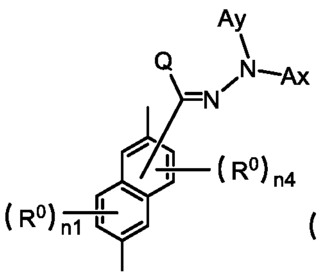
(II-6)

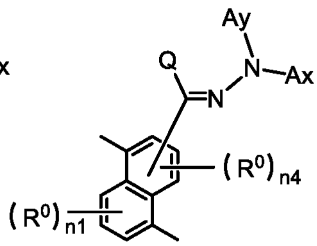
(II-7)

where in the formulas (II-1) to (II-7),

Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring of Ax may have a substituent,
Ay represents a hydrogen atom or an organic group having 1 to 30 carbon atoms which may have a substituent,
Q represents a hydrogen atom or an alkyl group having 1 to 6 carbon atoms, and
$R^0$ represents a halogen atom, a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N-N-dialkylamino group having 2 to 12 carbon atoms, an alkoxy group having 1 to 6 carbon atoms, a nitro group, $-C(=O)-R^a$, $-O-C(=O)-R^a$, $-C(=O)-O-R^a$, or $-SO_2R^a$, $R^a$ represents an alkyl group having 1 to 6 carbon atoms, or, an aromatic hydrocarbon ring group having 6 to 20 carbon atoms which may have an alkyl group having 1 to 6 carbon atoms or an alkoxy group having 1 to 6 carbon atoms as a substituent, and n1 represents an integer from 0 to 3, n2 represents an integer from 0 to 4, n3 is 0 or 1, and n4 represents an integer from 0 to 2,
where when a plurality of $R^0$ are present, these may be the same or different.

**15.** The polymerizable liquid crystal mixture according to claim 13 or 14, wherein $P^3$ and $P^4$ are each independently represented by the following formula (IV):

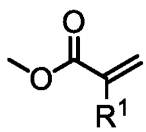
(IV)

where in the formula (IV), $R^1$ represents a hydrogen atom, a methyl group, or a chlorine atom.

**16.** A polymer obtainable by polymerizing the polymerizable liquid crystal mixture according to any one of claims 11 to 15.

**17.** An optical film composed of the polymer according to claim 16 as a constituent material.

**18.** An optically anisotropic body comprising a layer composed of the polymer according to claim 16 as a constituent material.

**19.** A polarizing plate comprising the optically anisotropic body according to claim 18 and a polarizing film.

**20.** A display device comprising the polarizing plate according to claim 19.

**21.** An antireflection film comprising the polarizing plate according to claim 19.

**22.** A compound represented by any of the following formulas (XI-1) to (XI-6):

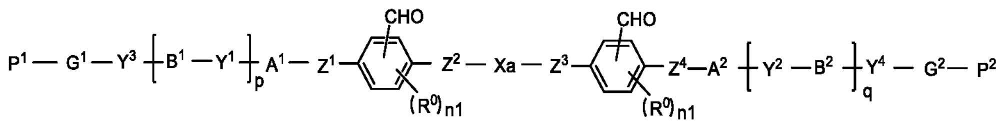

(XI-1)

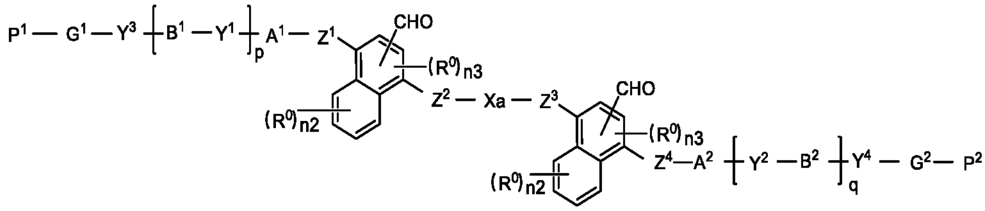

(XI-2)

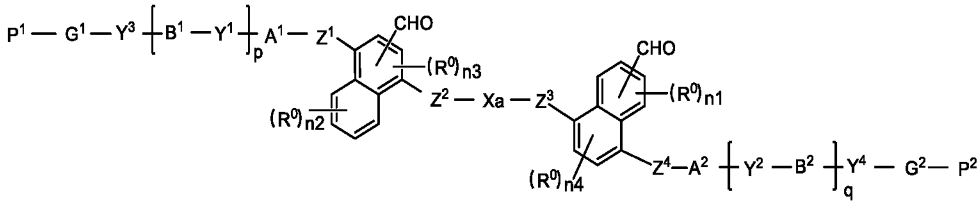

(XI-3)

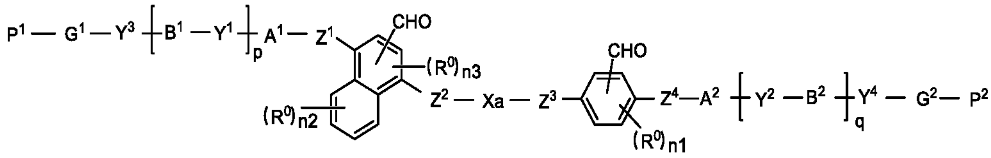

(XI-4)

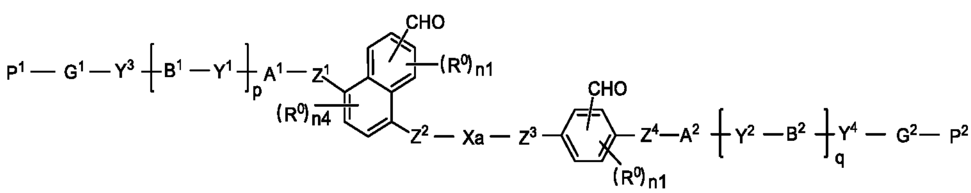

(XI-5)

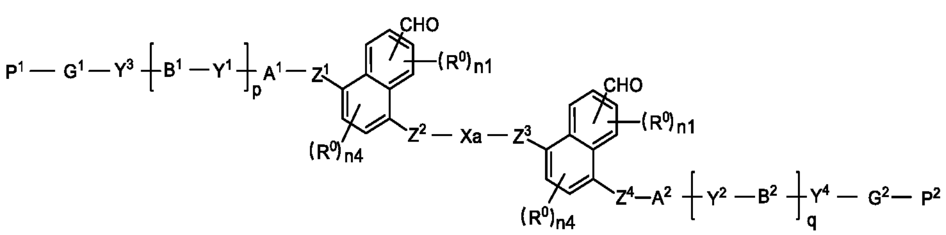

(XI-6)

where in the formulas (XI-1) to (XI-6),

Xa represents an organic group having 1 to 20 carbon atoms which may have a substituent,

$Z^1$ to $Z^4$ each independently represent a single bond, -O-, -O-$CH_2$-, -$CH_2$-O-, -O-$CH_2$-$CH_2$, -$CH_2$-$CH_2$-O-, -C(=O)-O-, -O-C(=O)-, -C(=O)-S-, -S-C(=O)-, -$NR^{20}$-C(=O)-, -C(=O)-$NR^{20}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-C(=O)-O-, -O-C(=O)-$CH_2$-, -$CH_2$-O-C(=O)-, -C(=O)-O-$CH_2$-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C≡C-, and $R^{20}$ represents a hydrogen atom or an alkyl group having 1 to 6 carbon atoms,

$A^1$ and $A^2$ each independently represent an aromatic group which may have a substituent,

$B^1$ and $B^2$ each independently represent a cyclic aliphatic group which may have a substituent, or, an aromatic group which may have a substituent,

$Y^1$ to $Y^4$ each independently represent a single bond, -O-, -C(=O)-, -C(=O)-O-, -O-C(=O)-, -$NR^{21}$-C(=O)-, -C(=O)-$NR^{21}$-, -O-C(=O)-O-, -$NR^{21}$-C(=O)-O-, -O-C(=O)-$NR^{21}$-, or, -$NR^{21}$-C(=O)-$NR^{22}$-, and $R^{21}$ and $R^{22}$ each independently represent a hydrogen atom or an alkyl group having 1 to 6 carbon atoms,

$G^1$ and $G^2$ each independently represent an organic group of either an alkylene group having 1 to 20 carbon atoms, or, an alkylene group having 3 to 20 carbon atoms in which at least one methylene group (-$CH_2$-) is substituted with -O- or -C(=O)-, and the hydrogen atom included in the organic group of $G^1$ and $G^2$ may be substituted with an alkyl group having 1 to 5 carbon atoms, an alkoxy group having 1 to 5 carbon atoms, or, a halogen atom, where the methylene group (-$CH_2$-) at each terminal of $G^1$ and $G^2$ is not substituted with -O- or -C(=O)-,

one of $P^1$ and $P^2$ represents a hydrogen atom or a polymerizable group, and the other of $P^1$ and $P^2$ represents a polymerizable group,

p and q each independently represent an integer from 0 to 2, and

$R^0$ represents a halogen atom, a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N-N-dialkylamino group having 2 to 12 carbon atoms, an alkoxy group having 1 to 6 carbon atoms, a nitro group, -C(=O)-$R^a$, -O-C(=O)-$R^a$, -C(=O)-O-$R^a$, or -$SO_2R^a$, $R^a$ represents an alkyl group having 1 to 6 carbon atoms, or, an aromatic hydrocarbon ring group having 6 to 20 carbon atoms which may have an alkyl group having 1 to 6 carbon atoms or an alkoxy group having 1 to 6 carbon atoms as a substituent, and n1 represents an integer from 0 to 3, n2 represents an integer from 0 to 4, n3 is 0 or 1, and n4 represents an integer from 0 to 2,

where when a plurality of $R^0$, $B^1$, $B^2$, $Y^1$ and $Y^2$ are present, these may be the same or different.

**23.** The compound according to claim 22 represented by any of the following formulas (XII-1) to (XII-3):

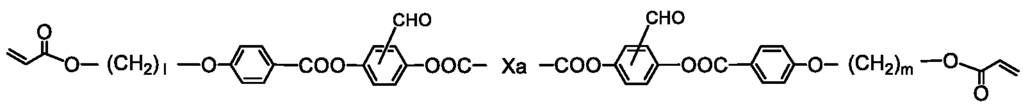

(XII-1)

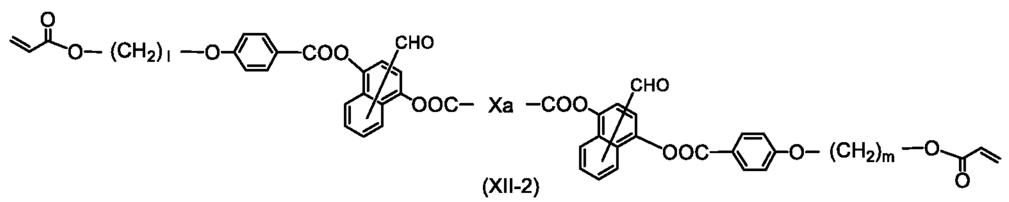

(XII-2)

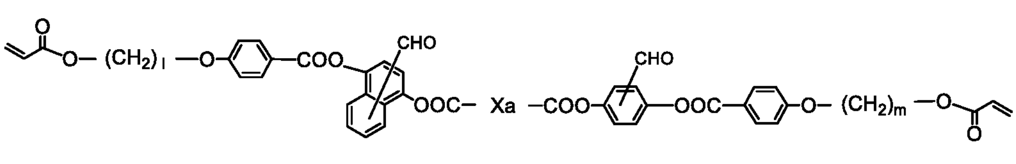

(XII-3)

where in the formulas (XII-1) to (XII-3),

Xa is the same as defined in the preceding claim, and
1 and m each independently represent an integer from 1 to 18.

FIG. 1

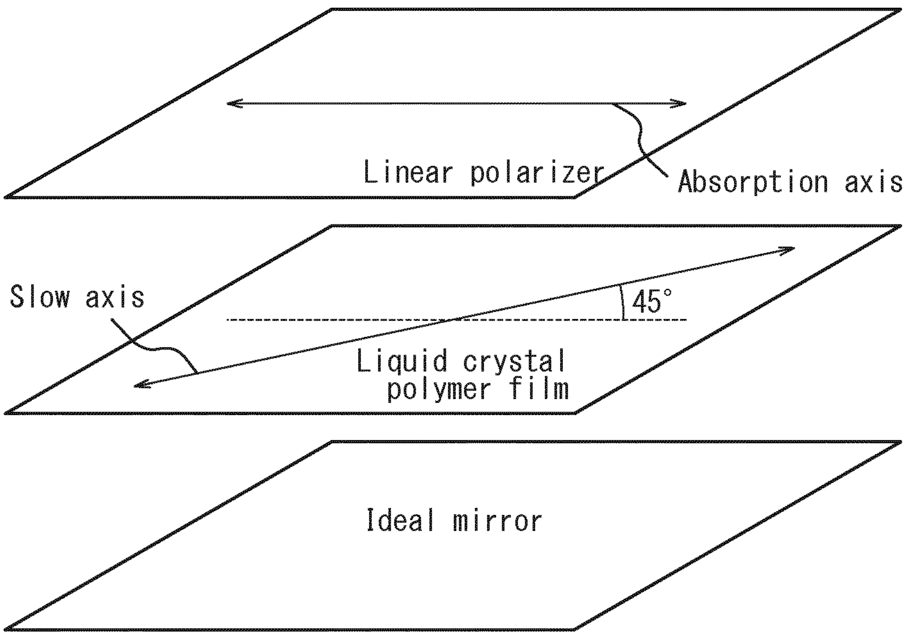
Linear polarizer
Absorption axis
Slow axis
45°
Liquid crystal polymer film
Ideal mirror

## INTERNATIONAL SEARCH REPORT

International application No. PCT/JP2018/009528

A. CLASSIFICATION OF SUBJECT MATTER
Int.Cl. C07D277/82(2006.01)i, C07C69/78(2006.01)i, C08F20/38(2006.01)i, C09K19/38(2006.01)i, G02B1/111(2015.01)i, G02B5/30(2006.01)i

According to International Patent Classification (IPC) or to both national classification and IPC

B. FIELDS SEARCHED

Minimum documentation searched (classification system followed by classification symbols)
Int.Cl. C07D277/82, C07C69/78, C08F20/38, C09K19/38, G02B1/111, G02B5/30

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2016/104317 A1 (DIC CORP.) 30 June 2016, claims, compounds (paragraphs [0002], [0373]-[0446]) (D11-1), (D11-2), (D11-4), (D11-5), (D11-6), (D11-7), (D12-2), (D12-9), (D4-1), example 74, paragraphs [0742]-[0827] & US 2017/0369783 A1, claims, paragraphs [0002], [0358], compounds (D11-1), (D11-2), (D11-4), (D11-5), (D11-6), (D11-7), (D12-2), (D12-9), (D4-1), example 74, paragraphs [0802]-[0820] & CN 107108458 A & KR 10-2017-0101194 A | 1-23<br>12-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 May 2018 (25.05.2018) | 05 June 2018 (05.06.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/009528

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | WO 2014/010325 A1 (NIPPON ZEON CO., LTD.) 16 January 2014, claims, example 4 & CN 103600039 A | 12-15<br>1-11, 16-23 |
| A | WO 2016/136533 A1 (DIC CORP.) 01 September 2016, claims, examples & US 2018/0022716 A1 & CN 107207419 A & KR 10-2017-0121174 A | 1-23 |
| A | JP 2006-243470 A (FUJI PHOTO FILM CO., LTD.) 14 September 2006, claims, compound G-50H (example 3) & US 2008/0186443 A1 & WO 2006/093351 A1 | 1-23 |
| A | WO 2014/061709 A1 (NIPPON ZEON CO., LTD.) 24 April 2014, claims, examples & US 2015/0274647 A1 & US 2017/0349539 A1 & EP 2910579 A1 & CN 104755512 A & KR 10-2015-0073961 A | 1-23 |

Form PCT/ISA/210 (continuation of second sheet) (January 2015)

INTERNATIONAL SEARCH REPORT

International application No.
PCT/JP2018/009528

<Concerning the subject of the search>

Compounds represented by formula (I-1) in claim 1 include an extremely large number of compounds.

Meanwhile, among the compounds represented by formula (I-1), only a very small portion having a structure in which a phenyl group or naphthyl group bonded to an aryl hydrazonyl group is bonded with a divalent organic group, that is, a structure in which $Ar^0$ and $Ar^1$ are represented by formulas (II-1)-(II-7) in claim 2 are specifically indicated in the specification as being produced and confirmed as having the properties of an optically anisotropic substance.

Accordingly, in the inventions in claim 1, and claims 6-8 and 10-21 referring to claim 1, the inventions pertaining to compounds in which $Ar^0$ and $Ar^1$ of formula (I-1) have structures other than those of formulas (II-1)-(II-7) are not sufficiently supported by the description, and thus do not comply with the requirements under PCT Article 6.

In addition, because of the above-mentioned reason, it cannot be said that the description of the specification has been sufficiently described that a person skilled in the art would be able to understand how to manufacture and use, as optically anisotropic members, compounds in which $Ar^0$ and $Ar^1$ of formula (I-1) have structures other than those of formulas (II-1)-(II-7), and the description of the specification does not comply with the requirements under PCT Article 5.

Accordingly, searches for the inventions in claims 1, 6-8, and 10-21 were carried out for compounds in which $Ar^0$ and $Ar^1$ of formula (I-1) are represented by formulas (II-1)-(II-7).

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014010325 A
- WO 2012141245 A
- WO 2012147904 A
- WO 2013046781 A
- WO 2013180217 A
- WO 2014061709 A
- WO 2014065176 A
- WO 2014126113 A
- WO 2015025793 A
- WO 2015064698 A
- JP 2015140302 A
- WO 2015129654 A
- WO 2015141784 A
- WO 2016159193 A
- WO 2012169424 A
- WO 2012176679 A
- WO 2015122385 A
- JP 2007002208 A
- JP 2009173893 A
- JP 2009274984 A
- JP 2010030979 A
- JP 2010031223 A
- JP 2011006360 A
- JP 2010024438 A
- WO 201276679 A
- WO 2015122384 A
- JP H05310845 A
- US 5179171 B
- JP H0597978 A
- US 5202388 B
- JP H11124429 A
- WO 9920676 A